# FASCICULE DE BREVET EUROPEEN

(11) **EP 2 773 749 B1**
(45) Date de publication et mention de la délivrance du brevet: **04.01.2017**
(21) Numéro de dépôt: 12797734.6
(22) Date de dépôt: 02.11.2012
(51) Int. Cl.: C12N 5/0787, G01N 33/50

(54) **LIGNEES DE MASTOCYTES HUMAINS, PREPARATION ET UTILISATIONS**
MENSCHLICHE MASTZELLLINIEN, IHRE HERSTELLUNG UND VERWENDUNGEN DAVON
HUMAN MAST CELL LINES, PREPARATION AND USES

(30) Priorité: 02.11.2011 FR 1159902
(43) Date de publication de la demande: 10.09.2014
(73) Titulaire: Centre National de la Recherche Scientifique, 75016 Paris (FR); Ecole Normale Superieure De Cachan, 94235 Cachan Cedex (FR); Medizinische Universität Wien, 1090 Wien (AT)
(72) Inventeur: AROCK, Michel, F-75013 Paris (FR); VALENT, Peter, A-1180 Vienna (AT); SALEH, Rosine, 92120 Montrouge (FR); WEDEH, Ghaith, 94230 Cachan (FR); AUCLAIR, Christian, 78730 Saint-Arnoult-en-Yvelines (FR)
(74) Mandataire: Starck-Loudes, Anne-Caroline
(86) Numéro de dépôt international: PCT/EP2012/071731
(87) Numéro de publication internationale: WO 2013/064639

(56) Documents cités:
- WO-A2-03/065986
- WO-A2-2010/105215
- LAIDLAW TANYA M ET AL: "Characterization of a novel human mast cell line that responds to stem cell factor and expresses functional Fc is an element of RI", JOURNAL OF ALLERGY AND CLINICAL IMMUNOLOGY, vol. 127, no. 3, mars 2011 (2011-03), pages 815-822.E5, XP002672758, ISSN: 0091-6749
- GUHL SVEN ET AL: "Mast cell lines HMC-1 and LAD2 in comparison with mature human skin mast cells--drastically reduced levels of tryptase and chymase in mast cell lines.", EXPERIMENTAL DERMATOLOGY, vol. 19, no. 9, septembre 2010 (2010-09), pages 845-847, XP002672759, ISSN: 1600-0625
- GIBBS BERNHARD F ET AL: "Effects of Stem Cell Factor on Hypoxia-Inducible Factor 1 Alpha Accumulation in Human Acute Myeloid Leukaemia and LAD2 Mast Cells", PLOS ONE, vol. 6, no. 7, juillet 2011 (2011-07), XP002672760, ISSN: 1932-6203
- MOLFETTA ROSA ET AL: "Negative signals from Fc epsilon RI engagement attenuate mast cell functions", ARCHIVUM IMMUNOLOGIAE ET THERAPIAE EXPERIMENTALIS, vol. 55, no. 4, août 2007 (2007-08), pages 219-229, XP002672761,

### SEQUENCE LISTING

<110> Centre National de la Recherche Scientifique; Ecole Normale Supérieure de Cachan; Medizinische Universität Wien
<120> Lignées de mastocytes humains, préparations et utilisations
<130> B1166
<160> 6
<170> PatentIn version 3.3
<210> 1
   <211> 2931
   <212> DNA
   <213> Homo sapiens
<220>
   <221> exon
   <222> (1237)..(1352)
   <223> Exon 8
<220>
   <221> exon
   <222> (1353)..(1547)
   <223> Exon 9
<400> 1
   atgagaggcg ctcgcggcgc ctgggatttt ctctgcgttc tgctcctact gcttcgcgtc      60
   cagacaggct cttctcaacc atctgtgagt ccaggggaac cgtctccacc atccatccat     120
   ccaggaaaat cagacttaat agtccgcgtg ggcgacgaga ttaggctgtt atgcactgat     180
   ccgggctttg tcaaatggac ttttgagatc ctggatgaaa cgaatgagaa taagcagaat     240
   gaatggatca cggaaaaggc agaagccacc aacaccggca aatacacgtg caccaacaaa     300
   cacggcttaa gcaattccat ttatgtgttt gttagagatc ctgccaagct tttccttgtt     360
   gaccgctcct tgtatgggaa agaagacaac gacacgctgg tccgctgtcc tctcacagac     420
   ccagaagtga ccaattattc cctcaagggg tgccagggga agcctcttcc caaggacttg     480
   aggtttattc ctgaccccaa ggcgggcatc atgatcaaaa gtgtgaaacg cgcctaccat     540
   cggctctgtc tgcattgttc tgtggaccag gagggcaagt cagtgctgtc ggaaaaattc     600
   atcctgaaag tgaggccagc cttcaaagct gtgcctgttg tgtctgtgtc caaagcaagc     660
   tatcttctta gggaagggga agaattcaca gtgacgtgca caataaaaga tgtgtctagt     720
   tctgtgtact caacgtggaa aagagaaaac agtcagacta aactacagga gaaatataat     780
   agctggcatc acggtgactt caattatgaa cgtcaggcaa cgttgactat cagttcagcg     840
   agagttaatg attctggagt gttcatgtgt tatgccaata atactttggg atcagcaaat     900
   gtcacaacaa ccttggaagt agtagataaa ggattcatta atatcttccc catgataaac     960
   actacagtat ttgtaaacga tggagaaaat gtagatttga ttgttgaata tgaagcattc    1020
   cccaaacctg aacaccagca gtggatctat atgaacagaa ccttcactga taaatgggaa   1080
   gattatccca agtctgagaa tgaaagtaat atcagatacg taagtgaact tcatctaacg   1140
   agattaaaag gcaccgaagg aggcacttac acattcctag tgtccaattc tgacgtcaat   1200
   gctgccatag catttaatgt ttatgtgaat acaaaa cca gaa atc ctg act tac     1254
                                           Pro Glu Ile Leu Thr Tyr
                                           1               5
   gac agg ctc gtg aat ggc atg ctc caa tgt gtg gca gca gga ttc cca     1302
   Asp Arg Leu Val Asn Gly Met Leu Gln Cys Val Ala Ala Gly Phe Pro
               10                  15                  20
   gag ccc aca ata gat tgg tat ttt tgt cca gga act gag cag aga tgc     1350
   Glu Pro Thr Ile Asp Trp Tyr Phe Cys Pro Gly Thr Glu Gln Arg Cys
           25                  30                  35
   tc  t gct tct gta ctg cca gtg gat gtg cag aca cta aac tca tct ggg   1398
   Ser   Ala Ser Val Leu Pro Val Asp Val Gln Thr Leu Asn Ser Ser Gly
         40                  45                  50
   cca ccg ttt gga aag cta gtg gtt cag agt tct ata gat tct agt gca     1446
   Pro Pro Phe Gly Lys Leu Val Val Gln Ser Ser Ile Asp Ser Ser Ala
   55                  60                  65                  70
   ttc aag cac aat ggc acg gtt gaa tgt aag gct tac aac gat gtg ggc     1494
   Phe Lys His Asn Gly Thr Val Glu Cys Lys Ala Tyr Asn Asp Val Gly
                   75                  80                  85
   aag act tct gcc tat ttt aac ttt gca ttt aaa ggt aac aac aaa gag     1542
   Lys Thr Ser Ala Tyr Phe Asn Phe Ala Phe Lys Gly Asn Asn Lys Glu
               90                  95                  100
   caa at ccatccccac accctgttca ctcctttgct gattggtttc gtaatcgtag       1597
   Gln
   ctggcatgat gtgcattatt gtgatgattc tgacctacaa atatttacag aaacccatgt   1657
   atgaagtaca gtggaaggtt gttgaggaga taaatggaaa caattatgtt tacatagacc   1717
   caacacaact tccttatgat cacaaatggg agtttcccag aaacaggctg agttttggga   1777
   aaaccctggg tgctggagct ttcgggaagg ttgttgaggc aactgcttat ggcttaatta   1837
   agtcagatgc ggccatgact gtcgctgtaa agatgctcaa gccgagtgcc catttgacag   1897
   aacgggaagc cctcatgtct gaactcaaag tcctgagtta ccttggtaat cacatgaata   1957
   ttgtgaatct acttggagcc tgcaccattg gagggcccac cctggtcatt acagaatatt   2017
   gttgctatgg tgatcttttg aatttttgga gaagaaaacg tgattcattt atttgttcaa    2077
   agcaggaaga tcatgcagaa gctgcacttt ataagaatct tctgcattca aaggagtctt   2137
   cctgcagcga tagtactaat gagtacatgg acatgaaacc tggagtttct tatgttgtcc   2197
   caaccaaggc cgacaaaagg agatctgtga gaataggctc atacatagaa agagatgtga   2257
   ctcccgccat catggaggat gacgagttgg ccctagactt agaagacttg ctgagctttt   2317
   cttaccaggt ggcaaagggc atggctttcc tcgcctccaa gaattgtatt cacagagact 2377
   tggcagccag aaatatcctc cttactcatg gtcggatcac aaagatttgt gattttggtc 2437
   tagccagaga catcaagaat gattctaatt atgtggttaa aggaaacgct cgactacctg 2497
   tgaagtggat ggcacctgaa agcattttca actgtgtata cacgtttgaa agtgacgtct 2557
   ggtcctatgg gatttttctt tgggagctgt tctctttagg aagcagcccc tatcctggaa 2617
   tgccggtcga ttctaagttc tacaagatga tcaaggaagg cttccggatg ctcagccctg 2677
   aacacgcacc tgctgaaatg tatgacataa tgaagacttg ctgggatgca gatcccctaa 2737
   aaagaccaac attcaagcaa attgttcagc taattgagaa gcagatttca gagagcacca 2797
   atcatattta ctccaactta gcaaactgca gccccaaccg acagaagccc gtggtagacc 2857
   attctgtgcg gatcaattct gtcggcagca ccgcttcctc ctcccagcct ctgcttgtgc 2917
   acgacgatgt ctga 2931
<210> 2
   <211> 976
   <212> PRT
   <213> Homo sapiens
<220>
   <221> DOMAIN
   <222> (26)..(519)
   <223> Domaine extra-cellulaire
<220>
   <221> DOMAIN
   <222> (543)..(582)
   <223> Domaine juxta-membranaire
<220>
   <221> DOMAIN
   <222> (762)..(937)
   <223> Domaine phosphotransferase
<400> 2
<210> 3
   <211> 2919
   <212> DNA
   <213> Homo sapiens
<220>
   <221> misc_feature
   <223> KIT D816V
<220>
   <221> misc_feature
   <222> (2434)..(2436)
   <223> mutation
<400> 3
   atgagaggcg ctcgcggcgc ctgggatttt ctctgcgttc tgctcctact gcttcgcgtc      60
   cagacaggct cttctcaacc atctgtgagt ccaggggaac cgtctccacc atccatccat     120
   ccaggaaaat cagacttaat agtccgcgtg ggcgacgaga ttaggctgtt atgcactgat     180
   ccgggctttg tcaaatggac ttttgagatc ctggatgaaa cgaatgagaa taagcagaat     240
   gaatggatca cggaaaaggc agaagccacc aacaccggca aatacacgtg caccaacaaa     300
   cacggcttaa gcaattccat ttatgtgttt gttagagatc ctgccaagct tttccttgtt    360
   gaccgctcct tgtatgggaa agaagacaac gacacgctgg tccgctgtcc tctcacagac    420
   ccagaagtga ccaattattc cctcaagggg tgccagggga agcctcttcc caaggacttg    480
   aggtttattc ctgaccccaa ggcgggcatc atgatcaaaa gtgtgaaacg cgcctaccat    540
   cggctctgtc tgcattgttc tgtggaccag gagggcaagt cagtgctgtc ggaaaaattc    600
   atcctgaaag tgaggccagc cttcaaagct gtgcctgttg tgtctgtgtc caaagcaagc    660
   tatcttctta gggaagggga agaattcaca gtgacgtgca caataaaaga tgtgtctagt    720
   tctgtgtact caacgtggaa aagagaaaac agtcagacta aactacagga gaaatataat    780
   agctggcatc acggtgactt caattatgaa cgtcaggcaa cgttgactat cagttcagcg    840
   agagttaatg attctggagt gttcatgtgt tatgccaata atacttttgg atcagcaaat    900
   gtcacaacaa ccttggaagt agtagataaa ggattcatta atatcttccc catgataaac    960
   actacagtat ttgtaaacga tggagaaaat gtagatttga ttgttgaata tgaagcattc   1020
   cccaaacctg aacaccagca gtggatctat atgaacagaa ccttcactga taaatgggaa   1080
   gattatccca agtctgagaa tgaaagtaat atcagatacg taagtgaact tcatctaacg   1140
   agattaaaag gcaccgaagg aggcacttac acattcctag tgtccaattc tgacgtcaat   1200
   gctgccatag catttaatgt ttatgtgaat acaaaaccag aaatcctgac ttacgacagg   1260
   ctcgtgaatg gcatgctcca atgtgtggca gcaggattcc cagagcccac aatagattgg   1320
   tatttttgtc caggaactga gcagagatgc tctgcttctg tactgccagt ggatgtgcag   1380
   acactaaact catctgggcc accgtttgga aagctagtgg ttcagagttc tatagattct   1440
   agtgcattca agcacaatgg cacggttgaa tgtaaggctt acaacgatgt gggcaagact   1500
   tctgcctatt ttaactttgc atttaaagag caaatccatc cccacaccct gttcactcct   1560
   ttgctgattg gtttcgtaat cgtagctggc atgatgtgca ttattgtgat gattctgacc   1620
   tacaaatatt tacagaaacc catgtatgaa gtacagtgga aggttgttga ggagataaat   1680
   ggaaacaatt atgtttacat agacccaaca caacttcctt atgatcacaa atgggagttt   1740
   cccagaaaca ggctgagttt tgggaaaacc ctgggtgctg gagctttcgg gaaggttgtt   1800
   gaggcaactg cttatggctt aattaagtca gatgcggcca tgactgtcgc tgtaaagatg   1860
   ctcaagccga gtgcccattt gacagaacgg gaagccctca tgtctgaact caaagtcctg   1920
   agttaccttg gtaatcacat gaatattgtg aatctacttg gagcctgcac cattggaggg   1980
   cccaccctgg tcattacaga atattgttgc tatggtgatc ttttgaattt tttgagaaga   2040
   aaacgtgatt catttatttg ttcaaagcag gaagatcatg cagaagctgc actttataag   2100
   aatcttctgc attcaaagga gtcttcctgc agcgatagta ctaatgagta catggacatg   2160
   aaacctggag tttcttatgt tgtcccaacc aaggccgaca aaaggagatc tgtgagaata   2220
   ggctcataca tagaaagaga tgtgactccc gccatcatgg aggatgacga gttggcccta   2280
   gacttagaag acttgctgag cttttcttac caggtggcaa agggcatggc tttcctcgcc   2340
   tccaagaatt gtattcacag agacttggca gccagaaata tcctccttac tcatggtcgg   2400
   atcacaaaga tttgtgattt tggtctagcc agagtcatca agaatgattc taattatgtg   2460
   gttaaaggaa acgctcgact acctgtgaag tggatggcac ctgaaagcat tttcaactgt   2520
   gtatacacgt ttgaaagtga cgtctggtcc tatgggattt ttctttggga gctgttctct   2580
   ttaggaagca gcccctatcc tggaatgccg gtcgattcta agttctacaa gatgatcaag   2640
   gaaggcttcc ggatgctcag ccctgaacac gcacctgctg aaatgtatga cataatgaag   2700
   acttgctggg atgcagatcc cctaaaaaga ccaacattca agcaaattgt tcagctaatt   2760
   gagaagcaga tttcagagag caccaatcat atttactcca acttagcaaa ctgcagcccc   2820
   aaccgacaga agcccgtggt agaccattct gtgcggatca attctgtcgg cagcaccgct   2880
   tcctcctccc agcctctgct tgtgcacgac gatgtctga                          2919
<210> 4
   <211> 976
   <212> PRT
   <213> Homo sapiens
<220>
   <221> MISC_FEATURE
   <223> Protéine KIT D816V
<220>
   <221> MISC_FEATURE
   <222> (816)..(816)
   <223> Mutation
<400> 4
   Met Arg Gly Ala Arg Gly Ala Trp Asp Phe Leu Cys Val Leu Leu Leu
   1               5                   10                  15
   Leu Leu Arg Val Gln Thr Gly Ser Ser Gln Pro Ser Val Ser Pro Gly
               20                  25                  30
   Glu Pro Ser Pro Pro Ser Ile His Pro Gly Lys Ser Asp Leu Ile Val
           35                  40                  45
   Arg Val Gly Asp Glu Ile Arg Leu Leu Cys Thr Asp Pro Gly Phe Val
       50                  55                  60
   Lys Trp Thr Phe Glu Ile Leu Asp Glu Thr Asn Glu Asn Lys Gln Asn
   65                  70                  75                  80
   Glu Trp Ile Thr Glu Lys Ala Glu Ala Thr Asn Thr Gly Lys Tyr Thr
                   85                  90                  95
   Cys Thr Asn Lys His Gly Leu Ser Asn Ser Ile Tyr Val Phe Val Arg
               100                 105                 110
   Asp Pro Ala Lys Leu Phe Leu Val Asp Arg Ser Leu Tyr Gly Lys Glu
           115                 120                 125
   Asp Asn Asp Thr Leu Val Arg Cys Pro Leu Thr Asp Pro Glu Val Thr
       130                 135                 140
   Asn Tyr Ser Leu Lys Gly Cys Gln Gly Lys Pro Leu Pro Lys Asp Leu
   145                 150                 155                 160
   Arg Phe Ile Pro Asp Pro Lys Ala Gly Ile Met Ile Lys Ser Val Lys
                   165                 170                 175
   Arg Ala Tyr His Arg Leu Cys Leu His Cys Ser Val Asp Gln Glu Gly
               180                 185                 190
   Lys Ser Val Leu Ser Glu Lys Phe Ile Leu Lys Val Arg Pro Ala Phe
           195                 200                 205
   Lys Ala Val Pro Val Val Ser Val Ser Lys Ala Ser Tyr Leu Leu Arg
       210                 215                 220
   Glu Gly Glu Glu Phe Thr Val Thr Cys Thr Ile Lys Asp Val Ser Ser
   225                 230                 235                 240
   Ser Val Tyr Ser Thr Trp Lys Arg Glu Asn Ser Gln Thr Lys Leu Gln
                   245                 250                 255
   Glu Lys Tyr Asn Ser Trp His His Gly Asp Phe Asn Tyr Glu Arg Gln
               260                 265                 270
   Ala Thr Leu Thr Ile Ser Ser Ala Arg Val Asn Asp Ser Gly Val Phe
           275                 280                 285
   Met Cys Tyr Ala Asn Asn Thr Phe Gly Ser Ala Asn Val Thr Thr Thr
       290                 295                 300
   Leu Glu Val Val Asp Lys Gly Phe Ile Asn Ile Phe Pro Met Ile Asn
   305                 310                 315                 320
   Thr Thr Val Phe Val Asn Asp Gly Glu Asn Val Asp Leu Ile Val Glu
                   325                 330                 335
   Tyr Glu Ala Phe Pro Lys Pro Glu His Gln Gln Trp Ile Tyr Met Asn
               340                 345                 350
   Arg Thr Phe Thr Asp Lys Trp Glu Asp Tyr Pro Lys Ser Glu Asn Glu
           355                 360                 365
   Ser Asn Ile Arg Tyr Val Ser Glu Leu His Leu Thr Arg Leu Lys Gly
       370                 375                 380
   Thr Glu Gly Gly Thr Tyr Thr Phe Leu Val Ser Asn Ser Asp Val Asn
   385                 390                 395                 400
   Ala Ala Ile Ala Phe Asn Val Tyr Val Asn Thr Lys Pro Glu Ile Leu
                   405                 410                 415
   Thr Tyr Asp Arg Leu Val Asn Gly Met Leu Gln Cys Val Ala Ala Gly
               420                 425                 430
   Phe Pro Glu Pro Thr Ile Asp Trp Tyr Phe Cys Pro Gly Thr Glu Gln
           435                 440                 445
   Arg Cys Ser Ala Ser Val Leu Pro Val Asp Val Gln Thr Leu Asn Ser
       450                 455                 460
   Ser Gly Pro Pro Phe Gly Lys Leu Val Val Gln Ser Ser Ile Asp Ser
   465                 470                 475                 480
   Ser Ala Phe Lys His Asn Gly Thr Val Glu Cys Lys Ala Tyr Asn Asp
                   485                 490                 495
   Val Gly Lys Thr Ser Ala Tyr Phe Asn Phe Ala Phe Lys Gly Asn Asn
               500                 505                 510
   Lys Glu Gln Ile His Pro His Thr Leu Phe Thr Pro Leu Leu Ile Gly
           515                 520                 525
   Phe Val Ile Val Ala Gly Met Met Cys Ile Ile Val Met Ile Leu Thr
       530                 535                 540
   Tyr Lys Tyr Leu Gln Lys Pro Met Tyr Glu Val Gln Trp Lys Val Val
   545                 550                 555                 560
   Glu Glu Ile Asn Gly Asn Asn Tyr Val Tyr Ile Asp Pro Thr Gln Leu
                   565                 570                 575
   Pro Tyr Asp His Lys Trp Glu Phe Pro Arg Asn Arg Leu Ser Phe Gly
   580 585 590
   Lys Thr Leu Gly Ala Gly Ala Phe Gly Lys Val Val Glu Ala Thr Ala
   595 600 605
   Tyr Gly Leu Ile Lys Ser Asp Ala Ala Met Thr Val Ala Val Lys Met
   610 615 620
   Leu Lys Pro Ser Ala His Leu Thr Glu Arg Glu Ala Leu Met Ser Glu
   625 630 635 640
   Leu Lys Val Leu Ser Tyr Leu Gly Asn His Met Asn Ile Val Asn Leu
   645 650 655
   Leu Gly Ala Cys Thr Ile Gly Gly Pro Thr Leu Val Ile Thr Glu Tyr
   660 665 670
   Cys Cys Tyr Gly Asp Leu Leu Asn Phe Leu Arg Arg Lys Arg Asp Ser
   675 680 685
   Phe Ile Cys Ser Lys Gln Glu Asp His Ala Glu Ala Ala Leu Tyr Lys
   690 695 700
   Asn Leu Leu His Ser Lys Glu Ser Ser Cys Ser Asp Ser Thr Asn Glu
   705 710 715 720
   Tyr Met Asp Met Lys Pro Gly Val Ser Tyr Val Val Pro Thr Lys Ala
   725 730 735
   Asp Lys Arg Arg Ser Val Arg Ile Gly Ser Tyr Ile Glu Arg Asp Val
   740 745 750
   Thr Pro Ala Ile Met Glu Asp Asp Glu Leu Ala Ile Asp Leu Glu Asp
   755 760 765
   Leu Leu Ser Phe Ser Tyr Gln Val Ala Lys Gly Met Ala Phe Leu Ala
   770 775 780
   Ser Lys Asn Cys Ile His Arg Asp Leu Ala Ala Arg Asn Ile Leu Leu
   785 790 795 800
   Thr His Gly Arg Ile Thr Lys Ile Cys Asp Phe Gly Leu Ala Arg Val
   805 810 815
   Ile Lys Asn Asp Ser Asn Tyr Val Val Lys Gly Asn Ala Arg Leu Pro
   820 825 830
   Val Lys Trp Met Ala His Glu Ser Ile Phe Asn Cys Val Tyr Thr Phe
           835                 840                 845
   Glu Ser Asp Val Trp Ser Tyr Gly Ile Phe Leu Trp Glu Leu Phe Ser
       850                 855                 860
   Leu Gly Ser Ser Pro Tyr Pro Gly Met Pro Val Asp Ser Lys Phe Tyr
   865                 870                 875                 880
   Lys Met Ile Lys Glu Gly Phe Arg Met Leu Ser Pro Glu His Ala Pro
                   885                 890                 895
   Ala Glu Met Tyr Asp Ile Met Lys Thr Cys Trp Asp Ala Asp Pro Leu
               900                 905                 910
   Lys Arg Pro Thr Phe Lys Gln Ile Val Gln Leu Ile Glu Lys Gln Ile
           915                 920                 925
   Ser Glu Ser Thr Asn His Ile Tyr Ser Asn Leu Ala Asn Cys Ser Pro
       930                 935                 940
   Asn Arg Gln Lys Pro Val Val Asp His Ser Val Arg Ile Asn Ser Val
   945                 950                 955                 960
   Gly Ser Thr Ala Ser Ser Ser Gln Pro Leu Leu Val His Asp Asp Val
                   965                 970                 975
<210> 5
   <211> 2913
   <212> DNA
   <213> Homo sapiens
<220>
   <221> misc_feature
   <223> KIT Delta 417-419 InsY
<220>
   <221> misc_feature
   <222> (417)..(419)
   <223> Mutation
<400> 5
   atgagaggcg ctcgcggcgc ctgggatttt ctctgcgttc tgctcctact gcttcgcgtc     60
   cagacaggct cttctcaacc atctgtgagt ccaggggaac cgtctccacc atccatccat    120
   ccaggaaaat cagacttaat agtccgcgtg ggcgacgaga ttaggctgtt atgcactgat    180
   ccgggctttg tcaaatggac ttttgagatc ctggatgaaa cgaatgagaa taagcagaat    240
   gaatggatca cggaaaaggc agaagccacc aacaccggca aatacacgtg caccaacaaa    300
   cacggcttaa gcaattccat ttatgtgttt gttagagatc ctgccaagct tttccttgtt    360
   gaccgctcct tgtatgggaa agaagacaac gacacgctgg tccgctgtcc tctcacagac 420
   ccagaagtga ccaattattc cctcaagggg tgccagggga agcctcttcc caaggacttg 480
   aggtttattc ctgaccccaa ggcgggcatc atgatcaaaa gtgtgaaacg cgcctaccat 540
   cggctctgtc tgcattgttc tgtggaccag gagggcaagt cagtgctgtc ggaaaaattc 600
   atcctgaaag tgaggccagc cttcaaagct gtgcctgttg tgtctgtgtc caaagcaagc 660
   tatcttctta gggaagggga agaattcaca gtgacgtgca caataaaaga tgtgtctagt 720
   tctgtgtact caacgtggaa aagagaaaac agtcagacta aactacagga gaaatataat 780
   agctggcatc acggtgactt caattatgaa cgtcaggcaa cgttgactat cagttcagcg 840
   agagttaatg attctggagt gttcatgtgt tatgccaata atacttttgg atcagcaaat 900
   gtcacaacaa ccttggaagt agtagataaa ggattcatta atatcttccc catgataaac 960
   actacagtat ttgtaaacga tggagaaaat gtagatttga ttgttgaata tgaagcattc 1020
   cccaaacctg aacaccagca gtggatctat atgaacagaa ccttcactga taaatgggaa 1080
   gattatccca agtctgagaa tgaaagtaat atcagatacg taagtgaact tcatctaacg 1140
   agattaaaag gcaccgaagg aggcacttac acattcctag tgtccaattc tgacgtcaat 1200
   gctgccatag catttaatgt ttatgtgaat acaaaaccag aaatcctgta caggctcgtg 1260
   aatggcatgc tccaatgtgt ggcagcagga ttcccagagc ccacaataga ttggtatttt 1320
   tgtccaggaa ctgagcagag atgctctgct tctgtactgc cagtggatgt gcagacacta 1380
   aactcatctg ggccaccgtt tggaaagcta gtggttcaga gttctataga ttctagtgca 1440
   ttcaagcaca atggcacggt tgaatgtaag gcttacaacg atgtgggcaa gacttctgcc 1500
   tattttaact ttgcatttaa agagcaaatc catccccaca ccctgttcac tcctttgctg 1560
   attggtttcg taatcgtagc tggcatgatg tgcattattg tgatgattct gacctacaaa 1620
   tatttacaga aacccatgta tgaagtacag tggaaggttg ttgaggagat aaatggaaac 1680
   aattatgttt acatagaccc aacacaactt ccttatgatc acaaatggga gtttcccaga 1740
   aacaggctga gttttgggaa aaccctgggt gctggagctt tcgggaaggt tgttgaggca 1800
   actgcttatg gcttaattaa gtcagatgcg gccatgactg tcgctgtaaa gatgctcaag 1860
   ccgagtgccc atttgacaga acgggaagcc ctcatgtctg aactcaaagt cctgagttac 1920
   cttggtaatc acatgaatat tgtgaatcta cttggagcct gcaccattgg agggcccacc 1980
   ctggtcatta cagaatattg ttgctatggt gatcttttga attttttgag aagaaaacgt 2040
   gattcattta tttgttcaaa gcaggaagat catgcagaag ctgcacttta taagaatctt 2100
   ctgcattcaa aggagtcttc ctgcagcgat agtactaatg agtacatgga catgaaacct 2160
   ggagtttctt atgttgtccc aaccaaggcc gacaaaagga gatctgtgag aataggctca 2220
   tacatagaaa gagatgtgac tcccgccatc atggaggatg acgagttggc cctagactta   2280
   gaagacttgc tgagcttttc ttaccaggtg gcaaagggca tggctttcct cgcctccaag   2340
   aattgtattc acagagactt ggcagccaga aatatcctcc ttactcatgg tcggatcaca   2400
   aagatttgtg attttggtct agccagagac atcaagaatg attctaatta tgtggttaaa   2460
   ggaaacgctc gactacctgt gaagtggatg gcacctgaaa gcattttcaa ctgtgtatac   2520
   acgtttgaaa gtgacgtctg gtcctatggg atttttcttt gggagctgtt ctctttagga   2580
   agcagcccct atcctggaat gccggtcgat tctaagttct acaagatgat caaggaaggc   2640
   ttccggatgc tcagccctga acacgcacct gctgaaatgt atgacataat gaagacttgc   2700
   tgggatgcag atcccctaaa aagaccaaca ttcaagcaaa ttgttcagct aattgagaag   2760
   cagatttcag agagcaccaa tcatatttac tccaacttag caaactgcag ccccaaccga   2820
   cagaagcccg tggtagacca ttctgtgcgg atcaattctg tcggcagcac cgcttcctcc   2880
   tcccagcctc tgcttgtgca cgacgatgtc tga                                2913
<210> 6
   <211> 974
   <212> PRT
   <213> Homo sapiens
<220>
   <221> MISC_FEATURE
   <223> KIT Delta 417-419 insY
<220>
   <221> MISC_FEATURE
   <222> (417)..(419)
   <223> Mutation
<400> 6
   Met Arg Gly Ala Arg Gly Ala Trp Asp Phe Leu Cys Val Leu Leu Leu
   1               5                   10                  15
   Leu Leu Arg Val Gln Thr Gly Ser Ser Gln Pro Ser Val Ser Pro Gly
               20                  25                  30
   Glu Pro Ser Pro Pro Ser Ile His Pro Gly Lys Ser Asp Leu Ile Val
           35                  40                  45
   Arg Val Gly Asp Glu Ile Arg Leu Leu Cys Thr Asp Pro Gly Phe Val
       50                  55                  60
   Lys Trp Thr Phe Glu Ile Leu Asp Glu Thr Asn Glu Asn Lys Gln Asn
   65                  70                  75                  80
   Glu Trp Ile Thr Glu Lys Ala Glu Ala Thr Asn Thr Gly Lys Tyr Thr
                   85                  90                  95
   Cys Thr Asn Lys His Gly Leu Ser Asn Ser Ile Tyr Val Phe Val Arg
               100                 105                 110
   Asp Pro Ala Lys Leu Phe Leu Val Asp Arg Ser Leu Tyr Gly Lys Glu
           115                 120                 125
   Asp Asn Asp Thr Leu Val Arg Cys Pro Leu Thr Asp Pro Glu Val Thr
       130                 135                 140
   Asn Tyr Ser Leu Lys Gly Cys Gln Gly Lys Pro Leu Pro Lys Asp Leu
   145                 150                 155                 160
   Arg Phe Ile Pro Asp Pro Lys Ala Gly Ile Met Ile Lys Ser Val Lys
                   165                 170                 175
   Arg Ala Tyr His Arg Leu Cys Leu His Cys Ser Val Asp Gln Glu Gly
               180                 185                 190
   Lys Ser Val Leu Ser Glu Lys Phe Ile Leu Lys Val Arg Pro Ala Phe
           195                 200                 205
   Lys Ala Val Pro Val Val Ser Val Ser Lys Ala Ser Tyr Leu Leu Arg
       210                 215                 220
   Glu Gly Glu Glu Phe Thr Val Thr Cys Thr Ile Lys Asp Val Ser Ser
   225                 230                 235                 240
   Ser Val Tyr Ser Thr Trp Lys Arg Glu Asn Ser Gln Thr Lys Leu Gln
                   245                 250                 255
   Glu Lys Tyr Asn Ser Trp His His Gly Asp Phe Asn Tyr Glu Arg Gln
               260                 265                 270
   Ala Thr Leu Thr Ile Ser Ser Ala Arg Val Asn Asp Ser Gly Val Phe
           275                 280                 285
   Met Cys Tyr Ala Asn Asn Thr Phe Gly Ser Ala Asn Val Thr Thr Thr
       290                 295                 300
   Leu Glu Val Val Asp Lys Gly Phe Ile Asn Ile Phe Pro Met Ile Asn
   305                 310                 315                 320
   Thr Thr Val Phe Val Asn Asp Gly Glu Asn Val Asp Leu Ile Val Glu
                   325                 330                 335
   Tyr Glu Ala Phe Pro Lys Pro Glu His Gln Gln Trp Ile Tyr Met Asn
               340                 345                 350
   Arg Thr Phe Thr Asp Lys Trp Glu Asp Tyr Pro Lys Ser Glu Asn Glu
           355                 360                 365
   Ser Asn Ile Arg Tyr Val Ser Glu Leu His Leu Thr Arg Leu Lys Gly
       370                 375                 380
   Thr Glu Gly Gly Thr Tyr Thr Phe Leu Val Ser Asn Ser Asp Val Asn
   385                 390                 395                 400
   Ala Ala Ile Ala Phe Asn Val Tyr Val Asn Thr Lys Pro Glu Ile Leu
                   405                 410                 415
   Tyr Arg Leu Val Asn Gly Met Leu Gln Cys Val Ala Ala Gly Phe Pro
               420                 425                 430
   Glu Pro Thr Ile Asp Trp Tyr Phe Cys Pro Gly Thr Glu Gln Arg Cys
           435                 440                 445
   Ser Ala Ser Val Leu Pro Val Asp Val Gln Thr Leu Asn Ser Ser Gly
       450                 455                 460
   Pro Pro Phe Gly Lys Leu Val Val Gln Ser Ser Ile Asp Ser Ser Ala
   465                 470                 475                 480
   Phe Lys His Asn Gly Thr Val Glu Cys Lys Ala Tyr Asn Asp Val Gly
                   485                 490                 495
   Lys Thr Ser Ala Tyr Phe Asn Phe Ala Phe Lys Gly Asn Asn Lys Glu
               500                 505                 510
   Gln Ile His Pro His Thr Leu Phe Thr Pro Leu Leu Ile Gly Phe Val
           515                 520                 525
   Ile Val Ala Gly Met Met Cys Ile Ile Val Met Ile Leu Thr Tyr Lys
       530                 535                 540
   Tyr Leu Gln Lys Pro Met Tyr Glu Val Gln Trp Lys Val Val Glu Glu
   545                 550                 555                 560
   Ile Asn Gly Asn Asn Tyr Val Tyr Ile Asp Pro Thr Gln Leu Pro Tyr
                   565                 570                 575
   Asp His Lys Trp Glu Phe Pro Arg Asn Arg Leu Ser Phe Gly Lys Thr
               580                 585                 590
   Leu Gly Ala Gly Ala Phe Gly Lys Val Val Glu Ala Thr Ala Tyr Gly
           595                 600                 605
   Leu Ile Lys Ser Asp Ala Ala Met Thr Val Ala Val Lys Met Leu Lys
       610                 615                 620
   Pro Ser Ala His Leu Thr Glu Arg Glu Ala Leu Met Ser Glu Leu Lys
   625                 630                 635                 640
   Val Leu Ser Tyr Leu Gly Asn His Met Asn Ile Val Asn Leu Leu Gly
                   645                 650                 655
   Ala Cys Thr Ile Gly Gly Pro Thr Leu Val Ile Thr Glu Tyr Cys Cys
               660                 665                 670
   Tyr Gly Asp Leu Leu Asn Phe Leu Arg Arg Lys Arg Asp Ser Phe Ile
           675                 680                 685
   Cys Ser Lys Gln Glu Asp His Ala Glu Ala Ala Leu Tyr Lys Asn Leu
       690                 695                 700
   Leu His Ser Lys Glu Ser Ser Cys Ser Asp Ser Thr Asn Glu Tyr Met
   705                 710                 715                 720
   Asp Met Lys Pro Gly Val Ser Tyr Val Val Pro Thr Lys Ala Asp Lys
                   725                 730                 735
   Arg Arg Ser Val Arg Ile Gly Ser Tyr Ile Glu Arg Asp Val Thr Pro
               740                 745                 750
   Ala Ile Met Glu Asp Asp Glu Leu Ala Ile Asp Leu Glu Asp Leu Leu
           755                 760                 765
   Ser Phe Ser Tyr Gln Val Ala Lys Gly Met Ala Phe Leu Ala Ser Lys
       770                 775                 780
   Asn Cys Ile His Arg Asp Leu Ala Ala Arg Asn Ile Leu Leu Thr His
   785                 790                 795                 800
   Gly Arg Ile Thr Lys Ile Cys Asp Phe Gly Leu Ala Arg Asp Ile Lys
                   805                 810                 815
   Asn Asp Ser Asn Tyr Val Val Lys Gly Asn Ala Arg Leu Pro Val Lys
               820                 825                 830
   Trp Met Ala His Glu Ser Ile Phe Asn Cys Val Tyr Thr Phe Glu Ser
           835                 840                 845
   Asp Val Trp Ser Tyr Gly Ile Phe Leu Trp Glu Leu Phe Ser Leu Gly
       850                 855                 860
   Ser Ser Pro Tyr Pro Gly Met Pro Val Asp Ser Lys Phe Tyr Lys Met
   865                 870                 875                 880
   Ile Lys Glu Gly Phe Arg Met Leu Ser Pro Glu His Ala Pro Ala Glu
                   885                 890                 895
   Met Tyr Asp Ile Met Lys Thr Cys Trp Asp Ala Asp Pro Leu Lys Arg
               900                 905                 910
   Pro Thr Phe Lys Gln Ile Val Gln Leu Ile Glu Lys Gln Ile Ser Glu
           915                 920                 925
   Ser Thr Asn His Ile Tyr Ser Asn Leu Ala Asn Cys Ser Pro Asn Arg
       930                 935                 940
   Gln Lys Pro Val Val Asp His Ser Val Arg Ile Asn Ser Val Gly Ser
   945                 950                 955                 960
   Thr Ala Ser Ser Ser Gln Pro Leu Leu Val His Asp Asp Val
                   965                 970

## Description

La présente description concerne une nouvelle lignée de mastocytes humains (identifiée dans le présent texte en tant que lignée ROSA KIT WT) dont les cellules expriment le récepteur KIT sauvage (également identifié en tant que CD117, KIT wild-type ou KIT WT dans le présent texte) ainsi que le récepteur de haute affinité des IgE (également identifié en tant que récepteur FcεR1 ou FcepsilonR1 dans le présent texte), ainsi que toute lignée cellulaire dérivée, tout clone cellulaire dérivé, et tout mutant de ladite lignée cellulaire mastocytaire humaine ayant conservé au moins une desdites caractéristiques morphologiques, ultrastructurales, phénotypiques et/ou fonctionnelles de ladite lignée ROSA KIT WT. Cette lignée a été obtenue à partir de cellules provenant du sang de cordon d'un sujet humain sain. La description concerne également des lignées particulières de cellules dérivées de la lignée ROSA KIT WT, en particulier les lignées identifiées dans le présent texte en tant que « ROSA KIT D816V » et « ROSA KIT Delta 417-419 insY » qui présentent un récepteur KIT (CD117) muté.
La description concerne aussi les utilisations des lignées décrites en recherche, en diagnostic, et/ou en thérapie, en particulier pour le criblage de molécules d'intérêt dans le traitement des pathologies dans lesquelles les mastocytes sont impliqués (allergies, maladies inflammatoires, cancers), en particulier dans lesquelles les mastocytes ont un rôle délétère.

### ART ANTERIEUR

L'allergie est une des pathologies les plus fréquentes (15-20%) et constitue, quelle que soit sa forme, un problème de santé d'autant plus préoccupant qu'on constate qu'elle est en constante augmentation dans les pays industrialisés. L'allergie est liée, dans la plupart des cas, à une déviation de la réponse immune normale conduisant à la synthèse d'immunoglobulines particulières, de type E (IgE), dirigées contre des antigènes de l'environnement (pollens, acariens de poussières, poils et phanères d'animaux de compagnie, antigènes alimentaires, etc.). Les cellules IgE-dépendantes effectrices de la réaction allergique sont les mastocytes (MC). Les mastocytes sont des cellules à distribution tissulaire ubiquitaire, issues des cellules hématopoïétiques multipotentes (non engagées) CD34+, qui jouent un rôle important dans l'initiation de la réponse immunitaire innée et adaptative, ainsi que dans les réactions allergiques IgE-dépendantes et dans diverses réactions inflammatoires. En effet, les mastocytes expriment à leur surface un grand nombre de récepteurs de haute affinité pour les Immunoglobulines de type E (Fcepsilon R1 ou FcεR1). Ces récepteurs fixent le fragment Fc de ces Immunoglobulines, ce qui les stabilisent à la surface de la cellule pendant des mois, voire des années. Lors d'un contact avec l'allergène correspondant à ces IgE, il s'en suit une agrégation des récepteurs par l'allergène, conduisant à l'activation d'une cascade de la signalisation (« transduction du signal ») à l'intérieur des cellules. Cette activation est suivie, en quelques secondes, par la dégranulation massive des cellules avec libération de médiateurs préformés comme l'histamine, l'héparine et des protéases telle que la tryptase. Quelques minutes après, la cellule activée synthétise *de novo* des quantités importantes de dérivés de l'acide arachidonique (leucotriènes et prostaglandines) à partir des lipides de la membrane. Ces médiateurs préformés et néosynthétisés libérés précocément par le mastocyte activé jouent un rôle central dans la réaction allergique immédiate qui peut aller de la simple rhinite allergique jusqu'au choc anaphylactique parfois mortel. En outre, quelques heures après l'activation IgE-dépendante, le mastocyte synthétise et libère une myriade de cytokines et de chimiokines qui interviennent dans la phase retardée de la réaction allergique en attirant et en activant d'autres types cellulaires (polynucléaires éosinophiles et neutrophiles, lymphocytes, monocytes) qui participent ainsi à la destruction chronique des tissus, comme c'est le cas par exemple au cours de l'asthme allergique.
Il n'existe à l'heure actuelle aucune thérapeutique facilement administrable et agissant de façon systémique ciblant les mécanismes moléculaires de l'activation IgE-dépendante. En effet, la plupart des thérapeutiques "anti-allergiques" mises en oeuvre actuellement sont purement symptomatiques et visent à inhiber les effets délétères des médiateurs mastocytaires libérés lors de l'activation de ces cellules (anti-histaminiques, anti-leucotriènes, etc.).
Pour pouvoir étudier les mécanismes moléculaires liés à l'activation IgE-dépendante des mastocytes et pour mettre au point des méthodes de criblage de molécules capable d'inhiber spécifiquement cette activation, il faut pouvoir disposer de populations pures de mastocytes humains fonctionnels, idéalement en quantité importante. Une des possibilités consiste à purifier des mastocytes humains à partir de tissus. Bien que cela soit théoriquement possible, en pratique le rendement de ces techniques est faible (au maximum 1 à 3 millions de mastocytes) et les méthodes utilisées, en plus d'être coûteuses, nuisent à l'obtention de mastocytes fonctionnels.
Pour contourner cette difficulté, les inventeurs ont mis au point une technique de culture en milieu liquide en présence continue de Stem Cell Factor (SCF) pour obtenir des populations pures de mastocytes humains normaux en partant de cellules souches CD34+ de sang de cordon ombilical. Ces cellules CD34+ sont d'abord purifiées par immunoaffinité et mises en culture en milieu liquide en présence de SCF qui favorise leur différenciation en mastocytes. Au bout de 8 à 10 semaines de culture dans ces conditions, ils obtiennent des populations pures (plus d'environ 99%) de mastocytes qui ne prolifèrent plus et qui ne sont pas congelables. Ces cellules sont ensuite utilisées dans des tests d'activation IgE-dépendants. Ce type de culture primaire présente toutefois les inconvénients majeurs identifiés ci-dessous:
- il est nécessaire de mettre en culture de façon répétée des cellules CD34+ provenant de différents sangs de cordon de façon à pouvoir disposer de quantités suffisantes de mastocytes tout au long des expériences, ceci ayant de plus un coût élevé lié aux réactifs utilisés pour la purification des cellules CD34+,
- le rendement d'obtention des mastocytes en fin de culture est extrêmement variable d'un sang de cordon à un autre et ne dépasse jamais 200 millions de cellules au final,
- les caractéristiques morphologiques et fonctionnelles des mastocytes obtenus en fin de culture varient d'un sang de cordon à l'autre. Les expériences nécessitent donc d'être renouvellées avec plusieurs lots de mastocytes pour obtenir des résultats statistiquement significatifs.

Outre son rôle au cours des réactions allergiques et/ou inflammatoires, le mastocyte humain est par ailleurs impliqué dans un groupe de pathologies tumorales rares : les mastocytoses. Les mastocytoses constituent un groupe hétérogène d'affections caractérisées par l'accumulation ou la prolifération anormale de mastocytes dans différents organes ou tissus. Elles sont rares et qualifiées de "maladies orphelines" (incidence de 2/300000 patients/an), d'apparition le plus souvent sporadique mais parfois familiale, et surtout très hétérogènes quant à leur expression clinique, leurs modalités évolutives et leur pronostic. La physiopathologie de ces maladies demeure mal connue et les traitements sont peu spécifiques. La peau est le seul tissu atteint dans les mastocytoses cutanées, affections bénignes observées préférentiellement chez l'enfant, souvent spontanément résolutives. Les mastocytoses systémiques sont définies par l'atteinte d'un ou plusieurs viscères ou tissus, généralement la möelle osseuse, avec ou sans atteinte cutanée. Elles représentent 10 à 30% des mastocytoses, surviennent généralement chez l'adulte (âge moyen au diagnostic: 60 ans) sans prédilection de sexe, et s'associent relativement fréquemment à une hémopathie myéloïde.

L'étude du mécanisme physiopathologique des mastocytoses s'est d'abord orientée vers la recherche d'anomalies de synthèse relatives au ligand de KIT, le Stem Cell Factor (SCF). Les conclusions de ces études ont permis d'écarter cette hypothèse. Les équipes se sont alors intéressées directement au récepteur KIT. En effet, l'activation de KIT a été constatée en l'absence du ligand SCF dans des lignées de cellules mastocytaires (lignée HMC-1). Des cas de mutations activatrices de KIT ont également été observées dans des hémopathies myéloïdes.

Le récepteur KIT est un récepteur monocaténaire transmembranaire qui appartient à la famille des récepteurs à activité tyrosine kinase intrinsèque. Il comporte, au niveau intracytoplasmique, un domaine kinase 1, site de liaison de l'ATP, et un domaine kinase 2, site de l'activité phosphotransférase. Il est exprimé par différents types cellulaires : les mastocytes, les progéniteurs hématopoïétiques, les mélanocytes, les cellules germinales et les cellules interstitielles de Cajal. L'activation du récepteur KIT provoque sa dimérisation et sa phosphorylation. Les tyrosines phosphorylées servent de sites de liaison pour des molécules qui relaient la transduction du signal. Ainsi, différentes voies de signalisation sont activées et engendrent des signaux de prolifération, de survie ou d'activation cellulaire selon la réceptivité de la cellule.
Concernant la structure de KIT au cours des mastocytoses, les résultats des études ont montré les faits suivants :
- Chez les patients adultes atteints de mastocytose systémique indolente, on retrouve dans plus de 85% des cas la même mutation activatrice acquise de KIT, affectant l'acide aminé occupant la position 816 de la séquence d'acides aminés SEQ ID NO :2 du récepteur KIT sauvage (KIT WT) (mutation Asp816Val ou mutation D816V conduisant à la substitution de l'asparagine occupant la position 816 de SEQ ID NO :2 par une valine - voir SEQ ID NO :4 correspondant à la séquence en acides aminés du récepteur mutant KIT D816V), située au niveau du domaine phosphotransférase (correspondant aux acides aminés occupant les positions 762 à 937 de SEQ ID NO : 2). Les autres patients adultes présentent soit une structure normale du récepteur KIT WT (correspondant à SEQ ID NO :2), soit de rares anomalies ou mutations (délétions, insertions, etc.) pouvant se situer soit dans le domaine phosphotransférase, soit dans le domaine juxta-membranaire du récepteur KIT (correspondant aux acides aminés occupant les positions 543 à 582 de SEQ ID NO : 2). La plupart de ces anomalies, et en particulier KIT D816V, sont transformantes aussi bien *in vitro* sur lignées cellulaires qu'*in vivo* chez la souris.
- Chez les enfants, la situation est différente. Si plus de 80% des enfants présentent une anomalie de KIT (le reste présentant un KIT WT), la mutation KIT D816V n'est pas majoritaire chez ces patients. En effet, 36% des enfants présentent cette mutation tandis que 44% des enfants présentent des mutations particulières (du type délétion, substitution, duplication ou insertion d'un ou de plusieurs nucléotides) de l'exon 8 (correspondant aux nucléotides occupant les positions 1237 à 1352 de la séquence nucléotidique SEQ ID NO : 1 codant le récepteur KIT WT) ou de l'exon 9 (correspondant aux nucléotides occupant les positions 1353 à 1547 de la séquence nucléotidique SEQ ID NO : 1 codant le récepteur KIT WT de séquence SEQ ID NO :2), formant ensemble le domaine « Ig5-like », qui codent pour la partie extramembranaire du récepteur (correspondant aux acides aminés occupant les positions 26 à 519 de SEQ ID NO : 2), plusieurs de ces mutations particulières ayant été décrites dans d'autres types de pathologies malignes comme des leucémies aiguës myéloïdes ou des tumeurs gastro-intestinales stromales. Les mutations KIT Delta417-419 insY, KIT S4761, KIT ITD502-503, et KIT K509I sont des exemples de telles anomalies susceptibles d'affecter la séquence SEQ ID NO :2 du récepteur KIT sauvage. Là encore, ces anomalies sont transformantes *in vitro* et *in vivo.*

A l'heure actuelle, le traitement des mastocytoses, en particulier chez l'adulte, est décevant. En effet, si l'Imatinib a montré une certaine efficacité chez les patients présentant un KIT WT ou des anomalies de KIT au niveau juxta-membranaire, cette molécule est totalement inactive sur la mutation KIT D816V, la plus fréquemment rencontrée chez l'adulte. En outre bien que le Dasatinib, autre inhibiteur de tyrosine-kinases, ait une activité intéressante *in vitro* sur la mutation D816V, son passage en thérapeutique s'est avéré décevant du fait d'une moindre efficacité que prévue et d'une grande toxicité chez l'homme. Le traitement des formes agressives avec mutation KIT D816V fait donc actuellement appel à des chimiothérapies peu spécifiques et lourdes, non dénuées d'effets secondaires.
Il devient donc nécessaire de disposer de modèles cellulaires de mastocytes humains aisément manipulables, présentant la mutation activatrice de KIT D816V, qui permettent d'effectuer des tests de criblage, en particulier à haut débit, afin de rechercher et d'identifier des molécules capable de cibler spécifiquement et donc plus efficacement cette anomalie de KIT.

les lignées mastocytaires humaines actuellement disponibles sont les suivantes:
1) La lignée HMC-1 dérivée du sang périphérique d'un patient atteint de mastocytose. Outre le fait qu'il s'agit d'une lignée leucémique présentant de nombreuses autres anomalies moléculaires pouvant être impliquées dans la prolifération anormale de ces cellules (non retrouvées chez les patients atteints de mastocytose systémique), cette lignée présente des limitations majeures :
   - elle n'exprime absolument pas le récepteur FcepsilonR1 et n'est donc pas activable par ce biais (cf. Guhl et al., « Mast ce// lines HMC-1 and LAD2 in comparison with mature skin mast cells drastically reduced levels of tryptase and chymase in mast cell lines » ; Vol. 19, no. 9, septembre 2010, pages 845-847),
   - elle ne comprend pas de clone ne portant que la mutation KIT D816V (mutation responsable de l'activité tyrosine kinase spontanée du récepteur KIT, rencontrées chez plus de 85% des patients atteints de mastocytose systémique), et
   - elle ne comprend pas de clone n'exprimant que le récepteur KIT sauvage (KIT WT)
Ce modèle ne peut être utilisé ni pour le criblage de molécules à activité anti-allergique potentielle, ni pour étudier les interactions entre la signalisation induite par l'activation de KIT et la signalisation induite par l'activation de FcepsilonR1 (FcεR1) au cours des mastocytoses, ni pour mesurer une éventuelle toxicité non spécifique de KIT D816V de toute molécule ciblant potentiellement cette anomalie. 2) La lignée LAD-2 et ses sous-clones (cf. WO2003/065986 et Gibbs Bernhard et al.,
« Effects of Stem Cell Factor on Hypoxia-Inducible Factor 1 Alpha Accumulation in Human Acute Myeloid Leukaemia and LAD2 Mast Cells » ; vol. 6, no. 7, juillet 2011): il s'agit d'une lignée mastocytaire dérivée de la moelle osseuse d'un patient atteint d'un sarcome à mastocyte (mastocytose systémique) et qui exprime le récepteur de haute affinité aux IgE et le récepteur KIT WT. Cette lignée qui reste dépendante du SCF pour sa survie et sa prolifération est potentiellement intéressante mais, outre le fait qu'elle a été obtenue à partir d'un prélèvement tumoral, son temps de doublement très long de l'ordre de 15 jours la rend impropre à l'utilisation dans des tests de criblage à haut débit qui nécessitent l'obtention simultanée d'un grand nombre de cellules.
3) La lignée LUVA: il s'agit d'une lignée dérivée de cellules de sang périphérique d'un patient allergique. Elle est indépendante de tout facteur de croissance pour sa prolifération et présente le récepteur de haute affinité aux IgE. Elle est donc potentiellement intéressante mais là encore, c'est une lignée obtenue à partir d'un prélèvement effectué chez un patient non sain. En outre son temps de doublement long la rend aussi impropre à une utilisation dans le cadre d'un criblage *in vitro* à haut débit. Enfin, Cette lignée ne permet pas d'étudier la signalisation par le récepteur KIT WT et ne permet pas le criblage à haut débit de molécules inhibant spécifiquement ce récepteur.
4) La lignée USF-MC1 (WO2010/105215). Il s'agit d'une lignée SCF indépendante, dérivée de progéniteurs de sang de cordon, qui présente le récepteur de haute affinité aux IgE, mais a été transformée par le virus SV40. Il est donc impossible d'anticiper les effets de cette transformation sur la signalisation du récepteur de haute affinité des IgE. Cette lignée ne permet pas d'étudier la signalisation par le récepteur KIT WT et ne permet pas le criblage à haut débit de molécules inhibant spécifiquement ce récepteur.

Le seul modèle cellulaire de mastocytes humains, portant une mutation de KIT, disponible à l'heure actuelle est la lignée HMC-1 et ses deux sous clones HMC-1.1 et HMC-1.2. En effet, HMC-1, comme le sous-clone HMC-1.1, présente une mutation juxtamembranaire KIT V560G et est sensible à l'imatinib, tandis que le sous-clone HMC-1.2 présente deux mutations de KIT (V560G et D816V) et est sensible au Dasatinib mais pas à l'Imatinib. Les inconvénients majeurs de ces lignées sont les suivants:
1) il n'existe pas de clone ne portant que la mutation D816V permettant d'interpréter un éventuel effet sélectif d'une molécule sur la mutation D816V isolée.
2) il n'existe pas de clone n'exprimant que KIT WT permettant de mesurer une éventuelle toxicité non spécifique de KIT D816V d'une molécule ciblant potentiellement cette anomalie.
3) la lignée HMC-1 est une lignée leucémique présentant de nombreuses anomalies moléculaires autres que le KIT muté, pouvant être impliquées dans la prolifération anormale de ces cellules, et non retrouvées chez les patients atteints de mastocytose systémique.
4) ces lignées n'expriment pas le récepteur de haute affinité pour les IgE et interdisent donc toute analyse des éventuelles interactions entre les voies de signalisation de KIT et celles du FcepsilonR1.

Ainsi, à l'heure actuelle, les modèles cellulaires utilisables dans le contexte des allergies ou des pathologies malignes mentionnées ci-dessus présentent de nombreuses limitations, soit parce qu'ils sont peu reproductibles et coûteux à produire (modèles de cultures primaires), soit parce qu'ils s'éloignent du mastocyte en terme de propriétés biologiques.

### RESUME DE L'INVENTION

Les inventeurs décrivent à présent, et pour la première fois, une lignée de mastocytes humains exprimant le récepteur KIT sauvage ainsi que le récepteur de haute affinité des IgE (FcεR1), pouvant être activés par l'un et/ou l'autre de ces récepteurs, ayant un temps de doublement rapide (d'au plus 72 heures), i.e. compatible avec une production en masse (en vue de tests de criblage à haut débit), facilement congelables et décongelables, et dont les caractéristiques morphologiques, ultrastructurales, phénotypiques et fonctionnelles restent stables dans le temps. Les cellules de cette lignée, obtenues à partir de progéniteurs hématopoïétiques provenant du sang de cordon ombilical d'un sujet humain sain, présentent les caractéristiques les plus proches connues à ce jour des caractéristiques des mastocytes humains normaux.

Un objet particulier de la présente invention concerne ainsi une lignée cellulaire mastocytaire humaine présentant les caractéristiques morphologiques, ultrastructurales et phénotypiques suivantes : i) présence de granulations intra-cytoplasmiques métachromatiques, ii) expression des récepteurs FcεR1 et KIT sauvage (CD117), et des marqueurs CD33, CD203c et CD300a.

Un autre objet particulier de la présente invention concerne une lignée cellulaire mastocytaire humaine présentant les caractéristiques fonctionnelles suivantes : i) dépendance stricte vis-à-vis du SCF pour sa survie et sa croissance, ii), temps de doublement d'au plus 72 heures, typiquement de 48 heures, iii) expression du FcεR1 augmentée par traitement avec l'interleukine 4 (IL-4) et/ou avec des IgE humaines, iv) expression du CD117 diminuée par traitement avec l'interleukine 4 (IL-4), v) augmentation immédiate de l'expression membranaire de CD203c et/ou libération immédiate d'histamine, de bêta-hexosaminidase et de tryptase, et de manière retardée de TNF-α, par activation :
i) en présence d'IgE spécifique et de l'antigène capable de se lier à ladite IgE spécifique,
ii) en présence d'IgE et d'anti-IgE, ou
iii) en présence d'anticorps anti-récepteur FcεR1.

Un objet préféré de l'invention est une lignée cellulaire mastocytaire humaine présentant l'ensemble des caractéristiques morphologiques, ultrastructurales, phénotypiques et fonctionnelles identifiées ci-dessus.

La description concerne aussi toute lignée cellulaire dérivée, clone cellulaire ou mutant d'une lignée cellulaire mastocytaire humaine selon l'invention ayant conservé au moins une, de préférence plusieurs, idéalement l'ensemble des caractéristiques morphologiques, ultrastructurales, phénotypiques et/ou fonctionnelles identifiées ci-dessus.

Une lignée cellulaire mastocytaire humaine préférée selon l'invention est la lignée cellulaire mastocytaire humaine identifiée comme **ROSA KIT WT** telle qu'enregistrée sous le numéro de dépôt CNCM I-4551 auprès de la CNCM le 2 novembre 2011.

Les inventeurs décrivent en outre des clones cellulaires dérivés de la lignée ROSA présentant respectivement les mutations activatrices de KIT les plus fréquemment rencontrées dans les mastocytoses (KIT D816V et KIT Delta 417-419 insY), ainsi que des lignées cellulaires mastocytaires préférées, dérivées de la lignée mastocytaire humaine ROSA KIT WT, lesdites lignées dérivées étant respectivement identifiées comme **ROSA KIT D816V** (telle qu'enregistrée sous le numéro de dépôt CNCM I-4552 auprès de la CNCM le 2 novembre 2011) et **ROSA KIT Delta 417-419 insY** (telle qu'enregistrée sous le numéro de dépôt CNCM I-4553 auprès de la CNCM le 2 novembre 2011).

Sont également décrits les lignées cellulaires mastocytaires dérivées, ainsi que les clones cellulaires, et les mutants (naturels ou obtenus de manière artificielle) desdites lignées ROSA KIT WT, ROSA KIT D816V et Delta 417-419 insY, ayant conservé au moins une de leurs caractéristiques.

L'invention concerne en outre un procédé de préparation d'une population de mastocytes humains capables de proliférer pendant une période supérieure à 6 mois dont au moins 99% des cellules présentent les caractéristiques des cellules d'une lignée cellulaire mastocytaire humaine selon l'invention de type ROSA KIT WT, ainsi que toute population de mastocytes humains, clone cellulaire ou lignée cellulaire mastocytaire humaine susceptible d'être obtenue à l'issue d'un tel procédé. Ce procédé comprend la culture de précurseurs hématopoïétiques provenant du sang de cordon ombilical d'un sujet humain sain dans un milieu comprenant au moins 25 ng/ml, de préférence au moins 50 ng/ml, de Stem Cell Factor (SCF) humain, et l'obtention d'une population de mastocytes humains présentant les caractéristiques souhaitées.

L'invention concerne aussi un procédé de préparation d'une lignée cellulaire mastocytaire présentant une mutation du récepteur KIT, de préférence une mutation activatrice du récepteur KIT (responsable de l'activation du récepteur KIT de manière constitutive) associée à une pathologie choisie parmi une mastocytose, une leucémie aiguë, un lymphome et une tumeur solide, comprenant la transformation d'une lignée cellulaire mastocytaire humaine selon l'invention, typiquement d'une lignée cellulaire de type ROSA KIT WT, par introduction, dans les cellules de ladite lignée, d'un acide nucléique codant pour un récepteur KIT muté, de manière à obtenir une lignée cellulaire mastocytaire présentant ladite mutation de KIT.

Un objet particulier de l'invention concerne ainsi une lignée cellulaire mastocytaire susceptible d'être obtenue à l'issue du procédé décrit ci-dessus. Une telle lignée présente une mutation du récepteur KIT. Avantageusement, cette mutation peut être responsable i) de l'acquisition par la lignée d'une indépendance vis-à-vis du SCF pour sa survie et sa croissance ; ii) de l'acquisition par la lignée d'une tumorigénicité *in vivo* chez le mammifère, iii) d'une augmentation de la capacité de la lignée à libérer un médiateur de l'inflammation ou de l'allergie en présence d'IgE spécifique et de l'antigène capable de se lier à ladite IgE spécifique, en présence d'IgE et d'anti-IgE, ou en présence d'anticorps anti-récepteur FcεR1, ou par activation d'un récepteur de type TLR, d'un récepteur aux fractions du complément, ou d'un récepteur de cytokine ou de chimiokine ; iv) d'un changement, typiquement d'une activation, de la signalisation intra-mastocytaire dans la lignée ; et/ou v) d'une interaction dudit récepteur KIT muté ou de l'une au moins de ses voies de signalisation avec au moins un autre récepteur mastocytaire ou l'une au moins de ses voies de signalisation.
La description concerne aussi toute lignée cellulaire dérivée, clone cellulaire ou mutant d'une telle lignée cellulaire mastocytaire humaine selon l'invention présentant une mutation du récepteur KIT, ayant conservé au moins une, de préférence plusieurs, idéalement l'ensemble de ses caractéristiques morphologiques, ultrastructurales, phénotypiques et/ou fonctionnelles, de préférence de ses caractéristiques fonctionnelles.

D'autres objets particuliers de l'invention sont les cellules, clones cellulaires, populations de cellules et sous-populations de cellules provenant d'une lignée cellulaire mastocytaire selon l'invention telle que décrite dans le présent texte, ainsi que les compositions comprenant de tels objets.

L'invention concerne aussi un kit (ensemble d'outils) permettant le criblage d'un agent d'intérêt comprenant i) au moins un produit choisi parmi une lignée cellulaire mastocytaire, une cellule, un clone cellulaire, une population de cellules, une sous-population de cellules ou une composition selon l'invention, et de préférence ii) des instructions écrites a) expliquant les différentes étapes de la culture et/ou de la conservation des cellules, b) détaillant la composition du ou des milieux de culture et/ou du milieu de conservation des cellules et/ou listant un ou des facteurs de croissance susceptibles d'être utilisés dans le cadre de la culture et/ou de la conservation desdites cellules, et/ou c) détaillant les utilisations possibles desdits lignées, cellules, clones cellulaire, populations de cellules, sous-populations de cellules et compositions ; ainsi que éventuellement iii) un produit supplémentaire choisi parmi un ou plusieurs milieux de culture, un ou plusieurs milieux d'entretien, un ou plusieurs facteur de croissance permettant ou favorisant la culture des cellules mastocytaires, et toute combinaison desdits produits.

L'invention concerne par ailleurs un modèle animal non humain comprenant au moins une cellule provenant d'une lignée cellulaire mastocytaire selon l'invention, de préférence d'une lignée choisie parmi la lignée ROSA KIT D816V, la lignée ROSA KIT Delta 417-419 insY et une lignée de type ROSA KIT D816V ou ROSA KIT Delta 417-419 insY. Elle concerne également l'utilisation d'une telle lignée pour évaluer *in vivo* l'intérêt, en particulier l'intérêt préventif ou thérapeutique, d'une molécule candidate.

Les lignées cellulaires selon l'invention représentent un progrès en terme économique car elles sont faciles à manipuler et à amplifier à moindre coût.
Ces lignées cellulaires peuvent être utilisées comme modèles cellulaires en particulier pour cribler des molécules ciblant spécifiquement le récepteur de haute affinité aux IgE, la voie de signalisation induite par l'activation du récepteur de haute affinité aux IgE, le récepteur KIT (mormal ou muté), la voie de signalisation cellulaire induite par l'activation du récepteur KIT normal ou une voie de signalisation cellulaire anormale induite par l'activation d'un récepteur KIT muté (par exemple KIT D816V et KIT Delta 417-419 insY).

Ces lignées peuvent être utilisées pour cribler un agent d'intérêt, typiquement un agent utile à la prévention, au diagnostic, au traitement et/ou au suivi d'une pathologie, typiquement d'une pathologie dans laquelle les mastocytes jouent un rôle, par exemple un rôle délétère. La pathologie visée est de préférence choisie parmi une maladie allergie (par exemple asthme allergique) ; une maladie inflammatoire ; une maladie auto-immune ; une maladie infectieuse ; l'asthme non allergique ; un urticaire ; ou une tumeur, typiquement une mastocytose, une leucémie aigüe, un lymphome ou une tumeur solide.

### DESCRIPTION DETAILLEE DE L'INVENTION

L'invention concerne des **lignées de mastocytes humains** présentant ou non un récepteur KIT de structure normale et un récepteur FcεR1, ainsi que leur procédé d'obtention et leurs utilisations.
L'invention concerne également les **cellules, clones cellulaires** (également identifiés dans le présent texte par le terme « clone »), **populations de cellules ou sous-populations de cellules** provenant des lignées cellulaires mastocytaires décrites dans le présent texte. Sont également décrits toute lignée cellulaire dérivée obtenue à partir d'une telle cellule, d'une telle population, d'une telle sous-population, ou d'un tel clone cellulaire et tout mutant d'une lignée cellulaire décrite dans le présent texte conservant au moins une caractéristique de ladite lignée, de préférence plusieurs, typiquement toutes les caractéristiques de ladite lignée. Un tel mutant peut être généré de manière spontanée (mutation naturelle ou spontanée) ou peut être créé par l'homme de manière volontaire par manipulation génétique (mutation artificielle).
L'invention concerne par ailleurs une **composition** comprenant une cellule, un clone cellulaire, une population de cellules (par exemple une lignée) et/ou une sous-population de cellules telles que décrits dans le présent texte. Il peut s'agir par exemple d'une culture cellulaire, typiquement d'une **culture de mastocytes humains**, de préférence de mastocytes présentant les caractéristiques morphologiques, ultrastructurales, phénotypiques et/ou fonctionnelles décrites dans le présent texte.

Le terme « **culture** » désigne ici, de manière générale, une cellule ainsi qu'une population de cellules (ensemble de cellules) cultivées *in vitro.*
Une culture développée directement à partir d'un prélèvement cellulaire ou tissulaire est dénommée « **culture primaire** » et comprend généralement à une population hétérogène de cellules.
Le terme « **lignée** » désigne une population homogène de cellules obtenue après au moins une étape de sous-culture d'une culture primaire (étape également identifiée par le terme « **passage** » qui permet d'obtenir un grand nombre de cellules à partir de cellules préexistantes), typiquement après plusieurs étapes consécutives de sous-culture (ou passages consécutifs) de cellules provenant d'une population de cellules issue d'une culture primaire.
Le terme « **clone** » ou « **clone cellulaire** » désigne un ensemble de cellules issues d'une seule cellule, d'une culture primaire ou d'une lignée (SCHAEFFER. In Vitro Cellular and Developmental Biology, 26, 91-101, 1990) par exemple obtenu par la technique des dilutions limites de la lignée cellulaire.

L'invention concerne en outre un kit (ensemble d'outils) permettant le criblage *in vitro* d'un agent d'intérêt comprenant i) au moins un produit choisi parmi une lignée cellulaire mastocytaire, une cellule, un clone cellulaire, une population de cellules, une sous-population de cellules ou une composition selon l'invention, et de préférence ii) des instructions écrites a) expliquant les différentes étapes de la culture et/ou de la conservation des cellules, b) détaillant la composition du ou des milieux de culture et/ou du milieu de conservation des cellules et/ou listant un ou des facteurs de croissance susceptibles d'être utilisés dans le cadre de la culture et/ou de la conservation desdites cellules, et/ou c) détaillant les utilisations possibles desdits lignées, cellules, clones cellulaire, populations de cellules, sous-populations de cellules et compositions ; ainsi que éventuellement iii) un produit supplémentaire choisi parmi un ou plusieurs milieux de culture, un ou plusieurs milieux d'entretien, un ou plusieurs facteur de croissance permettant ou favorisant la culture des cellules mastocytaires, et toute combinaison desdits produits.

Un exemple de milieu de culture susceptible d'être proposé dans le kit est le suivant : milieu Iscove Modified Dulbecco Medium (IMDM)-Glutamax® (Invitrogen) supplémenté avec lesproduits suivants : Pénicilline/Streptomycine 100 U/ml (P/S) (Invitrogen), 1% de pyruvate de sodium (Invitrogen), 1% de vitamines (Invitrogen), 1% de Glutamine (Invitrogen), 2% d'acides aminés non essentiels (Invitrogen), 1% d'une solution commerciale d'insuline-transferrine-sélénite de sodium (Invitrogen) et 0,3% d'albumine bovine (BSA) (PAA).
Un milieu de conservation typique comprend : 90% de sérum de veau foetal et 10% de Diméthylsulfoxyde (DMSO).

Des facteurs de croissance susceptibles d'être utilisés dans le cadre de la culture et/ou de la conservation des cellules selon l'invention sont par exemple dans la liste constituée par le SCF et/ou l'IL-4.

Un kit préféré selon l'invention, utilisable dans les tests de criblage à haut débit de molécules pouvant inhiber l'activation du récepteur de haute affinité aux IgE, comprend par exemple :
a) au moins un produit choisi parmi la lignée cellulaire mastocytaire ROSA KIT WT, un produit issu de ladite lignée choisi parmi une cellule, un clone cellulaire, une population de cellules, une sous-population de cellules, ou une composition selon l'invention comprenant un produit choisi parmi ladite lignée, ladite cellule, ledit clone, ladite population et/ou ladite sous-population, et de préférence : b) des instructions écrites expliquant les différentes étapes de la culture et/ou de la conservation desdites cellules, détaillant la composition du ou des milieux de culture et/ou du milieu de conservation desdites cellules et/ou listant un ou des facteurs de croissance susceptibles d'être utilisés dans le cadre de la culture et/ou de la conservation desdites cellules, c) des instructions écrites décrivant les différentes étapes, et/ou identifiant des réactifs, permettant d'obtenir une activation du récepteur de haute affinité des IgE exprimé par les cellules de la lignée cellulaire mastocytaire ROSA KIT WT, et/ou d) l'identification des médiateurs à doser pour s'assurer que l'activation dudit récepteur de haute affinité des IgE a été effective.

Un autre kit préféré selon l'invention, utilisable dans les tests de criblage à haut débit de molécules visant à inhiber spécifiquement l'activité catalytique due récepteur KIT D816V, comprend par exemple :
a) au moins un produit 1 choisi parmi la lignée cellulaire mastocytaire ROSA KIT D816V, un produit issu de ladite lignée choisi parmi une cellule, un clone cellulaire, une population de cellules, une sous-population de cellules, ou une composition selon l'invention comprenant un produit choisi parmi ladite lignée, ladite cellule, ledit clone, ladite population et/ou ladite sous-population, et de préférence : b) au moins un produit 2 choisi parmi la lignée cellulaire mastocytaire ROSA KIT WT, un produit issu de ladite lignée choisi parmi une cellule, un clone cellulaire, une population de cellules, une sous-population de cellules, ou une composition selon l'invention comprenant un produit choisi parmi ladite lignée, ladite cellule, ledit clone, ladite population et/ou ladite sous-population, et c) au moins un produit 3 choisi parmi la lignée cellulaire mastocytaire ROSA KIT Delta 417-419 InsY, un produit issu de ladite lignée choisi parmi une cellule, un clone cellulaire, une population de cellules, une sous-population de cellules, ou une composition selon l'invention comprenant un produit choisi parmi ladite lignée, ladite cellule, ledit clone, ladite population et/ou ladite sous-population et de préférence d) des instructions écrites expliquant les différentes étapes de la culture et/ou de la conservation des cellules, détaillant la composition du ou des milieux de culture et/ou du milieu de conservation des cellules et/ou listant un ou des facteurs de croissance susceptibles d'être utilisés dans le cadre de la culture et/ou de la conservation desdites cellules, et de préférence e) des instructions écrites décrivant les différentes étapes et/ou identifiant des réactifs permettant de mesurer le degré de prolifération de chacun des 3 produits et permettant d'estimer la spécificité de l'inhibition de la prolifération obtenue en fonction de la nature du récepteur KIT porté par l'un et/ou l'autre des trois produits.

L'invention concerne aussi un modèle animal non **humain**, typiquement un modèle immunoinsuffisant, comprenant au moins une cellule ou au moins une population de cellules selon l'invention, de préférence une cellule ou population de cellules provenant d'une lignée cellulaire mastocytaire selon l'invention, par exemple d'une lignée cellulaire présentant une mutation du récepteur KIT, de préférence une mutation activatrice du récepteur KIT, de préférence une cellule provenant d'une lignée choisie parmi la lignée ROSA KIT D816V, la lignée ROSA KIT Delta 417-419 insY et une lignée de type ROSA KIT D816V ou ROSA KIT Delta 417-419 insY. L'invention concerne également un modèle animal non humain comprenant une tumeur formée par une cellule selon l'invention.
De tels modèles peuvent être avantageusement utilisés pour évaluer *in vivo* l'intérêt, en particulier l'intérêt préventif ou thérapeutique, d'une molécule candidate ; pour vérifier l'efficacité en termes de prévention ou de traitement thérapeutique des molécules identifiées à l'aide des outils cellulaires décrits dans la présente invention, utilisables *in vitro* ; ou encore pour vérifier *in vivo* l'éventuelle toxicité desdites molécules.

Les **mastocytes** sont des cellules du système immunitaire, qui interviennent dans la réponse inflammatoire, notamment dans les phénomènes d'allergie et d'hypersensibilité. Elles sont localisées dans le tissu conjonctif, notamment au niveau de la peau, des tractus digestif et respiratoire, et dans les muqueuses intestinales et respiratoires. Il existe également un petit nombre de mastocytes dans la moelle osseuse et dans les organes lymphoïdes.
Les mastocytes matures, quelle que soit leur localisation, ont en commun certaines caractéristiques telles que la présence de nombreuses granulations intra cytoplasmiques métachromatiques (i.e. aptes à transformer le bleu de toluidine en rouge violacé du fait de la présence d'héparine dans lesdites granulations). Il s'agit de cellules arrondies d'un diamètre de 13 à 22 mm ; elles possèdent un noyau arrondi, unique, central ou le plus souvent excentré. Le cytoplasme est entièrement rempli de granulations capables de recouvrir le noyau du fait de leur l'abondance.
Ces granulations renferment différentes substances chimiques synthétisées par les mastocytes, notamment l'histamine, la sérotonine, des protéoglycannes tels que l'héparine ou le chondroïtine sulfate, des enzymes, notamment des protéases, des cytokines telles que le TNF-alpha, et des facteurs "chimioattractants" des éosinophiles et des neutrophiles (ABRAHAM et MALAVIYA. Infection and Immunity, 65, 3501, 1997). Ces substances pro-inflammatoires sont libérées brutalement (phénomène dénommé « dégranulation ») lors de l'activation mastocytaire. Dans un deuxième temps se met en place une réponse secondaire, liée à la synthèse *de novo* de médiateurs tels que les leucotriènes, prostaglandines, PAF (platelet activating factor), mais aussi d'interleukines (IL4, IL5, IL6, IL10, IL12, IL13), de cytokines (TGF-beta, IFN-gamma, GM-CSF) et de chimiokines (MCP-1, IL8, RANTES) (MOQBEL et al. Immunology, 60, 425, 1987; BEFUS. Reg. Immunol., 2, 176, 1989). L'ensemble de ces facteurs participe activement à l'enclenchement d'un processus inflammatoire et à la mise en place d'une réponse immunitaire spécifique dépendante des lymphocytes T.
Les mastocytes constituent en fait une population cellulaire très hétérogène. En effet, deux sous-populations mastocytaires distinctes qui présentent des caractéristiques biochimiques et fonctionnelles très différentes ont été caractérisées : les mastocytes muqueux et les mastocytes séreux. Ces deux sous-populations peuvent être différenciées par les substances actives élaborées et stockées dans les granules. Ainsi, chez la souris, les mastocytes muqueux produisent principalement de l'histamine, de la chymase et du chondroïtine sulfate A et E, et les mastocytes séreux produisent principalement de l'histamine, de la sérotonine, de la chymase, de la tryptase et de l'héparine. Chez l'homme, les mastocytes muqueux également dénommés MCT (tryptase +) produisent principalement de l'histamine, de la tryptase, de l'héparine, et du chondroïtine sulfate A et E, et les mastocytes séreux également dénommés MCTC (tryptase+ et chymase+) produisent en outre de la chymase.

Les mastocytes sont dérivés de précurseurs hématopoïétiques (GALLI. Lab. Invest. 62, 5-33, 1990). Des populations de mastocytes peuvent être obtenues à partir de cultures de moelle osseuse de souris en présence de milieux conditionnés de lymphocytes T stimulés ou en présence d'interleukine-3 (IL-3) (Razin et al. E, J Immunol. 1984 Mar;132(3):1479-86). A partir d'une suspension de cellules hématopoïétiques de souris, on obtient ainsi par culture en présence d'IL-3 une population pure de mastocytes de type muqueux en 3 semaines. Les inventeurs ont en outre montré qu'il était possible d'obtenir des populations pures de mastocytes séreux chez la souris en cultivant les cellules du péritoine de ces animaux en présence de Stem Cell Factor (MALBEC et al. J Immunol 2007; 178(10):6465-75).

L'obtention de cultures *in vitro* de mastocytes humains s'est avérée plus difficile. Des cultures de cellules de moelle osseuse ou de cordon ombilical en présence d'IL-3 ont conduit à l'apparition de granulocytes basophiles (TADOKORO et al. J. Exp. Med., 158, 857-871, 1983). La co-culture de cellules de cordon ombilical humain avec des fibroblastes 3T3 de souris a toutefois permis d'obtenir des mastocytes matures après quatre semaines de culture (FURITSU et al. Proc. Natl. Acad. Sci. USA, 86, 10039-10043, 1989). Les inventeurs ont ensuite démontré que la différenciation et la prolifération des mastocytes humains *in vitro* dépend de la présence du Stem Cell Factor (SCF), ligand du récepteur KIT (VALENT. Immunol Tokay,15, 111-114. 1994). On peut donc obtenir des populations de mastocytes humains quasiment pures par culture primaire de progéniteurs CD34+ de moelle osseuse ou de sang de cordon pendant 8 à 10 semaines en présence de SCF humain (YOSHIKUBO et al. Exp Hematol. 2006; 34(3):320-9).

Les cultures de mastocytes constituent un outil utile pour l'étude des mécanismes impliqués dans différents phénomènes inflammatoires et/ou immunitaires, par exemple dans le cadre de la réponse allergique, ou de la réponse immunitaire à l'agression par divers pathogènes (VARADARADJALOU et al. Eur J Immunol. 2003 Apr;33(4):899-906). Cependant, comme indiqué précédemment, on n'a jusqu'à présent réussi à différencier et maintenir *in vitro* des cultures de mastocytes humains que sous forme de cultures primaires non immortalisées et qui ne peuvent être obtenues qu'après un temps de culture long et avec un coût d'obtention élevé, difficilement compatible avec une application au criblage à haut débit à la recherche de molécules d'intérêt. En outre, on ne dispose actuellement que d'un petit nombre de lignées de mastocytes humains, dont les caractéristiques les rendent impropres à l'application précitée (BUTTERFIELD et al. Leuk Res. 1988;12(4):345-55; KIRSHENBAUM et al. Leuk Res 2003; 27(8):677-82; LAIDLAW et al. J Allergy Clin Immunol. 2011; 127(3):815-22.e1-5).

Lors d'une culture primaire de mastocytes obtenus à partir de cellules CD34+ (précurseurs hématopoïétiques) de sang de cordon humain provenant d'un individu sain, les inventeurs sont parvenus à isoler et caractériser une nouvelle lignée mastocytaire humaine, identifiée en tant que « ROSA KIT WT » dans le cadre de la présente invention. Cette lignée présente des caractéristiques morphologiques, ultrastructurales, phénotypiques et fonctionnelles comparables à celles de mastocytes humains normaux.
La lignée ROSA KIT WT est en outre avantageusement négative pour les agents infectieux les plus courants: VIH, HbS, HbC et mycoplasmes.

Un objet particulier de l'invention concerne ainsi la lignée cellulaire mastocytaire humaine identifiée dans le présent texte en tant que « **ROSA KIT WT** » telle qu'enregistrée sous le numéro de dépôt CNCM I-4551 auprès de la CNCM (Collection Nationale de Culture de Microorganismes auprès de l'Institut Pasteur, 25, rue du Docteur Roux, F-75724 Paris Cedex 15, France) le 2 novembre 2011.
Comme indiqué précédemment, les inventeurs décrivent également toute cellule, tout clone cellulaire (par exemple obtenu par la technique des dilutions limites de la lignée cellulaire), toute population de cellules et toute sous-population de cellules issue de ladite lignée « ROSA KIT WT », ainsi que toute lignée cellulaire dérivée de ladite lignée « ROSA KIT WT » obtenue à partir d'une telle cellule, d'une telle population, d'une telle sous-population ou d'un tel clone cellulaire, conservant au moins une caractéristique de la lignée ROSA KIT WT, typiquement une caractéristique morphologique, ultrastructurale, phénotypique ou fonctionnelle, de préférence une caractéristique fonctionnelle, encore plus préférentiellement plusieurs desdites caractéristiques, de manière encore plus préférée toutes les caractéristiques de ladite lignée. La description concerne en outre tout mutant de la lignée « ROSA KIT WT » conservant au moins une caractéristique de la lignée ROSA KIT WT, typiquement une caractéristique morphologique, ultrastructurale, phénotypique ou fonctionnelle, de préférence plusieurs desdites caractéristiques, encore plus préférentiellement toutes les caractéristiques de ladite lignée.

Des cellules considérées comme dérivées d'une lignée cellulaire selon l'invention sont par exemples les cellules modifiées par transformation génétique, par exemple par mutagenèse, à l'aide de techniques connues de l'homme du métier.

La présente invention concerne aussi une lignée cellulaire mastocytaire humaine, identifiée dans le présent texte en tant que « **lignée de type ROSA KIT WT »,** présentant au moins l'une, de préférence plusieurs, des caractéristiques morphologiques, ultrastructurales, phénotypiques, ou fonctionnelles suivantes :
- phénotype mastocytaire
- présence de granulations intra-cytoplasmiques métachromatiques,
- présence de granulations intra-cytoplasmiques contenant de l'héparine,
- présence de granulations intra-cytoplasmiques contenant de l'histamine, de la tryptase, de bêta-hexosaminidase, et/ou de l'histidine décarboxylase (HDC),
- expression du récepteur KIT sauvage (également identifié en tant que CD117, KIT wild-type ou KIT WT dans le présent texte) et activation possible de la cellule mastocytaire par ce récepteur,
- expression du récepteur de haute affinité des IgE (également identifié en tant que FcεR1 ou FcepsilonR1 dans le présent texte) et activation possible de la cellule mastocytaire par ce récepteur,
- expression des marqueurs CD33, CD203c, CD300a, CD4 et/ou CD9 (caractéristiques du phénotype des mastocytes humains isolés à partir de différents tissus de sujets humains sains), de préférence des marqueurs CD33, CD203c et CD300a,
- dépendance vis-à-vis du Stem Cell Factor (SCF) pour sa survie et sa croissance, typiquement dans le cadre d'une culture continue,
- présente un temps de doublement court, d'au plus 72 heures, typiquement d'environ 48 heures, compatible avec l'obtention rapide d'un grand nombre de cellules (utilisables dans le cadre de méthode de criblage *in vitro* à haut débit de chimiotèques importantes) ;
- augmentation de l'expression du FcεR1 par traitement avec l'interleukine 4 (IL-4) et/ou avec des IgE humaines,
- diminution de l'expression du CD117 par traitement avec l'interleukine 4 (IL-4),
- augmentation immédiate de l'expression membranaire de CD203c et/ou libération immédiate d'histamine, de bêta-hexosaminidase et/ou de tryptase, et, de préférence de manière retardée de TNF-α, par activation de préférence :
   i) en présence d'IgE spécifique et de l'antigène capable de se lier à ladite IgE spécifique,
   ii) en présence d'IgE et d'anti-IgE,
   iii) en présence d'anticorps anti-récepteur FcεR1,
   iv) en présence d'un ligand d'un autre récepteur, choisi par exemple parmi un récepteur TLR (« Toll like Receptor »), tel que TLR-2, TLR-3, TLR-4, TLR-9 ; un récepteur pour une fraction du complément (par exemple un récepteur pour une fraction C3 et/ou une fraction C5 du complément) ; et un récepteur de cytokine ou de chimiokine, et/ou
   v) en présence d'un activateur non spécifique (tel que le ionophore calcique A23187).

Un objet préféré de l'invention est une lignée cellulaire mastocytaire humaine présentant l'ensemble des caractéristiques morphologiques, ultrastructurales, phénotypiques et fonctionnelles identifiées ci-dessus.

Un objet particulier de la présente invention concerne par ailleurs une lignée cellulaire mastocytaire humaine de type ROSA KIT WT, présentant au moins une, typiquement plusieurs, de préférence l'ensemble des caractéristiques morphologiques, ultrastructurales et/ou phénotypiques suivantes : i) présence de granulations intra-cytoplasmiques métachromatiques, ii) expression des récepteurs FcεR1 et KIT sauvage (CD117), et des marqueurs CD33, CD203c et CD300a.

Un autre objet particulier de la présente invention concerne une lignée cellulaire mastocytaire humaine de type ROSA KIT WT présentant les caractéristiques fonctionnelles suivantes : i) dépendance, de préférence dépendance stricte, vis-à-vis du SCF pour sa survie et sa croissance, ii), temps de doublement d'au plus 72 heures, de préférence d'environ 48 heures, iii) expression du FcεR1 augmentée par traitement avec l'interleukine 4 (IL-4) et/ou avec des IgE humaines, iv) expression du CD117 diminuée par traitement avec l'interleukine 4 (IL-4), v) augmentation immédiate de l'expression membranaire de CD203c et/ou libération immédiate d'histamine, de bêta-hexosaminidase et de tryptase, et de manière retardée de TNF-α, par activation :
i) en présence d'IgE spécifique et de l'antigène capable de se lier à ladite IgE spécifique,
ii) en présence d'IgE et d'anti-IgE, et/ou
iii) en présence d'anticorps anti-récepteur FcεR1.

La présente description concerne également, comme indiqué précédemment, toute lignée cellulaire dérivée de la lignée cellulaire mastocytaire humaine de type ROSA KIT WT décrite ci-dessus et tout clone cellulaire ou mutant de ladite lignée, lesdits lignée cellulaire dérivée, clone cellulaire et mutant ayant conservé au moins une, de préférence plusieurs (par exemple au moins une caractéristique phénotypique et au moins une caractéristique fonctionnelle), idéalement l'ensemble des caractéristiques morphologiques, ultrastructurales, phénotypiques et/ou fonctionnelles identifiées ci-dessus caractéristiques de ladite lignée cellulaire mastocytaire humaine de type ROSA KIT WT.

La lignée ROSA KIT WT et les lignées de type ROSA KIT WT présentent un récepteur KIT de structure normale, ont un temps de doublement court, d'environ 48 heures, sont congelables et décongelables de manière répétée à l'aide de méthodes classiques connues de l'homme du métier (la congélation-décongélation n'entraînant pas de modification morphologique et/ou fonctionnelle des cellules), et ont un coût de production faible, ce qui rend possible leur production en grandes quantités, et leur exploitation dans des tests de criblage à haut débit.
La lignée ROSA KIT WT et les lignées de type ROSA KIT WT de même que leurs clones (cf. clones isolés et analysés, identifiés par les inventeurs en tant que « ROSA 1 » à « ROSA 8 ») possèdent la capacité de proliférer dans des milieux de culture basiques (par exemple du RPMI-1640 avec 10% sérum de veau foetal) complémentés en cytokine, typiquement en SCF, tout en conservant leurs caractéristiques morphologiques, ultrastructurales, phénotypiques et fonctionnelles, ce qui réduit d'autant le coût de production des cellules en grande quantité.

La lignée ROSA KIT WT et les lignées de type ROSA KIT WT sont également avantageusement capables de maturer lors de la co-culture avec des sous-couches de cellules stromales, de préférence lors d'une co-culture à long terme (typiquement plus de 30 jours) avec de telles sous-couches. Les inventeurs ont ainsi cultivé la lignée ROSA KIT WT sur une sous-couche de cellules stromales de souris (lignée MS-5) et ont démontré que ce traitement induit une différenciation très importante des cellules de la lignée ROSA KIT WT, se traduisant par exemple par une augmentation très nette du nombre de granulations dans les cellules, associée à une diminutrion significative du rapport surface noyau/surface cellule.

Les cellules et lignées selon l'invention sont en outre, comme indiqué précédemment, avantageusement activables par le couple IgE-anti IgE ou par un couple IgE spécifiques-allergènes correspondants et peuvent donc être utilisées par exemple pour des études physiopathologiques des mécanismes de l'allergie et pour mettre au point et/ou mettre en oeuvre des tests de criblage à haut débit de molécules à la recherche de propriétés antiallergiques. L'activation des mastocytes par un tel couple conduit à une dégranulation avec libération immédiate typiquement d'histamine et de bêta-hexosaminidase, ladite libération étant classiquement associée à une augmentation importante de l'expression membranaire du CD203c, et à une libération retardée de TNF-alpha. Les différents événements de cette cascade peuvent aisément être mis en évidence et mesurés dans le cadre d'une méthode selon l'invention telles que décrites plus loin dans le présent texte, à l'aide de techniques connues de l'homme du métier. Les cellules et lignées selon l'invention sont par ailleurs activables par d'autres molécules capables de cibler spécifiquement ou non d'autres récepteurs membranaires des mastocytes, par exemple un ligand du récepteur TLR2 (tel que le peptidoglycane ou PGN), un ligand du récepteur TLR4 (tel que le lipopolysaccharide ou LPS), ou un activateur non spécifique (tel que le ionophore calcique A23187).

Un autre objet de l'invention concerne un procédé de préparation d'une population de mastocytes humains dont au moins 80% des cellules, de préférence au moins 95%, encore plus préférentiellement au moins 98%, de manière tout à fait préférée au moins 99%, idéalement 100% des cellules présentent les caractéristiques des cellules d'une lignée mastocytaire humaine selon l'invention, de préférence les caractéristiques suivantes :
i) prolifération possible pendant une période supérieure à six mois,
ii) renferment des granulations contenant de l'histamine, de la tryptase et/ou de l'héparine, et
iii) expriment le récepteur KIT sauvage (CD117 ou KIT WT) et le récepteur FcεR1.
Ce procédé comprend la mise en culture de précurseurs ou progéniteurs hématopoïétiques, de préférence de cellules souches, provenant du sang de cordon ombilical d'un sujet humain sain, dans un milieu de culture comprenant au moins 5 ng/ml, de préférence au moins 25 ng/ml, encore plus préférentiellement au moins 50 ng/ml, typiquement entre environ 50 ng/ml et environ 200 ng/ml, par exemple environ 60 ng/ml, 65 ng/ml, 70 ng/ml, 75 ng/ml, 100 ng/ml, 125 ng/ml, 150 ng/ml ou 175 ng/ml, de manière tout à fait préférée environ 80 ng/ml de Stem Cell Factor (SCF) humain pendant une durée suffisante pour obtenir la population de mastocytes humains présentant la ou les caractéristiques souhaitées.

Selon un mode de mise en oeuvre préféré de la présente invention, les cellules sont cultivées dans ce milieu pendant au moins 2 jours (48 heures), par exemple 72 heures, typiquement entre 30 et 100 jours, de préférence entre 50 à 70 jours pour obtenir une population pure (100% de mastocytes) ou substantiellement pure (99% de mastocytes) de mastocytes. Les cellules peuvent ensuite être maintenues en culture (« entretenues ») dans le même milieu aussi longtemps que souhaité par l'homme du métier.
Le procédé selon l'invention permet ainsi d'obtenir une population de mastocytes humains plus ou moins pure selon la durée de culture des cellules, de préférence une lignée cellulaire mastocytaire humaine. Les cellules ainsi obtenues conservent avantageusement les caractéristiques des mastocytes d'origine.
Un objet particulier de l'invention concerne ainsi une culture primaire de mastocytes obtenus à partir de précurseurs ou progéniteurs hématopoïétiques provenant du sang de cordon ombilical d'un sujet humain sain (« normal ») dont les mastocytes exprime un récepteur KIT normal (KIT WT), i.e., un récepteur fonctionnel présentant une structure normale.
Un tel procédé permet typiquement, par passages successifs en présence de SCF humain, de préparer la lignée ROSA KIT WT de mastocytes humains et les lignées de type ROSA KIT WT selon l'invention.

Selon un mode de réalisation particulier du procédé selon l'invention, il est possible d'obtenir des cellules mastocytaires qui expriment le récepteur de haute affinité des IgE (FcεR1) en nombre élevé en prévoyant, dans le procédé décrit précédemment, une étape de mise en contact des précurseurs hématopoïétiques avec de l'interleukine 4 (IL-4) (par exemple 20 ng/ml pendant quatre à cinq jours) et/ou des IgE monomériques (par exemple 10 µg/ml pendant quatre à cinq jours).
Le niveau d'expression des récepteurs FcεR1 peut être mesuré à l'aide de la méthode de cytométrie en flux, typiquement après immunomarquage avec un anticorps anti-FcεR1 associé à un fluorochrome tel que FITC (fluorescein isothiocyanate 1) ou APC (Allophycocyanine). L'élévation du nombre de récepteurs FcεR1 augmente le niveau de dégranulation des cellules stimulées :
i) en présence d'IgE spécifique et de l'antigène capable de se lier à ladite IgE spécifique,
ii) en présence d'IgE et d'anti-IgE, et/ou
iii) en présence d'anticorps anti-récepteur FcεR1,

L'invention couvre par ailleurs toute cellule, population de cellules, sous-population de cellules, lignée cellulaire mastocytaire humaine ou clone cellulaire susceptible d'être obtenue à l'issue d'un procédé selon l'invention.

Selon encore un autre mode de mise en oeuvre selon l'invention, le procédé comprend en outre une étape de transformation des cellules en culture par un acide nucléique responsable de l'expression par la cellule d'un récepteur KIT muté.
L'acide nucléique est de préférence un oncogène codant un récepteur KIT muté dont la fonction se trouve modifiée. Il s'agit typiquement d'un récepteur KIT présentant une structure anormale. La mutation peut inhiber la fonction normale de KIT ou au contraire l'activer, par exemple l'activer de manière constitutive. Il s'agit de préférence d'une mutation activatrice de KIT associée à une pathologie dans laquelle les mastocytes sont impliqués, par exemple à une pathologie dans laquelle les mastocytes ont un rôle délétère. Il s'agit typiquement d'une mutation activatrice du récepteur KIT associée à une pathologie choisie de préférence parmi une mastocytose, une leucémie aigüe, un lymphome et une tumeur solide.
L'oncogène peut être par exemple un oncogène codant pour le KIT muté en D816V ou pour le KIT muté Delta 417-419 insY (deux anomalies de KIT fréquemment rencontrées au cours des mastocytoses), respectivement dénommés « oncogène KIT D816V » et « oncogène KIT Delta 417-419 insY ». Un tel oncogène est immortalisant, i.e., il permet la culture des cellules transformées dans un milieu dépourvu de SCF.

L'invention concerne ainsi un procédé de préparation d'une lignée cellulaire mastocytaire présentant une mutation du récepteur KIT, comprenant la transformation d'une lignée cellulaire mastocytaire humaine selon l'invention, typiquement d'une lignée cellulaire de type ROSA KIT WT, par introduction, dans les cellules de ladite lignée, d'un acide nucléique codant pour un récepteur KIT muté, de manière à obtenir une lignée cellulaire mastocytaire présentant ladite mutation de KIT, typiquement à l'aide d'un vecteur d'expression. Avantageusement, ce procédé peut en outre comprendre une étape de sélection des cellules effectivement transformée (par exemple par mise en évidence de l'expression d'un gène rapporteur introduit en même temps que l'oncogène dans les cellules).

Dans un mode de réalisation préféré du procédé de l'invention, le vecteur d'expression est un vecteur rétroviral, de préférence un vecteur lentiviral. Ce vecteur peut être facilement choisi par l'homme du métier parmi les vecteurs permettant l'expression d'un transgène dans les cellules de mammifères. Le gène rapporteur peut également être facilement sélectionné par l'homme du métier parmi les gènes codant des marqueurs connus tels que par exemple la luciférase, la GFP (Green Fluorescent Protein) et ses dérivés tels que l'EGFP, les protéines émettant une fluorescence bleue (EBFP, EBFP2, Azurite, mKamala1), les protéines émettant une fluorescence cyan ou bleu clair (ECFP, Cerulean, CyPet), les protéines émettant une fluorescence jaune (YFP, Citrine, Venus, YPet), le DsRed et ses dérivés, le Keima et ses dérivés, la glucuronidase (GUS), la beta -Glucosidase, la phosphatase alcaline, la peroxidase du raifort (« horseradish peroxidase » ou HRP) et la beta-galactosidase (LacZ).
Un tel procédé permet d'obtenir une population de cellules, en particulier une lignée cellulaire mastocytaire, présentant une mutation de KIT. Lorsque la mutation est une mutation activatrice de KIT, le procédé permet d'obtenir des cellules mastocytaires exprimant un récepteur KIT activé de manière constitutive.

Un objet particulier de l'invention concerne ainsi une lignée cellulaire mastocytaire susceptible d'être obtenue à l'issue du procédé décrit ci-dessus. Une lignée cellulaire dérivée, un clone cellulaire ou un mutant de ladite lignée ayant conservé au moins une caractéristique de ladite lignée, typiquement une caractéristique morphologique, ultrastructurale, phénotypique ou fonctionnelle, de préférence plusieurs desdites caractéristiques, encore plus préférentiellement toutes les caractéristiques de ladite lignée, sont également décrits.

Une telle lignée présente une mutation du récepteur KIT. Avantageusement, cette mutation peut être responsable i) de l'acquisition par la lignée d'une indépendance vis-à-vis du SCF pour sa survie et sa croissance ; ii) de l'acquisition par la lignée d'une tumorigénicité *in vivo* chez le mammifère, iii) d'une augmentation de la capacité de la lignée à libérer un médiateur de l'inflammation ou de l'allergie en présence d'IgE spécifique et de l'antigène capable de se lier à ladite IgE spécifique, en présence d'IgE et d'anti-IgE, ou en présence d'anticorps anti-récepteur FcεR1, ou par activation d'un récepteur de type TLR, d'un récepteur de fraction du complément, ou d'un récepteur de cytokine ou de chimiokine ; iv) d'un changement, typiquement d'une activation, de la signalisation intra-mastocytaire dans la lignée ; et/ou v) d'une interaction dudit récepteur KIT muté ou de l'une au moins de ses voies de signalisation avec au moins un autre récepteur mastocytaire ou l'une au moins de ses voies de signalisation.
La description concerne aussi toute lignée cellulaire dérivée, clone cellulaire ou mutant d'une telle lignée cellulaire mastocytaire humaine selon l'invention présentant une mutation du récepteur KIT, ayant conservé au moins une, de préférence plusieurs, idéalement l'ensemble de ses caractéristiques morphologiques, ultrastructurales, phénotypiques et/ou fonctionnelles, de préférence de ses caractéristiques fonctionnelles.

Les inventeurs ont ainsi transfecté des cellules de la lignée ROSA KIT WT à l'aide de vecteurs lentiviraux apportant un acide nucléique de séquence SEQ ID NO : 3 codant pour le récepteur KIT muté en D816V (SEQ ID NO: 4) ou apportant un acide nucléique de séquence SEQ ID NO : 5 codant pour le récepteur KIT muté Delta 417-419 insY (SEQ ID NO : 6), et ont pu établir deux autres nouvelles lignées indépendantes du SCF pour leur prolifération, i.e. les lignées identifiées dans le présent texte en tant que **« ROSA KIT D816V** » et **« ROSA KIT Delta 417-419 insY** ». Ces lignées, particulièrement faciles à cultiver en grandes quantités, présentent un phénotype très proche de celui des mastocytes anormaux rencontrés au cours des mastocytoses.

Un objet particulier de l'invention concerne ainsi la lignée cellulaire mastocytaire humaine identifiée dans le présent texte en tant que « **ROSA KIT D816V** » telle qu'enregistrée sous le numéro de dépôt CNCM I-4552 auprès de la CNCM le 2 novembre 2011, dérivée de la lignée mastocytaire humaine ROSA KIT WT. Comme indiqué précédemment, la description concerne également toute cellule, tout clone cellulaire, toute population de cellules et toute sous-population de cellules issue de ladite lignée « ROSA KIT D816V », ainsi que toute lignée cellulaire dérivée de ladite lignée « ROSA KIT D816V » obtenue à partir d'une telle cellule, d'une telle population, d'une telle sous-population ou d'un tel clone, conservant au moins une caractéristique de la lignée ROSA KIT D816V, typiquement une caractéristique morphologique, ultrastructurale, phénotypique ou fonctionnelle, de préférence plusieurs desdites caractéristiques, encore plus préférentiellement toutes les caractéristiques de ladite lignée. La description concerne en outre tout mutant de la lignée « ROSA KIT D816V » conservant au moins une caractéristique de la lignée ROSA KIT D816V, typiquement une caractéristique morphologique, ultrastructurale, phénotypique ou fonctionnelle, de préférence plusieurs desdites caractéristiques, encore plus préférentiellement toutes les caractéristiques de ladite lignée.

La présente invention concerne aussi une lignée cellulaire mastocytaire humaine, dérivée de la lignée ROSA KIT WT, identifiée dans le présent texte en tant que « **lignée de type ROSA KIT D816V** », présentant au moins l'une, de préférence plusieurs, de manière encore plus préférée l'ensemble, des caractéristiques morphologiques, ultrastructurales, phénotypiques ou fonctionnelles suivantes :
- croissance totalement indépendante de la présence de cytokine, SCF en particulier,
- culture possible dans des milieux de culture peu onéreux, comme par exemple du RPMI-1640 contenant 10% de sérum de veau foetal,
- phénotype mastocytaire,
- expression simultanée des marqueurs CD33, CD203c et CD300a (caractéristiques du phénotype des mastocytes humains)
- expression du récepteur KIT présentant la mutation D816V (l'expression membranaire de KIT étant significativement plus élevée que dans la lignée parentale ROSA KIT WT),
- expression des ARN messagers de KIT D816V,
- expression d'un récepteur FcepsilonR1 fonctionnel (l'agrégation par le couple IgE-anti IgE induisant une surexpression membranaire du CD203c démontre le caractère fonctionnel du récepteur FcεR1),
- présence de granulations intra-cytoplasmiques contenant de l'histamine, de la tryptase, de la bêta-hexosaminidase, et/ou de l'histidine décarboxylase
- temps de doublement rapide d'au plus 72 heures, typiquement d'environ 48h,
- facilement congelable et décongelable à l'aide de méthodes classiques connues de l'homme du métier,
- capacité à former des colonies en milieu semi-solide, lesdites colonies, lorsqu'elles sont retransférées individuellement en milieu liquide, étant elles-mêmes capables de donner naissance à des sous-clones de la lignée parentale, et
- résistance à l'Imatinib et sensibilité au Dasatinib en terme d'inhibition de prolifération

Un autre objet particulier de l'invention concerne la lignée cellulaire mastocytaire humaine identifiée dans le présent texte en tant que **« ROSA KIT Delta 417-419 insY** » telle qu'enregistrée sous le numéro de dépôt CNCM I-4553 auprès de la CNCM le 2 novembre 2011, dérivée de la lignée mastocytaire humaine ROSA KIT WT. Comme indiqué précédemment, la description concerne également toute cellule, tout clone cellulaire, toute population de cellules et toute sous-population de cellules issue de ladite lignée « ROSA KIT Delta 417-419 insY », ainsi que toute lignée cellulaire dérivée de ladite lignée « ROSA KIT Delta 417-419 insY » obtenue à partir d'une telle cellule, d'une telle population, d'une telle sous-population ou d'un tel clone, conservant au moins une caractéristique de la lignée ROSA KIT Delta 417-419 insY, typiquement une caractéristique morphologique, ultrastructurale, phénotypique ou fonctionnelle, de préférence plusieurs desdites caractéristiques, encore plus préférentiellement toutes les caractéristiques de ladite lignée. La description concerne en outre tout mutant de la lignée « ROSA KIT Delta 417-419 insY » conservant au moins une caractéristique de la lignée ROSA KIT Delta 417-419 insY, typiquement une caractéristique morphologique, ultrastructurale, phénotypique ou fonctionnelle, de préférence plusieurs desdites caractéristiques, encore plus préférentiellement toutes les caractéristiques de ladite lignée.

La présente invention concerne aussi une lignée cellulaire mastocytaire humaine, dérivée de la lignée ROSA KIT WT, identifiée dans le présent texte en tant que « **lignée de type ROSA KIT Delta 417-419 insY** », présentant au moins l'une, de préférence plusieurs, encore plus préférentiellement l'ensemble, des caractéristiques morphologiques, ultrastructurales, phénotypiques ou fonctionnelles suivantes :
- croissance totalement indépendante de la présence de cytokine, SCF en particulier,
- culture possible dans des milieux de culture peu onéreux, comme par exemple du RPMI-1640 contenant 10% de sérum de veau foetal,
- phénotype mastocytaire,
- expression simultanée des marqueurs CD33, CD203c et CD300a (caractéristiques du phénotype des mastocytes humains)
- expression du récepteur KIT présentant la mutation Delta 417-149 insY (l'expression membranaire de KIT étant significativement plus élevée que dans la lignée parentale ROSA KIT WT),
- expression des ARN messagers de KIT Delta 417-149 insY,
- expression d'un récepteur FcepsilonR1 fonctionnel (l'agrégation par le couple IgE-anti IgE induisant une surexpression membranaire du CD203c démontre le caractère fonctionnel du récepteur FcεR1),
- présence de granulations intra-cytoplasmiques contenant de l'histamine, de la tryptase, de la bêta-hexosaminidase, et/ou de l'histidine décarboxylase,
- temps de doublement rapide d'au plus 72 heures, typiquement d'environ 48h,
- facilement congelable et décongelable à l'aide de méthodes classiques connues de l'homme du métier,
- capacité à former des colonies en milieu semi-solide, lesdites colonies, lorsqu'elles sont retransférées individuellement en milieu liquide, étant elles-mêmes capables de donner naissance à des sous-clones de la lignée parentale, et
- sensibilité à l'Imatinib et au Dasatinib en terme d'inhibition de prolifération.

Les lignées« ROSA KIT D816V », « ROSA KIT Delta 417-419 insY » ainsi que les lignées de type ROSA KIT D816V ou ROSA KIT Delta 417-419 insY prolifèrent de manière autonome et peuvent avantageusement être cultivées dans un milieu dépourvu de SCF comme indiqué ci-dessus.

D'autres méthodes, distinctes de la transfection par vecteur lentiviral et connues de l'homme du métier, peuvent être mises en oeuvre pour obtenir la transformation les cellules telles que l'électroporation (Neumann E et al (1982). EMBO J. 1 (7): 841-5) ou la lipofection (Felgner PL et al. (1987) Proc Natl Acad Sci U S A 84: 7413-7417).

L'invention concerne aussi un procédé d'obtention ou d'isolement de cellules clonales comprenant la dilution limite d'une lignée cellulaire selon l'invention.

L'invention concerne en outre des clones cellulaires dérivés de la lignée ROSA présentant respectivement les mutations activatrices de KIT les plus fréquemment rencontrées dans les mastocytoses (KIT D816V et KIT Delta 417-419 insY).

Sont également considérés comme des objets de la présente description les lignées cellulaires mastocytaires dérivées, ainsi que les clones cellulaires, et les mutants (naturels ou obtenus de manière artificielle) desdites lignées ROSA KIT WT, ROSA KIT D816V et Delta 417-419 insY, ayant conservé au moins une de leurs caractéristiques, de préférence plusieurs, de manière encore plus préférée l'ensemble de leurs caractéristiques.

L'invention concerne par ailleurs un modèle animal non humain comprenant au moins une cellule provenant d'une lignée cellulaire mastocytaire selon l'invention, de préférence d'une lignée choisie parmi la lignée ROSA KIT D816V, la lignée ROSA KIT Delta 417-419 insY et une lignée de type ROSA KIT D816V ou ROSA KIT Delta 417-419 insY. Elle concerne également l'utilisation d'une telle lignée pour évaluer *in vivo* l'intérêt, en particulier l'intérêt préventif ou thérapeutique, d'une molécule candidate.

Les cellules selon l'invention représentent un progrès en terme économique car elles sont faciles à manipuler, à cultiver et à amplifier en très grandes quantités en utilisant des réactifs et matériels peu onéreux. Elles sont avantageusement utilisables en particulier en recherche ou dans le cadre de méthodes de criblage à haut débit de molécules d'intérêt. Les modèles animaux selon l'invention qui comprennent de telles cellules sont eux-mêmes avantageusement utilisables notamment pour vérifier l'efficacité des molécules criblées sur la prévention ou le traitement des pathologies dans lesquelles les mastocytes jouent un rôle, en particulier exercent un rôle délétère (mastocytoses par exemple).

Les cellules et lignées cellulaires selon l'invention peuvent être utilisées comme outil de recherche, en particulier comme modèles cellulaires.
Elles présentent un intérêt tout particulier en tant que modèle *in vitro* pour l'étude des mécanismes cellulaires et/ou intracellulaires qui sont impliqués dans l'activation de ces cellules. Les cellules mastocytaires selon l'invention peuvent ainsi être utilisées pour tester *in vitro* la capacité d'activation mastocytaire d'un agent ou d'une molécule d'intérêt ou au contraire sa capacité à inhiber l'activation mastocytaire, en particulier l'activation IgE-dépendante desdites cellules médiée par le récepteur de haute affinité des IgE, ou l'activation mastocytaire médiée par le récepteur KIT, à l'aide de technique connues de l'homme du métier. Il est également possible d'utiliser ces cellules pour tester la capacité d'un agent ou d'une molécule d'intérêt à moduler (i.e. activer, inhiber ou modifier) la tumorigénicité, la clonogénicité, la survie, l'apoptose, la prolifération, la différentiation, l'activation, la fonction, le phénotype, l'aspect morphologique et/ou ultrastructural de mastocytes humains.
Les cellules mastocytaires et lignées selon l'invention peuvent ainsi être utilisés pour étudier l'activation mastocytaire en réponse à une substance choisie par exemple parmi un allergène, un microorganisme (par exemple une bactérie, un virus et/ou un parasite) et un produit dérivé de l'un de ces microorganismes.

Les cellules et lignées cellulaires selon l'invention peuvent aussi être utilisées par exemple dans le cadre d'études physiopathologiques portant en particulier sur les pathologies mentionnées dans la présente description, ou pour le criblage à haut débit de molécules d'intérêt. Elles peuvent être utilisées en particulier pour cribler des molécules ciblant spécifiquement le récepteur de haute affinité aux IgE, la voie de signalisation induite par l'activation du récepteur de haute affinité aux IgE, le récepteur KIT (mormal ou muté), la voie de signalisation cellulaire induite par l'activation du récepteur KIT normal ou une voie de signalisation cellulaire anormale induite par l'activation d'un récepteur KIT muté (par exemple KIT D816V et KIT Delta 417-419 insY), dans ce dernier cas par exemple pour identifier des molécules présentant des propriétés antiprolifératives.
Les cellules et lignées de l'invention peuvent aussi être utilisées pour cribler un agent ou une molécule d'intérêt utile à la prévention, au diagnostic, au traitement et/ou au suivi d'une pathologie, typiquement d'une pathologie dans laquelle les mastocytes jouent un rôle bénéfique ou délétère.
La pathologie visée est de préférence choisie parmi une allergie, une maladie inflammatoire, une maladie auto-immune, une infection, l'asthme non allergique, un urticaire, et une tumeur, typiquement une mastocytose, une hémopathie myéloïde, par exemple une leucémie aigüe myéloïde, un lymphome et une tumeur solide, par exemple une tumeur gastrointestinale stromale (GIST).
Les cellules mastocytaires selon l'invention peuvent aussi par exemple être utilisées dans une méthode selon l'invention de criblage d'antigène vaccinaux ou de réactifs de diagnostic. Elles peuvent aussi être utilisées pour identifier de nouveaux biomarqueurs permettant de diagnostiquer une pathologie dans laquelle les mastocytes ont un rôle bénéfique ou délétère, ou de nouveaux biomarqueurs signalant la sensibilité ou au contraire la résistance à une molécule utilisée dans le traitement ou la prévention d'une telle pathologie.

L'identification de tels biomarqueurs peut être réalisée par analyse du profil d'expression de cellules sensibles à une molécule connue particulière, de cellules différentes résistantes à la même molécule et/ou de cellules résistantes à différentes molécules connues. L'identification peut aussi être réalisée par RT-PCR, Western Blot, Immunohistochimie, etc.

La molécule d'intérêt peut être une molécule ayant un effet cytotoxique ou cytostatique sur les cellules. Alternativement, il peut s'agir d'une molécule augmentant l'efficacité thérapeutique d'une autre molécule utilisée comme médicament, d'une molécule augmentant ou restaurant la sensibilité des cellules à une molécule à laquelle elles sont résistantes, d'une molécule prévenant, réduisant ou retardant l'apparition d'une telle résistance, ou encore d'une molécule permettant un traitement alternatif par rapport aux traitements existants.

La molécule d'intérêt peut être une molécule naturelle, recombinante ou synthétique.
La molécule susceptible d'être testée peut être choisie par exemple parmi une molécule chimique, un polypeptide, une protéine, un acide nucléique (par exemple un siRNA, un ribozyme, etc.) et/ou un anticorps. Différentes doses de la molécule peuvent être testées à l'aide d'une même méthode.

Les cellules mastocytaires selon l'invention peuvent en outre être utilisées pour tester *in vitro* l'impact d'un traitement thérapeutique choisi parmi une exposition à un rayonnement (par exemple une radiothérapie), une chimiothérapie, une immunothérapie, une thérapie génique, et toute combinaison de ces traitements.

Les méthodes selon l'invention décrites ci-dessous qui mettent en oeuvre des cellules ou populations cellulaires selon l'invention, en particulier des lignées cellulaires selon l'invention, illustrent des exemples d'applications possibles des produits selon l'invention.

Chaque méthode décrite dans le présent texte peut évidemment comprendre, lorsque le texte ci-dessous ne l'indique pas, une étape d'application, à des cellules témoins, du traitement décrit pour les cellules étudiées à des fins de comparaison, cette étape étant réalisée à l'identique mais en l'absence de la molécule à tester lorsqu'il s'agit d'un témoin ou contrôle négatif, ou en l'absence d'une molécule connue pour son efficacité vis-à-vis du paramètre à tester lorsqu'il s'agit d'un témoin ou contrôle positif.

Une méthode selon l'invention permet d'évaluer la capacité d'au moins une molécule candidate à moduler la tumorigénicité, la clonogénicité, la survie, l'apoptose, la prolifération, la différentiation, l'activation, la fonction, le phénotype, l'aspect morphologique et/ou ultrastructural de mastocytes humains. Cette méthode comprend :
a) la mise en contact d'une cellule, d'un clone cellulaire, d'une lignée cellulaire, d'une population de cellules mastocytaires, d'une sous-population de cellules ou d'une composition selon l'invention, typiquement d'une lignée cellulaire mastocytaire, avec au moins une molécule candidate telle que définie précédemment ; et
b) la détermination, à l'aide de techniques connues de l'homme du métier, de la tumorigénicité, la clonogénicité, la survie, l'apoptose, la prolifération, la différentiation, l'activation, la fonction, le phénotype, l'aspect morphologique et/ou ultrastructural des mastocytes appartenant à ladite lignée, cellule, clone cellulaire, population de cellules, sous-population de cellules, ou composition, exposés à ladite au moins une molécule candidate, ladite détermination permettant d'évaluer la capacité correspondante de ladite au moins une molécule candidate à moduler la tumorigénicité, la clonogénicité, la survie, l'apoptose, la prolifération, la différentiation, l'activation, la fonction, le phénotype, l'aspect morphologique et/ou ultrastructural de mastocytes humains.

La détermination de la tumorigénicité des mastocytes peut être réalisée par exemple par injection d'une ou de plusieurs de ces cellules par voie sous cutanée ou intraveineuse à des souris immunodéprimées (souris SCID, NOD-SCID et/ou NSG) et préalablement irradiées de façon sub-léthale. Selon le type d'injection, les souris sont sacrifiées soit lorsqu'apparaît une tumeur sous-cutanée au point d'injection, soit lorsque l'état général de la souris s'altère (cas de l'injection intraveineuse). La nature mastocytaire de la prolifération peut ensuite être confirmée par marquage spécifique en immuno-histochimie ou en immuno-cytochimie des cellules tumorales (positivité pour la tryptase et le CD45 humain) soit au niveau des cellules de la tumeur sous-cutanée (en cas d'injection sous-cutanée) soit au niveau des cellules médullaires (en cas d'injection intra-veineuse).
La détermination de la clonogénicité des mastocytes peut être réalisée par exemple par culture d'un nombre déterminé de ces cellules en milieu-semi-solide à base de méthylcellulose ou d'Agar en boite de Pétri, et mesure du nombre de clones ou de colonies obtenus après un temps d'incubation variable (préférentiellement entre 7 et 21 jours) à l'étuve à 37°C sous 5% de CO2 dans l'air.
La détermination de la survie, de l'apoptose et/ou de la prolifération des mastocytes peuvent être réalisée respectivement par détermination du pourcentage de cellules vivantes après coloration par un colorant vital comme le bleu trypan ; par détermination cytofluorimétrique du pourcentage de cellules apoptotiques ayant fixées l'annexine V marquée par FITC ; et par détermination de la réduction des sels de tétrazolium ou technique au MTT (Le jaune de tétrazolium MTT (3 - (4, 5-dimethylthiazolyl-2) -2,5-diphényltétrazolium) est réduit dans de cellules métaboliquement actives, donc qui prolifèrent, en partie par l'action d'enzymes de type déshydrogénase. Le formazan intracellulaire résultant donne un précipité pourpre pouvant être solubilisé et quantifié par mesure spectrophotométrique).
La détermination de la différentiation des mastocytes peut être évaluée par mise en évidence de marqueurs de la différentiation des mastocytes humains tels que l'augmentation du nombre de granulations intra cytoplasmiques métachromatiques, l'augmentation de l'expression du récepteur de haute affinité des IgE (FcεRI) et/ou l'apparition d'une positivité pour la chymase (i.e. production de chymase par les mastocytes).
La détermination de l'activation des mastocytes peut être évaluée par mise en évidence de la libération de médiateurs tels que définis plus bas dans la description.
La modulation de la fonction des mastocytes peut être mise en évidence par exemple, mais pas uniquement, par la capacité de ces cellules à englober et à détruire des bactéries ou des virus, ou par la capacité de ces cellules à présenter l'antigène à d'autres cellules du système immunitaire à l'aide de techniques connues de l'homme du métier.
La détermination du phénotype et de l'aspect morphologique des mastocytes peut être réalisée par microscopie optique ou électronique et la détermination de l'utltrastructure des mastocytes par microscopie électronique.

Une autre méthode selon l'invention permet de déterminer la capacité d'une molécule candidate à interférer avec la liaison d'au moins un ligand ou d'au moins un substrat à au moins un récepteur de mastocyte humain ou à au moins un autre substrat mastocytaire ; à moduler (i.e. activer, inhiber ou modifier comme indiqué précédemment) la transduction d'au moins un signal dans un mastocyte humain ; et/ou à moduler la synthèse et/ou la libération d'au moins un médiateur par un mastocyte humain. Cette méthode comprend :
a) la mise en contact d'une cellule, d'un clone cellulaire, d'une lignée cellulaire, d'une population de cellules mastocytaires, d'une sous-population de cellules ou d'une composition selon l'invention, typiquement d'une lignée cellulaire mastocytaire, avec au moins une molécule candidate telle que définie précédemment, et
b) la mise en évidence ou la mesure i) d'une liaison d'au moins un ligand ou d'au moins un substrat à au moins un récepteur de mastocyte humain ou à au moins un autre substrat mastocytaire, ii) de la synthèse ou de la libération d'au moins un médiateur par un mastocyte humain, et/ou iii) de la transduction d'au moins un signal dans les mastocytes de ladite lignée, de la cellule, du clone cellulaire, de la population de cellules, de la sous-population de cellules, ou de la composition, de manière à déterminer respectivement i) la capacité de ladite au moins une molécule candidate à interférer avec la liaison d'au moins un ligand ou d'au moins un substrat à au moins un récepteur présent à la surface d'un mastocyte humain ou à au moins un autre substrat mastocytaire, ii) à moduler la synthèse ou la libération d'au moins un médiateur par un mastocyte humain et/ou iii) à moduler la transduction d'au moins un signal dans un mastocyte humain.

Par « **récepteur de mastocyte humain** » on entend les récepteurs présents à la surface des mastocytes ainsi que les récepteurs présents dans les mastocytes qui sont impliqués dans la transmission d'au moins un signal intracellulaire initié par l'activation du récepteur. Des récepteurs présents à la surface des mastocytes sont par exemple FcεR1, KIT WT, FcyR1, TLR2, TLR3, TLR4, CA₃R, EP_{3/4}, A₃R, CCR1 et CCR1/5. Il peut également s'agir d'un récepteur muté, par exemple un récepteur KIT muté.
Des récepteurs présents à l'intérieur des mastocytes sont par exemple, mais pas uniquement, des récepteurs cytoplasmiques capables de lier spécifiquement une hormone stéroïde telle que par exemple la testostérone, l'oestradiol, la progestérone, la cortisone, ou l'aldostérone ; une hormone thyroïdienne ; la vitamine D3 ; et les dérivés de l'acide rétinoïque.
Une méthode préférée selon l'invention permet de déterminer la capacité d'une molécule candidate à interférer avec la liaison d'au moins un ligand ou d'au moins un substrat à au moins un récepteur choisi par FcεR1, KIT WT, et un récepteur KIT muté tel que décrit dans la présente demande.

Par « **ligand** » on entend toute molécule, de quelque nature chimique que ce soit, capable de se lier spécifiquement à un récepteur mastocytaire membranaire (par exemple mais pas uniquement l'IgE qui se lie à son récepteur de haute affinité le FcεRI ; ou le Stem Cell Factor qui se lie à son récepteur spécifique KIT) ou cytoplasmique (par exemple, mais pas uniquement, l'acide rétinoïque tout-trans qui se lie à son récepteur RARalpha, ou la vitamine D3 qui se lie à son récepteur VD3R) et capable d'induire par sa liaison un changement de conformation, une activation, une inhibition, une surexpression, une sous-expression et/ou une dégradation de ce récepteur..

Par « **substrat mastocytaire** » on entend toute molécule, de quelque nature chimique que ce soit, présente à l'intérieur, à l'extérieur ou dans la membrane du mastocyte et capable d'être activée directement ou indirectement après liaison d'un ligand à un récepteur mastocytaire membranaire, intracytoplasmique ou nucléaire (par exemple, mais pas uniquement, la molécule protéique Syk, substrat du récepteur FcεRI et qui est recrutée quand ce récepteur est activé, ou la molécule STAT-5, substrat de la molécule JAK2, elle-même recrutée et activée lors de l'activation du récepteur KIT).

Par « **signal** » on entend tout évènement intracellulaire, membranaire ou extracellulaire engendré par l'activation d'un récepteur mastocytaire membranaire et/ou intracellulaire et induisant la différenciation, la prolifération, l'apoptose, l'activation et/ou la migration du mastocyte.

Le terme « **médiateur** » désigne toute substance, de quelque nature chimique que ce soit, stockée dans les granulations ou le cytoplasme (histamine, tryptase, HDC) ou néo-synthétisée (prostaglandine, leucotriène, cytokines comme le TNF-alpha et/ou chimiokines comme l'Interleukine-8) par le mastocyte, et libérée dans le cytoplasme, la membrane ou le milieu environnant du mastocyte, spontanément ou après activation de cette cellule.

La mise en évidence ou la mesure d'une liaison d'un ligand ou d'un substrat à un récepteur de mastocyte humain ou à un autre substrat mastocytaire peut être réalisée par exemple par utilisation d'un ligand, d'un substrat radioactif, d'un substrat fluorescent, ou d'un substrat marqué avec une enzyme ; et/ou par immunoprécipitation coordonnée du ligand et/ou du substrat et du récepteur et/ou d'un autre substrat ou d'un autre ligand.
La mise en évidence ou la mesure de la synthèse ou de la libération d'un médiateur par un mastocyte humain peut être réalisée par exemple par mesure directe du médiateur à l'intérieur ou à l'extérieur du mastocyte en utilisant une technique d'immuno-marquage ou une technique immuno-enzymatique (ELISA) spécifiques du médiateur, ou une technique de chromatographie en phase liquide ou en phase gazeuse.
La mise en évidence ou la mesure de la transduction d'un signal dans les mastocytes peut être réalisée par exemple par la technique de western-blot utilisant un ou des anticorps spécifiques d'un ou plusieurs substrats intracytoplasmiques ou intranucléaires. Ces anticorps peuvent reconnaitre le ou les substrats sous forme native, ou sous forme activée (par exemple, mais pas uniquement, sous forme phosphorylée). Une telle technique permet d'une part de mesurer le niveau d'expression dudit ou desdits substrats sous forme native et de comparer ce(ces) niveau(x) entre cellules traitées différemment, mais aussi de mesurer le niveau d'activation dudit ou desdits substrats et de comparer ce(ces) niveau(x) entre cellules traitées différemment.

Dans le cas de l'allergie par exemple, les cellules selon l'invention et plus particulièrement les cellules issues de la culture primaire ROSA KIT WT, peuvent être utilisées dans des tests de criblage à haut débit de bibliothèques de molécules candidates (ou molécules test), à la recherche de molécules inhibant l'activation IgE-dépendantes des dites cellules.

Une autre méthode selon l'invention permet de déterminer la capacité d'une molécule candidate à augmenter ou à diminuer la survie et/ou la prolifération, et/ou à inhiber ou induire l'apoptose des mastocytes humains. Cette méthode comprend :
a) la mise en contact d'une cellule, d'un clone cellulaire, d'une lignée cellulaire, d'une population de cellules mastocytaires, d'une sous-population de cellules ou d'une composition selon l'invention, typiquement d'une lignée cellulaire mastocytaire, avec au moins une molécule candidate telle que définie précédemment ; et
b) la détermination du niveau de la survie et/ou de la prolifération et/ou de l'apoptose des mastocytes de ladite lignée, de la cellule, du clone cellulaire, de la population de cellules, de la sous-population de cellules, ou des cellules au sein de la composition, une augmentation ou une diminution de la survie et/ou de la prolifération et/ou l'inhibition ou l'induction de l'apoptose desdites cellules déterminant la capacité de la au moins une molécule candidate respectivement à augmenter ou diminuer la survie et/ou la prolifération et/ou à inhiber ou induire l'apoptose des mastocytes humains.

La détermination ou la mesure du niveau de la survie peut être réalisée par décompte du pourcentage de cellules vivantes après utilisation d'un colorant vital comme le bleu trypan. La détermination ou la mesure de la prolifération des mastocytes selon l'invention peut être réalisée par mesure directe de la taille d'une tumeur ou par mesure indirecte à l'aide d'un marqueur fluorescent comme la GFP par exemple, dont l'intensité lumineuse sera proportionnelle à la taille de la tumeur, et qui pourra être suivie chez l'animal par imagerie en temps réel du site de la tumeur et/ou du corps entier.
La détermination ou la mesure de l'apoptose des mastocytes selon l'invention peut être réalisée par mesure de la fixation de l'annexine V. La perte de l'asymétrie membranaire, mesurée par la liaison de l'Annexine V aux phosphatidylsérines, peut être détectée au moyen du kit APOPTESTTM-Fluos (Dako, France) : après exposition, 10⁶ cellules sont incubées (10-15 min.) dans 100 µl de solution de marquage froide (1 µl d'Annexine-V/FITC et 2,5 µl d'iodure de propidium (IP) 250 µg/ml) dans 96 µl de tampon de marquage. Ensuite, 250 µl de tampon de marquage sont ajoutés et les échantillons sont analysés sur un cytomètre de flux FacsCan®.

Une molécule capable de diminuer ou inhiber la prolifération ou la survie et/ou d'induire ou d'augmenter l'apoptose des mastocytes humains peut être utilisée dans la prévention ou le traitement d'une tumeur telle que décrite dans le présent texte, par exemple d'une mastocytose. L'efficacité d'une telle molécule ou de plusieurs d'entre elles peut être évaluée *in vitro* ou *ex vivo* par exemple par la mesure du niveau de libération d'un ou de plusieurs médiateurs par les mastocytes impliqués dans la mastocytose, typiquement par les mastocytes d'un sujet souffrant de mastocytose, typiquement d'un sujet humain, un niveau de libération diminué par rapport au niveau observé sur les mêmes mastocytes non exposé à ladite ou auxdites molécules révélant l'efficacité de ladite ou desdites molécules.

Dans le cadre des mastocytoses par exemple, les cellules selon l'invention et plus particulièrement les cellules issues des lignées transformées ROSA KIT D816V et ROSA KIT Delta 417-419 insY peuvent être utilisées dans des tests de criblage à haut débit de bibliothèques de molécules candidates, à la recherche de molécules inhibant la prolifération, la survie et/ou l'activation des dites cellules.

L'invention concerne en outre une méthode d'évaluation de la toxicité d'une molécule candidate capable de ou susceptible de cibler les mastocytes humains porteurs d'une ou de plusieurs mutations du récepteur KIT, typiquement d'une mutation activatrice de KIT, par exemple d'une mutation située dans le domaine phosphotransférase du récepteur KIT WT (correspondant aux acides aminés occupant les positions 762 à 937 de SEQ ID NO : 2) telle que par exemple la mutation D816V (Asp816Val), la mutation A814V (Ala814Val) ou la mutation R815K (Arg815Lys) ; dans le domaine juxta-membranaire du récepteur KIT telle que par exemple la mutation V559I (Val559IIe), la mutation V560G (Val560Gly) ou la mutation D572A (Asp572Ala) ; ou dans le domaine extra-membranaire du récepteur KIT, telle que par exemple la mutation KIT Delta 417-419insY, la mutation KIT S476I (Ser476IIe), la mutation KIT ITD502-503 (« Internal Tandem Duplication », ou duplication interne en tandem, i.e. répétition deux fois des acides aminés 502 et 503), ou la mutation KIT K509I (Lys509IIe), ladite méthode comprenant :
a) la mise en contact d'une cellule, d'un clone cellulaire, d'une lignée cellulaire, d'une population de cellules mastocytaires, d'une sous-population de cellules mastocytaires ou d'une composition selon l'invention, de préférence de la lignée cellulaire mastocytaire ROSA KIT WT, avec au moins une molécule candidate telle que définie précédemment capable de ou susceptible de cibler les mastocytes humains porteurs d'une mutation du récepteur KIT ; et
b) l'évaluation de l'éventuelle toxicité de ladite molécule vis-à-vis de ladite cellule, dudit clone cellulaire, de ladite lignée cellulaire, de ladite population de cellules mastocytaires, de ladite sous-population de cellules, de préférence de ladite lignée cellulaire mastocytaire ROSA KIT WT, typiquement par détermination et/ou mesure de la survie des cellules.

La présente invention concerne par ailleurs des méthodes permettant de tester l'intérêt, en particulier l'efficacité en terme de prévention ou de traitement d'une pathologie, ou la toxicité d'au moins une molécule candidate sur des **modèles animaux** selon l'invention comprenant une cellule mastocytaire selon l'invention, typiquement une population de cellules mastocytaires.

L'invention enseigne ainsi une méthode particulière de détermination de la capacité d'au moins une molécule candidate à prévenir ou traiter au moins une tumeur et ses éventuelles métastases. Cette méthode comprend typiquement :
a) l'administration de la au moins une molécule candidate à un modèle animal selon l'invention, typiquement un modèle animal non humain comprenant au moins une cellule provenant d'une lignée cellulaire mastocytaire selon l'invention, de préférence d'une lignée choisie parmi la lignée ROSA KIT D816V, la lignée ROSA KIT Delta 417-419 insY et une lignée de type ROSA KIT D816V ou ROSA KIT Delta 417-419 insY; et
b) la mesure de la prolifération des cellules de ladite au moins une tumeur et desdites éventuelles métastases dudit modèle animal, l'inhibition ou la diminution de la prolifération desdites cellules chez ledit modèle animal indiquant que la au moins une molécule candidate peut être utilisée pour prévenir ou traiter ladite au moins une tumeur et ses éventuelles métastases.

Une telle méthode permet d'évaluer *in vivo* l'intérêt, en particulier l'intérêt préventif ou thérapeutique, d'une molécule candidate criblée à l'aide de l'une des méthodes praticables *in vitro* ou *ex vivo* décrites dans le présent texte qui utilisent une cellule selon l'invention.

Une autre méthode selon l'invention permet de déterminer la capacité d'une molécule candidate à moduler l'infectiosité d'au moins un agent infectieux vis-à-vis des mastocytes humains. Cette méthode comprend :
a) la mise en contact d'une cellule, d'un clone cellulaire, d'une lignée cellulaire, d'une population de cellules mastocytaires, d'une sous-population de cellules ou d'une composition selon l'invention, typiquement d'une lignée cellulaire mastocytaire, avec au moins une molécule candidate telle que définie précédemment et au moins un agent infectieux ; et
b) la détermination de la capacité dudit au moins un agent infectieux à infecter le mastocyte, ladite détermination permettant de déterminer la capacité de la au moins une molécule candidate à moduler l'infectiosité dudit au moins un agent infectieux vis-à-vis des mastocytes humains.

L'agent infectieux peut être un microorganisme, par exemple un virus tel que le virus de la Dengue, une bactérie, un champignon ou un parasite.

Une autre méthode selon l'invention permet de déterminer la capacité d'une molécule candidate à moduler l'infectiosité d'un virus vis-à-vis des mastocytes humains, ainsi que son aptitude à se répliquer dans une cellule selon l'invention par mise en contact d'une première population de cellules mastocytaires infectées par ledit virus avec une seconde population de cellules mastocytaires non infectées par ledit virus et détermination de la capacité dudit virus à infecter la seconde population de cellules mastocytaires.

Les figures et exemples suivants illustrent l'invention en décrivant l'obtention de cultures et de lignées de mastocytes humains conformes à l'invention, sans en limiter la portée.

### LEGENDES DES FIGURES

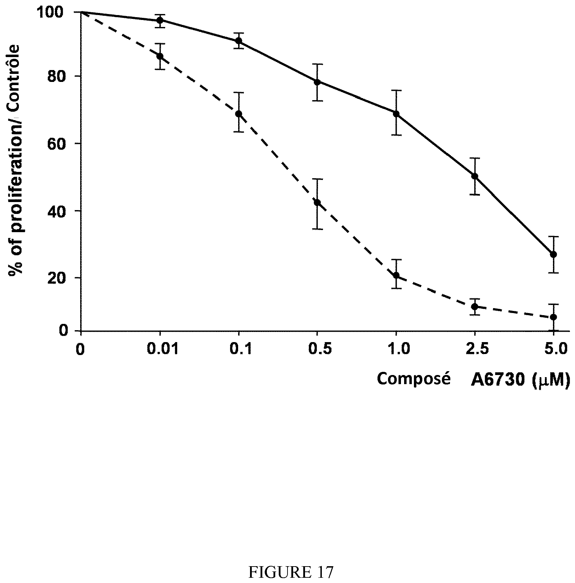

**Figure 1** **:** caractérisation des cellules ROSA KIT WT par coloration de May-Grünwald Giemsa (MGG), coloration au bleu de toluidine et microscopie électronique à transmission (TEM).
**Figure 2** **:** Expression du récepteur KIT (histogramme de gauche) et du récepteur de haute affinité aux IgE (FcεR1) par les cellules de la lignée ROSA KIT WT (analyse par cytométrie en flux).
**Figure 3** **:** Mise en évidence du contenu en tryptase et en HDC des cellules de la lignée ROSA KIT WT par méthode immunocytochimique sur cellules cytocentrifugées.
**Figure 4** **:** Etude par cytométrie en flux de différents composés, dont l'IL-4, sur l'expression de KIT et de FcεR1 par les cellules de la lignée ROSA KIT WT.
**Figure 5** **:** Etude par cytométrie en flux de l'effet de différents traitements sur l'expression du CD203c membranaire par les cellules de la lignée ROSA KIT WT.
**Figure 6** **:** Mesure par méthode spectrophotométrique de l'activité β-hexosaminidase libérée par les cellules de la lignée ROSA KIT WT après leur stimulation.
**Figure 7** : Mesure du pourcentage d'histamine libérée par les cellules de la lignée ROSA KIT WT après leur stimulation par le couple IgE-anti-IgE.
**Figure 8** : Mesure du TNF-α libéré dans le surnageant des cellules de la lignée ROSA KIT WT après leur stimulation pendant 6 heures par le couple IgE-anti-IgE.
**Figure 9** **:** Aspect morphologique et phénotypique des cellules de la lignée ROSA KIT WT co-cultivées pendant plus de trois mois sur une sous-couche de cellules lipoblastiques de souris MS-5 (A : coloration au MGG ; B : coloration au bleu de toluidine ; C : marquage par immunocytochimie de la chymase ; D : analyse par cytométrie en flux de différents marqueurs membranaires).
**Figure 10** **:** Mesure de l'efficacité d'infection par cytométrie en flux (% de cellules positives pour la GFP) 4 jours après contact des cellules ROSA KIT WT avec les différentes constructions lentivirales utilisées à concentrations croissantes.
**Figure 11** **:** Aspect morphologique des cellules des lignées ROSA KIT Delta 417-419 insY et KIT D816V après coloration au MGG. Présence de cellules immatures, sans aucune granulation pour la lignée ROSA KIT Delta 417-419 insY (flèche rouge) tandis que dans la lignée ROSA KIT D816V on retrouve des cellules en voie d'apoptose, avec de nombreux grains.
**Figure 12** **:** Etude par cytométrie en flux de l'expression de certains marqueurs de membrane par des cellules des lignées ROSA KIT Delta 417-419 insY et KIT D816V traitées ou non par du SCF (80 ng/mL) et par des cellules de la lignée ROSA KIT WT.

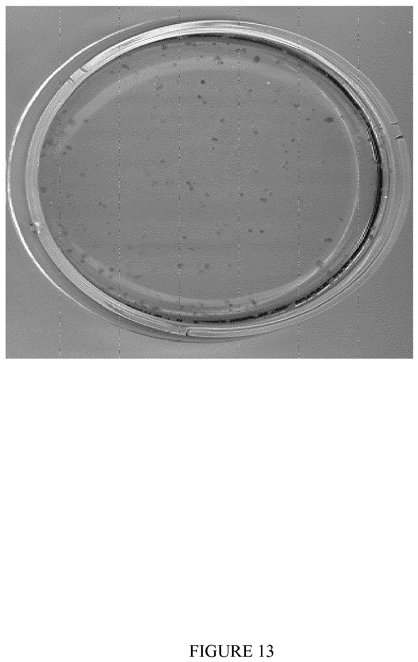

**Figure 13** **:** Exemple de l'aspect d'une culture en milieu semi-solide (méthylcellulose à 1%) des cellules de la lignée ROSA KIT Delta 417-419 insY après incubation pendant 15 jours en boite de Pétri. Les colonies, de tailles variables, sont identifiables à l'oeil nu.
**Figure 14** **:** Mise en évidence par Western Blot de la phosphorylation spontanée de KIT dans les lignées HMC-1.2, ROSA KIT D816V et ROSA KIT Delta 417-419 InsY. Les lysats cellulaires préparés à partir des cellules non stimulées ou stimulées pendant 5 minutes avec du SCF humain ont été soumis à une électrophorèse en SDS-PAGE et traités par un anticorps anti-P KIT Y703 (Cell signaling, réf : 3073S). Les membranes ont ensuite été déshybridées puis réhybridées avec un anticorps anti-KIT (Santacruz Biotechnology, réf : J2709).
**Figure 15** **:** Mise en évidence de l'effet des inhibiteurs de tyrosine kinase sur la prolifération des lignées ROSA KIT WT, ROSA KIT Delta 417-419 InsY et ROSA KIT D816V. Les valeurs présentées sont comparées avec celles obtenues sans inhibiteur. Les résultats représentent la moyenne +/- écart-type de triplicats.

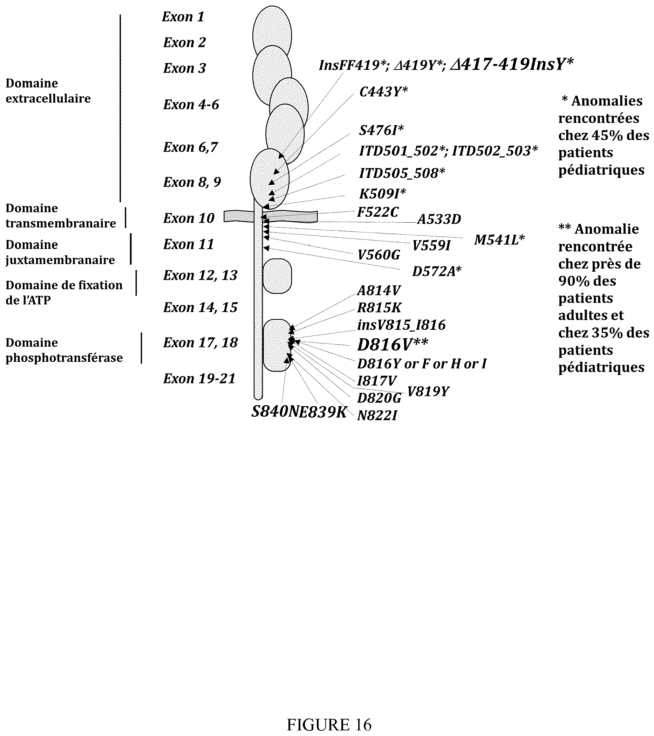

**Figure 16** **:** Structure du récepteur KIT (CD117) et principales anomalies rencontrées au cours des mastocytoses.
**Figure 17** **:** Effet de l'inhibition spécifique d'AKT sur la prolifération des deux lignées cellulaires ROSA.
   Les cellules ROSA^{KIT WT} (-) or ROSA^{KIT D816V} (---) sont ensemencées pendant 48 heures en présence de concentrations variables (0.01 à 5µM) de l'inhibiteur puissant et sélectif de AKT1/AKT2, le composé A6730 (dilué dans du DMSO à une concentration finale de 0.1%), ou en présence de DMSO seul (concentration finale de 0.1 %) dans SFM (avec rhSCF à 80 ng/mL pour les cellules ROSA KIT WT mais pas pour les cellules ROSA KIT D816V). A la fin de cette période d'incubation, 10µL de MTT ont été ajoutés dans chaque puits (3-(4,5-Dimethyltiazol-2-yl)-2,5-diphenyltetrazolium bromide) et les cellules ont été incubées pendant 3 heures supplémentaires dans un incubateur à 37°C. Le nombre de cellules vivantes a ensuite été mesuré pour chaque condition par lecture de l'absorbance à 450 nm. Les données apparaissant sur la Figure 17 représentent les moyennes ± la déviation standard obtenues à partir de 3 expériences indépendantes et sont exprimées en pourcentage de prolifération pour chaque condition par rapport au contrôle (DMSO seul) représentant 100% de prolifération.

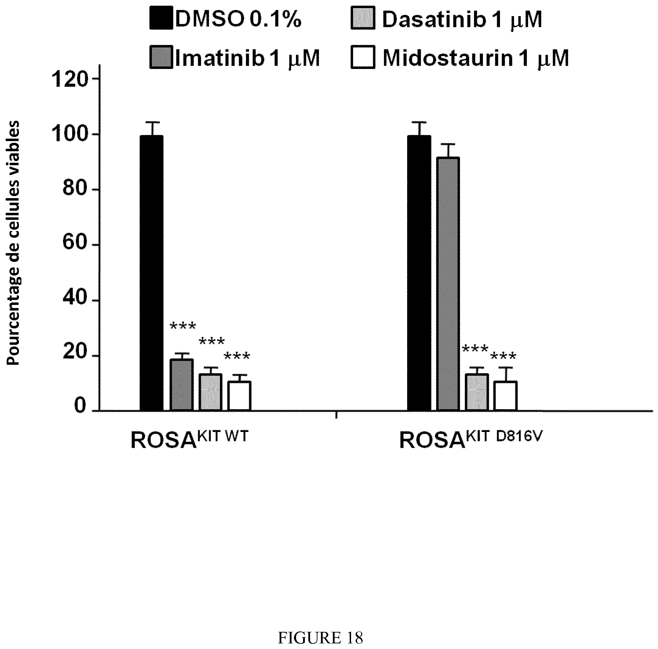

**Figure 18** **:** Effet de trois inhibiteurs de tyrosine kinase sur la prolifération des deux lignées cellulaires ROSA.
   Les cellules ont été ensemencées 48 heures en présence d'Imatinib, Dasatinib ou Midostaurin (1µM, fourni dans du DMSO à une concentration finale de 0.1 %) ou en présence de DMSO seul (concentration finale de 0.1 %) dans un milieu de culture classique (contenant du SCF humain à 80 ng/mL pour les cellules ROSA^{KIT WT} mais sans SCF pour les cellules ROSA^{KIT D816V}). A la fin de cette période d'incubation, 10pL de MTT ont été ajoutés dans chaque puits (3-(4,5-Dimethyltiazol-2-yl)-2,5-diphenyltetrazolium bromide) et les cellules ont été incubées pendant 3 heures additionnelles dans un incubateur à 37°C. Le nombre de cellules vivantes a ensuite été mesuré pour chaque condition par lecture de l'absorbance à 450 nm. Les données apparaissant sur la Figure 18 représentent les moyennes ± la déviation standard obtenues à partir de 3 expériences indépendantes. *** : *valeur significativement différentes du contrôle (DMSO seul) à p<0.0001.*

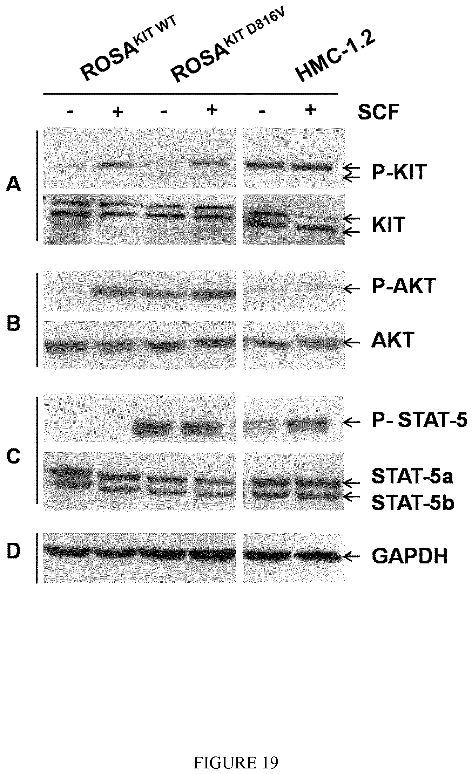

**Figure 19** **:** Démonstration par Western-blot de la phosphorylation spontanée de KIT, AKT et STAT-5 dans les cellules ROSA^{KIT D816V}.

Les lysats cellulaires provenant de lignées cellulaires ROSA non stimulées et de la lignée cellulaire HMC-1.2 ou de cellules identiques stimulées pendant 10 minutes avec du SCF humain ont été soumises à une électrophorèse en SDS-PAGE et traitées avec un KIT anti-humain total ou avec un anti-P-KIT Y703 (A), avec un AKT anti-humain total ou un anti-P-AKT S473 (B), ou avec un STAT-5 anti-humain total ou un anti-P-STAT-5 Y694 (C). D : un GAPDPH anti-humain a été utilisé comme contrôle de charge.

### EXEMPLES

### EXEMPLE 1 : ISOLEMENT, CARACTERISATION ET ENTRETIEN DE POPULATIONS PURES DE MASTOCYTES A PARTIR DE SANG DE CORDON HUMAIN NORMAL : LA LIGNEE ROSA KIT WT

### 1) Prélèvement et mise en culture des cellules

Le prélèvement de sang de cordon ombilical normal a été recueilli sur héparinate de lithium le 9 septembre 2009. Le sang (40mL) a été immédiatement dilué au demi dans du tampon PBS (Invitrogen) et les cellules mononucléées ont été séparées des autres éléments sanguins par centrifugation à 700g pendant 30 min sur un gradient de Ficoll (Eurobio). Les cellules mononucléées, qui forment un anneau à l'interface entre le Ficoll et le sérum dilué, ont été récupérées et lavées avec du tampon PBS. La numération et la viabilité cellulaire ont été effectuées en utilisant le bleu trypan (Sigma). Les progéniteurs hématopoïétiques de sang de cordon exprimant l'antigène de surface CD34 ont été ensuite sélectionnés positivement par tri immuno-magnétique en utilisant le système MACS (Milteny). 85.10⁴ cellules CD34+ ont ainsi été obtenues.
Ces cellules CD34+ purifiées ont ensuite été ensemencées à 5.10⁴ par mL (17 ml au total) en milieu Iscove Modified Dulbecco Medium (IMDM)-Glutamax® (Invitrogen) supplémenté avec de la Pénicilline/Streptomycine 100 U/ml (P/S) (Invitrogen), 1% de pyruvate de sodium (Invitrogen), 1% de vitamines (Invitrogen), 1% de Glutamine (Invitrogen), 2% d'acides aminés non essentiels (Invitrogen), 1% d'une solution commerciale d'insuline-transferrine-sélénite de sodium (Invitrogen) et 0,3% d'albumine bovine (BSA) (PAA).
Différentes cytokines ont été ajoutées à la suspension cellulaire afin d'orienter la différenciation vers le mastocyte : SCF à 80 ng/mL (R&D), IL-6 à 50 ng /mL (R&D) et IL-3 à 1 ng/mL (R&D). L'IL-3 et l'IL-6 n'étant ajoutées que pendant la première semaine de culture. Les cellules ont ensuite été placées dans une étuve à 37°C, sous atmosphère humide contenant 5% de CO₂.

Le milieu a été renouvelé une à deux fois par semaine et les cellules ont été entretenues à 7.10⁵ par mL pendant 8 à 12 semaines jusqu'à obtention d'une population pure de mastocytes.
A l'inverse du comportement habituel de ce type de culture, les cellules du cordon du 9 septembre 2009 dénommées « **ROSA KIT WT** » ont continué à proliférer dans le même milieu de culture contenant 80 ng/mL de SCF humain jusqu'à ce jour avec un temps de doublement de 48 heures. Les cellules sont donc maintenues en culture depuis le début par dilution au 1/3 ou 1/4 dans du milieu de culture neuf tous les 3 ou 4 jours. Les cellules sont congelables et décongelables de façon répétée en utilisant les méthodes de congélation classiques (Serum de Veau Foetal (SVF) décomplémenté (PAA) contenant 10% de DMSO (Sigma)).

### 2) Caractérisation des cellules

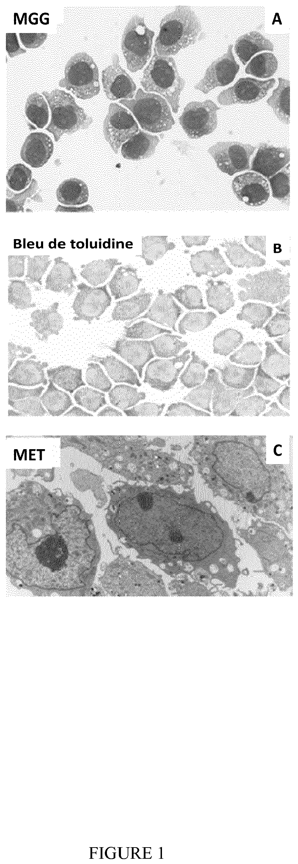

L'identification de la lignée ROSA KIT WT comme lignée mastocytaire a été effectuée par coloration au bleu de toluidine et coloration de May-Grünwald Giemsa (MGG), étude en microscopie électronique à transmission et mise en évidence de la tryptase et de l'histidine décarboxylase (HDC) intracellulaires.
Après coloration au MGG (figure 1), les cellules de la lignée ROSA KIT WT apparaissent arrondies avec un rapport nucléo-cytoplasmique très important. Dans le cytoplasme, qui est basophile, on note la présence de nombreuses granulations de couleur violet foncé; le nombre et la taille des granulations varient : elles sont petites et peu nombreuses dans les cellules considérées comme immatures ; elles sont nombreuses et volumineuses dans les cellules considérées comme matures. Leur métachromasie a été révélée par la technique de coloration au bleu de toluidine.
La caractérisation morphologique a été complétée par une observation en microscopie électronique (figure 1). Les mastocytes de la lignée ROSA KIT WT observés sont des cellules qui ont un noyau volumineux, avec une chromatine lâche suggérant qu'il s'agit des cellules assez jeunes. Dans le noyau, on observe un à deux nucléoles. Le cytoplasme contient des ribosomes, de nombreuses mitochondries et de nombreuses granulations. Ces granulations sont du même type que celles décrites dans des mastocytes humains en culture primaire.

### Mise en évidence du récepteur KIT et du récepteur des IgE

Des marquages directs à différents temps de la culture ont été réalisés en cytométrie en flux sur les cellules de la lignée ROSA KIT WT en culture avec un anticorps dirigé contre la chaine alpha (α) du récepteur à haute affinité aux IgE couplé au fluorochrome FITC (Fluorescéine) (Biolegend) ou avec l'anticorps anti KIT (CD117) humain couplé à l'APC (Biolegend).
- On utilise 1 ml de cellules en culture à environ 10⁶ cellules/ml pour chaque tube.
- Les cellules sont centrifugées pour éliminer le milieu de culture.
- Les culots sont repris dans 100 µl de PBS+SVF 1%, et incubés pendant 45 mn à +4°C en présence d'anticorps anti IgE humain FITC (20 µl/1 million de cellules), ou en présence d'anticorps anti KIT humain couplé à l'APC (20 µl/1 million de cellules).
- On effectue deux rinçages dans 3 ml de PBS, 0,1% SVF, puis les tubes sont centrifugés.
- Les culots sont recueillis dans 300 µl de PBS, 0,1% SVF.
La lecture est faite en cytométrie en flux (BD Facs Calibur).

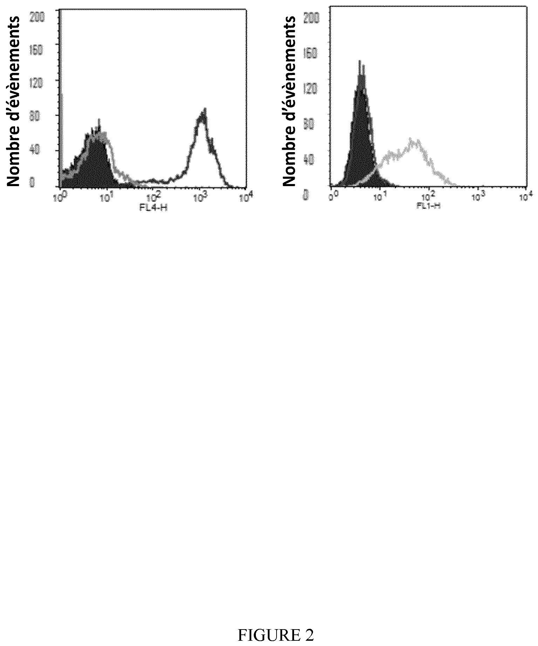

Comme le montre la figure 2, il apparaît que les cellules de la lignée ROSA KIT WT analysées expriment de façon significative le récepteur à l'IgE ainsi que le récepteur KIT.

### Mise en évidence de l'expression de tryptase et de l'histidine décarboxylase (HDC)

La présence de tryptase et de l'histidine décarboxylase (HDC) intracellulaires a été mise en évidence dans les cellules de la lignée ROSA KIT WT par des méthodes immuno-histochimiques sur cellules cyto-centrifugées.
La tryptase et l'HDC sont détectées en utilisant un anticorps monoclonal anti-tryptase (Dako) ou un anticorps anti HDC (Santa Cruz).
Des marquages indirects ont été réalisés sur lames après cytocentrifugation des cellules pendant 10 minutes à 500 tours par minutes.
- Après cytocentrifugation les cellules sont fixées avec de l'acétone pendant 8 mn.
- Elles sont ensuite rincées à 2 reprises en TBS et séchées à l'air pendant 20 min, puis incubées avec le 1 er anticorps (anti tryptase dilué au 1 /250^{ème} dans du TBS-1%BSA, ou anti HDC dilué au 1/250) 1 heure à température ambiante en chambre humide. Après 3 rinçages en TBS (3 x 5 min), les cellules sont incubées avec le 2ème anticorps (Biotinylated Goat anti-Mouse IgG (Dako)) 30 min à température ambiante en chambre humide, puis rincées trois fois (5 min à chaque fois) en TBS.
- Les cellules sont ensuite incubées avec le troisième anticorps (Streptavidin AP (Dako)) 30 min à température ambiante en chambre humide. Après 3 rinçages en TBS (3 x 5 min), on applique sur chaque lame 100µl de New Fuchsin pendant 10 minutes. On rince à nouveau 3 fois (5 min à chaque fois) en TBS, puis on incube à température ambiante pendant 3 min avec de l'hématoxyline. On rince enfin 5 min avec H₂O et on monte les lames en Aquatex.
La lecture est effectuée au microscope optique.

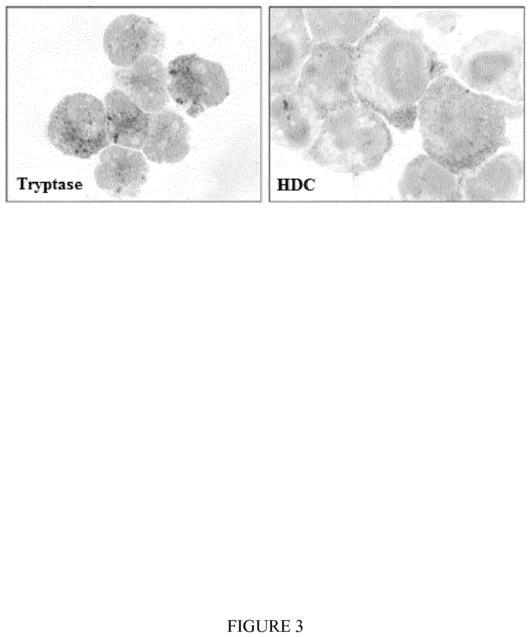

On observe un marquage positif des deux enzymes dans la quasi-totalité des cellules ROSA (figure 3). La forte positivité de la tryptase confirme la nature mastocytaire des cellules de la lignée ROSA KIT WT.

### 3) Effet du traitement par l'IL-4 ou par d'autres molécules des cellules de la lignée ROSA KIT WT sur leur expression de FcεRI et de KIT

Comme il a été montré dans la littérature que l'interleukine-4 (IL-4) induit une diminution d'expression de KIT et une augmentation d'expression du FcεR1 par les mastocytes humains normaux, les inventeurs ont voulu vérifier si ce phénomène pouvait aussi être observé sur la lignée ROSA KIT WT. Pour cela, ils ont utilisé la technique suivante :
- On utilise 10 ml de cellules en culture à environ 2.10⁵ cellules/ml pour chaque tube.
- Les cellules sont centrifugées et le culot est repris dans 10 ml du milieu de culture neuf puis les cellules sont traitées par l'IL-4 (20ng/ml) (R&D) pendant 5 jours.
- Les cellules sont centrifugées pour éliminer le milieu de culture.
- Les culots sont repris dans 100 µl de PBS+SVF 1%, et incubés pendant 45 min à +4°C en présence d'anticorps anti IgE humain FITC (20 µl/1 million de cellules (Merck Bioscience)), ou en présence d'anticorps anti KIT humain couplé à l'APC (20 µl/1 million de cellules) (Biolegend).
- On effectue deux rinçages dans 3 ml de PBS, 0,1% SVF, puis les tubes sont centrifugés.
- Les culots sont recueillis dans 300 µl de PBS, 0,1% SVF.
La lecture est faite en FACS (BD Facs Calibur).

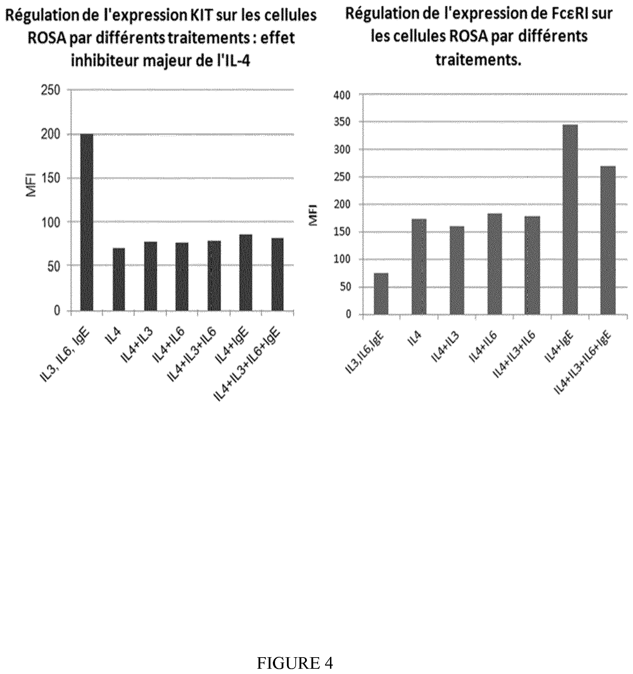

Les résultats obtenus sont présentés sur la figure 4 et montrent que ce traitement par l'IL-4 induit bien une diminution de l'expression de KIT et une augmentation de celle du récepteur de haute affinité aux IgE sur les cellules ROSA KIT WT. Ces cellules se comportent donc bien comme des mastocytes humains normaux de ce point de vue.

### 4) Mise en évidence de l'activation des cellules de la lignée ROSA KIT WT par le couple IgE-anti IgE

Afin de savoir si les cellules de la lignée ROSA KIT WT sont activables par le couple IgE-anticorps anti-IgE, conduisant à une dégranulation avec libération immédiate d'histamine et de β-hexosaminidase associée à une augmentation importante de l'expression membranaire du CD203c et à une libération retardée de TNF-alpha, des cellules sont traitées par IL-4 (20ng/ml) et IgE (2µg/ml) (Merck Bioscience) pendant 5 jours puis stimulées par anti IgE (5 ou 10 µg/ml) (Biovalley) ou par le calcium ionophore (Cai) (Sigma) (1 µmol/l) pendant 1 ou 6 heures. Le surnageant de 1 heure ainsi que le culot sont utilisés pour doser l'histamine et pour mesurer l'expression de CD203c à la surface cellulaire alors que le TNF-alpha est mesuré par méthode ELISA spécifique dans le surnageant des cellules après 6 heures de stimulation.

### a) Augmentation de l'expression du CD203c membranaire sur les cellules de la lignée ROSA KIT WT traitées IL4 et IgE puis stimulées par l'anti-IgE

Un ml de cellules traitées par IL-4 (20ng/ml) et IgE (2µg/ml) pendant 5 jours sont lavées deux fois par 3ml de PBS puis remises dans du milieu de culture à une concentration de 1.10⁶ cellules/ml. Ces cellules sont ensuite stimulées par l'anti-IgE (5 ou 10 µg/ml) ou par l'ionophore calcique (Cai) (1 µmol/l) pendant 1 heure. Les cellules sont lavées avec du PBS et remises dans 100 µl PBS, 1%SVF en présence d'un anticorps dirigé contre le CD203c humain couplé au phycoérythrine (PE) (20 µl/1 million de cellules) (Biolegend).

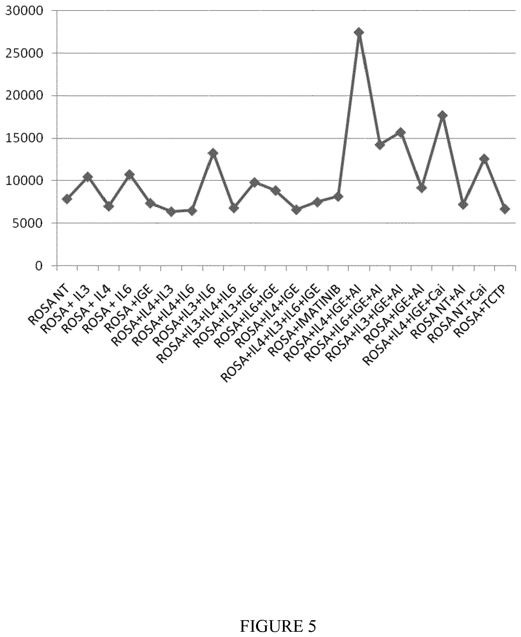

Les résultats, présentés dans la figure 5, montrent une augmentation nette de l'expression de CD203c lorsque ces cellules sont traitées par IL-4+IgE et stimulées par l'anti-IgE. Cette augmentation est aussi observée lorsque ces cellules sont traitées par l'ionophore calcique.

### b) Libération de la β-hexosaminidase dans le surnageant des cellules de la lignée ROSA KIT WT après activation

Nous avons aussi mesuré l'activation des cellules de la lignée ROSA KIT WT en déterminant l'activité de la β-hexosaminidase libérée. Les cellules ROSA traitées par IL-4 (20ng/ml) et IgE (2µg/ml), sont activées ensuite pendant 1 heure à 37°C en présence de 5% de CO₂ par l'anticorps anti-IgE (5 µg/ml) ou par le calcium ionophore (Cai) (1 µmol/l).
Les cellules sont ensuite centrifugées et les surnageants sont récupérés et congelés à -80°C jusqu'au moment du dosage. Les cellules sont ensuite lysées. L'activité de la β-hexosaminidase est mesurée dans le surnageant ainsi que dans le culot cellulaire comme décrit par SCHWARTZ et AUSTEN, J Invest Dermatol., 74, 349-353, 1980.
L'hydrolyse du p-Nitrophenyl-2-acetamido-2-deoxy-β-D-glucopyranoside (ou 4-Nitrophenyl N-acetyl β-D-glucosaminide) par la α-hexosaminidase entraine la libération d'un produit chromophore : le p-Nitrophenol. Ce dernier est mesuré par spectrophotométrie 405 nm.
- On ajoute 50 µl du p-Nitrophenyl-2-acetamido-2-deoxy-β-D-glucopyranoside (Sigma) à 5µl de surnageant
- Incubation 2 heures à 37°c sous agitation.
- Ajout de 150µl de solution d'arrêt (7,5 g de glycine dans 500 ml d'H₂O PH=10,7).
- Lecture à 405 nm.

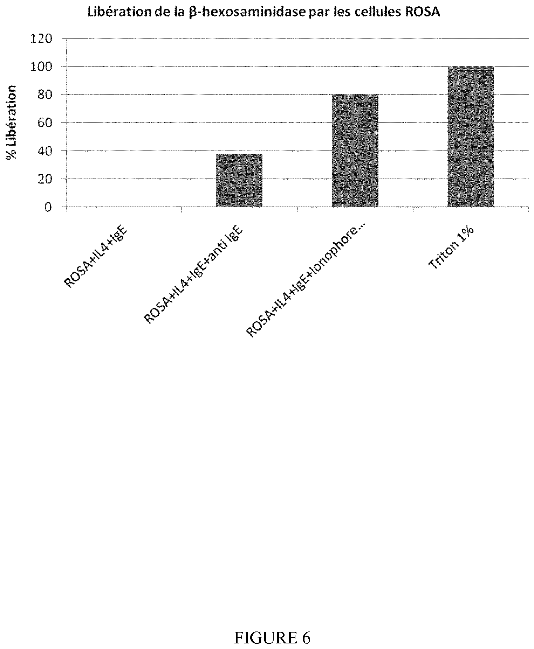

Les résultats obtenus et présentés dans la figure 6 montrent que la stimulation des cellules de la lignée ROSA KIT WT par le couple IgE-anti-IgE entraîne une induction de la libération de β-hexosaminidase qui peut aller jusqu'à 38% du contenu total enzyme des cellules. Comme attendu, le ionophore calcique, stimulant non-spécifique, induit une libération de β-hexosaminidase qui peut aller jusqu'à 80% du contenu total enzyme des cellules, ce qui est conforme avec les données de la littérature.

### c) Mesure de la libération d'histamine par les cellules de la lignée ROSA KIT WT après leur stimulation

### 1) Préparation des cellules :

50 000 cellules traitées par IL-4 (20ng/ml) et IgE (2µg/ml) pendant 1 ou 4 jours ont été activées pendant 30 minutes à 37°C en présence de 5% de CO₂ à l'aide de différentes concentrations de l'anticorps anti-IgE. Les surnageants ainsi que les culots sont récupérés et congelés à -80°C jusqu'au moment du dosage de l'histamine.

### 2) Dosage de l'histamine :

Le dosage de l'histamine a été réalisé dans les surnageants et les culots en utilisant un kit de dosage radio-immunologique (histamine radio-immunoassay (RIA) ; Immunotech, Marseille,France) conformément aux indications du fournisseur (MOREL et DELAAGE. J Allergy Clin Immunol. 1988 Oct;82(4):646-54 - VALENT et al. Proc Natl Acad Sci USA. 1989 Jul;86(14):5542-6).

Le pourcentage net de libération d'histamine est calculé en utilisant la formule suivante :
% de libération = S - S contrôle x 100/ (S+P) - S contrôle
Dans laquelle :
   S= Histamine dans le surnageant
   C= histamine dans le culot.

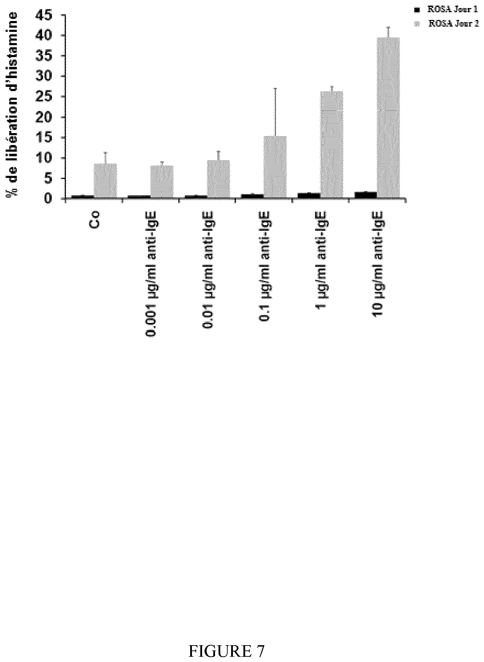

Les résultats obtenus (figure 7) montrent qu'il existe une augmentation dose-dépendante (en fonction de la concentration d'anti-IgE rajoutée) de la libération d'histamine par les cellules de la lignée ROSA KIT WT lorsqu'elles sont stimulées par le couple IgE-anti-IgE. Cette libération est d'autant plus significative si les cellules ont été sensibilisées au préalable par l'IL-4 et les IgE, de préférence pendant au moins 4 jours. La libération maximale d'histamine obtenue, aux alentours de 40% du contenu total en histamine des cellules est tout-à-fait comparable à la libération de bêta-hexosaminidase par les mêmes cellules stimulées dans les mêmes conditions (figure 6).

### d) Dosages du TNF-alpha libéré dans le surnageant des cellules de la lignée ROSA KIT WT après leur stimulation

A l'aide d'un kit ELISA spécifique (Enzyme Linked ImmunoSorbent Assay, R&D), nous avons quantifié la libération tardive de TNF-alpha par les cellules ROSA traités par IL-4 (20ng/ml) et IgE (2µg/ml), activées ensuite pendant 6 heures à 37°C en présence de 5% de CO₂ par différentes concentrations de l'anticorps anti IgE (5 ou 10 µg/ml) ou par le calcium ionophore (Cai) (1 µmol/l).
Les cellules sont ensuite centrifugées et les surnageants sont récupérés et congelés à -80°C jusqu'au moment du dosage de TNF-alpha.

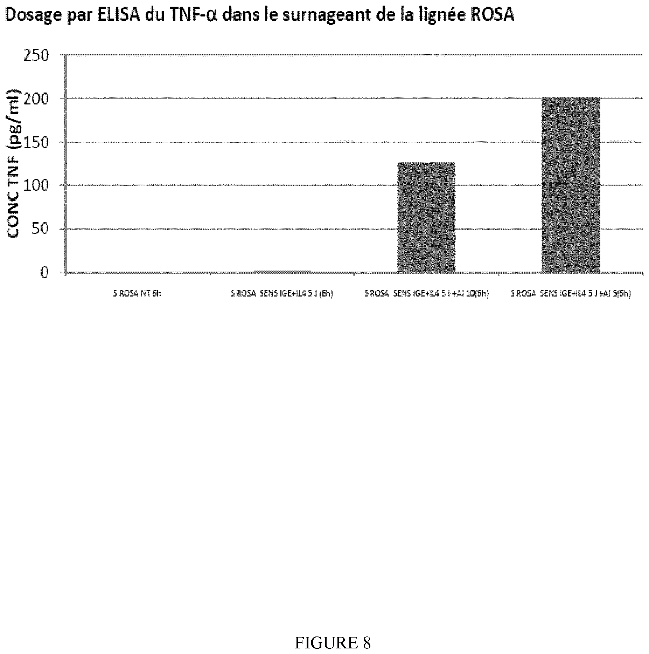

Les résultats obtenus et présentés dans la figure 8 montrent qu'il existe une augmentation dose dépendante de la libération de TNF-alpha par les cellules ROSA KIT WT après leur stimulation par le couple IgE-anti-IgE.

### 5) Mise en évidence de l'aptitude des cellules de la lignée ROSA KIT WT à maturer de façon terminale après coculture sur une sous-couche de cellules MS-5

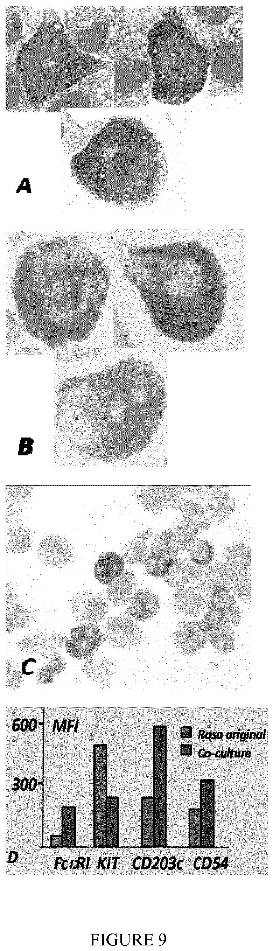

Les cellules de la lignée ROSA KIT WT présentent un phénotype de mastocytes humains relativement immatures (négativité de la chymase, métachromasie relativement peu importante). Nous avions précédemment démontré que la culture de progéniteurs hématopoïétiques humains CD34+ sur une sous-couche de cellules lipoblastiques de souris (lignée MS-5) permettait la différenciation mastocytaire terminale de ces progéniteurs (AROCK et al. Ann N Y Acad Sci. 1994 May 28;725:59-68). Nous avons donc cultivé des cellules de la lignée ROSA KIT WT sur une sous-couche de cellules MS-5 à confluence, et ce pendant plus de 3 mois, le milieu de culture étant renouvelé par moitié tous les 3 ou 4 jours. Au bout de ce temps de coculture, les cellules non adhérentes de la co-culture ont été analysées pour leur aspect morphologique après coloration au MGG (figure 9A), leur métachromasie au bleu de toluidine (figure 9B), leur contenu en chymase (immunocytochimie) (figure 9C) et l'expression de différents marqueurs de surface, y compris le KIT, le FcεR1, le CD54 et le CD203c (figure 9D). L'analyse de ces résultats montre que les cellules de la lignée ROSA KIT WT peuvent acquérir un phénotype de mastocytes humains totalement matures (MCTC) après ce temps de co-culture. En effet les cellules présentent une augmentation considérable du nombre de granulations dans leur cytoplasme (coloration du MGG) qui sont métachromatiques (coloration au bleu de toluidine). En outre, les cellules co-cultivées expriment la chymase et sont plus fortement positives que les cellules non co-cultivées pour l'expression du FcεR1, du CD54 et du CD203c, qui sont tous les trois des marqueurs de maturation.

### EXEMPLE 2 : OBTENTION DE LIGNEES IMMORTALISEES DE MASTOCYTES HUMAINS TRANSFECTEES PAR UN RECEPTEUR KIT DE STRUCTURE ANORMALE : LES LIGNEES ROSA KIT D816V ET ROSA KIT Delta 417-419 InsY

Dans le but d'obtenir et de caractériser des sous-clones SCF-indépendant de la lignée ROSA de départ, les cellules ont été transfectées par des vecteurs lentiviraux apportant une construction codant pour le KIT muté en D816V ou le KIT muté Delta 417-419 insY. Ces deux anomalies acquises de la structure du récepteur KIT sont rencontrées fréquemment respectivement au cours des mastocytoses systémiques de l'adulte (KIT D816V ; FÉGER et al. Int Arch Allergy Immunol. 2002 Feb;127(2):110-4) ou au cours des mastocytoses cutanées de l'enfant (KIT Delta 417-419 ins Y ; BODEMER et al. J Invest Dermatol 2010; 30(3):804-15). De plus, nous avons montré que la mutation D816V était capable d'induire une mastocytose systémique chez la souris transgénique (ZAPPULLA et al. J Exp Med. 2005 Dec 19;202(12):1635-41).

### 1) Procédure de transfection utilisée

### a) Mutagénèse dirigée et vecteurs plasmidiques

Pour préparer les constructions correspondantes, l'ADNc codant pour l'isoforme courte de KIT sauvage humain a été excisé du vecteur pBS-hkitWT par digestion Sal I-Acc65. Le fragment d'ADNc a été sous-cloné dans le vecteur pENTR1A (Invitrogen, France). Les deux mutations de KIT (KIT muté en D816V ou le KIT muté Delta 417-419 insY) ont été introduites dans le vecteur pENTR1A-hkitWT codant pour la protéine fluorescent en vert (GFP) en utilisant un Kit QuickChange^{™} de mutagénèse dirigée (Stratagene, Pays-Bas) conformément aux instructions du fabricant. Pour le mutant KIT D816V, le codon 816 de KIT WT (GAC) a été remplacé par le codon GTC. Pour le mutant KIT Delta 417-419 InsY, les codons 417/418/419 de KIT WT (ACTTACGAC) ont été remplacés par le codon TAC.

### b) Transformation bactérienne pour amplification et purification des ADN plasmidiques codant pour les formes mutées de KIT

On ajoute 5-10 µl de plasmide par tube de bactéries (XL10-Gold^{®} Ultracompetent Cells #200314, Stratagene) et on fait subir aux bactéries un choc thermique (30 minutes sur la glace puis 30-40 secondes à 42°C puis 2 minutes sur glace. On ajoute ensuite 1 ml de milieu (soc) sans ampicilline par tube et on incube 30 minutes à 37°C sous agitation. Les bactéries transformées sont ensuite étalées à l'aide de billes stériles sur boite de Pétri contenant du milieu LB+ampicilline (2 boites de Pétri pour chaque plasmide codant soit pour KITD816V soit pour KITD 417-419 insY). Ces boites de Pétri sont ensuite incubées à 37°C toute la nuit. Après cette incubation, on pique une colonie bactérienne pour chaque plasmide et on la suspend dans 200 mL de milieu LB+ampicilline qui est mis à incuber à 37°C toute la nuit. La préparation est ensuite centrifugée 15 minutes à 4°C à 8000g, le surnageant est éliminé et on ajoute sur le culot 12 ml de tampon RES contenant la RNAase (NucIeoBond^{®} AX), puis 12 ml du tampon de lyse LYS et on mélange avec précaution en inversant le tube 8 fois. On incube le mélange 5 minutes à température ambiante. On passe ensuite le lysat dans la colonne NucIeoBond^{®} xtra qu'on laisse se vider par gravité. On rince la colonne avec 15 ml de tampon EQU puis on jette le filtre en retournant la colonne. On lave la colonne avec 25 ml de tampon de lavage WASH et on élue l'ADN plasmidique avec le tampon d'élution ELU. On collecte le tampon d'élution avec l'ADN plasmidique dans un tube de 50 ml et on précipite l'ADN plasmidique élué par ajout d'isopropanol à température ambiante pendant 2 minutes. On centrifuge à 15000 g pendant 30 minutes à température ambiante, on élimine précautionneusement le surnageant et on ajoute de l'éthanol à 70% à température ambiante sur les culots et on centrifuge à 15000 g pendant 5 minutes à température ambiante, puis on élimine précautionneusement et complètement l'éthanol avec une pipette et on laisse sécher le culot à température ambiante. On dissout ensuite le culot dans un volume adéquat de tampon TE et on détermine le rendement et la pureté de l'ADN plasmidique par spectrophotométrie UV (nanodrop). L'intégrité du plasmide est confirmée par électrophorèse sur gel d'agarose

### c) Production des stocks lentiviraux

Pour produire des stocks lentiviraux, les cellules 293T (épithélium de rein humain) ont été utilisées et l'infection lentivirale a été effectuée par la méthode du phosphate de calcium en suivant la méthode décrite dans : ZUFFEREY et al. J Virol. 1998 December; 72(12): 9873-9880.
Pour ce faire, les cellules 293T (5 x 10⁶ cellules) ont été ensemencées dans des flasks de culture de 75 cm² (T-75) et infectées le lendemain avec 8 µg de Gag-pol, 3 µg d'un plasmide codant pour l'enveloppe virale du virus de la stomatite vésiculaire G (VSV-G) et 15µg de l'ADN plasmidique codant soit pour KITD816V soit pour KIT Delta 417-418 419 inserY. Vingt-quatre heures plus tard, le surnageant de culture est filtré avec un filtre 0,45µm (low protein binding Durapore ; Milipore). Le surnageant filtré a été ensuite ultracentrifugé à 20 000 rpm pendant 2h sous vide. Après centrifugation, le surnageant a été éliminé et le culot (virus) a été repris dans 200µl de PBS. Ces stocks viraux ont été aliquotés dans des tubes et congelés à -80°C jusqu'à leur utilisation.

### d) Infection des cellules ROSA KIT WT avec les vecteurs lentiviraux

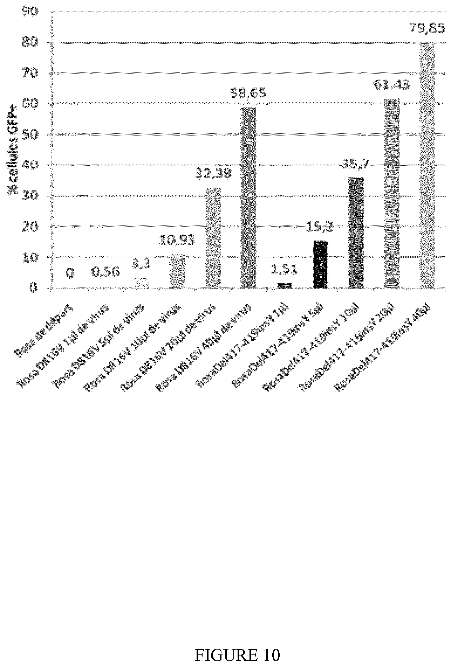

Pour l'infection des cellules ROSA KIT WT, les cellules (10⁶) ont été incubées pendant 1 heure dans 1 mL de milieu contenant du SCF (80 ng/ml) et 8 µg/ml de polybrène (hexadimethrine bromide). Les cellules ont ensuite été incubées pendant 3 heures avec des quantités variables de particules infectieuses, centrifugées et diluées dans du milieu neuf contenant du SCF. L'efficacité de l'infection a été mesurée en cytométrie en flux (détection de la fluorescence verte de la GFP) 4 jours après (figure 10).

Les cellules ont ensuite été repiquées dans le même milieu pendant 3 semaines, puis les cellules infectées positives pour la GFP ont été sélectionnées par tri cellulaire en cytométrie en flux et cultivées immédiatement dans un milieu sans SCF. Elles sont, depuis, entretenues par dilution régulière (tous les 3 ou 4 jours) dans du milieu neuf sans SCF.
Ceci nous a permis d'établir deux nouvelles lignées SCF indépendantes, **ROSA KIT D816V** et **ROSA KIT Delta 417-419 insY** dont les principales caractéristiques sont décrites ci-dessous.
Ces deux lignées sont utilisables pour le criblage à haut débit de molécules à visée anti-proliférative dirigées soit contre la molécule de KIT muté, soit contre l'une ou l'autre des molécules intracellulaires impliquées dans la transduction du signal KIT muté.

### 2) Principales caractéristiques des lignées ROSA KIT D816V et KIT Delta 417-419 insY

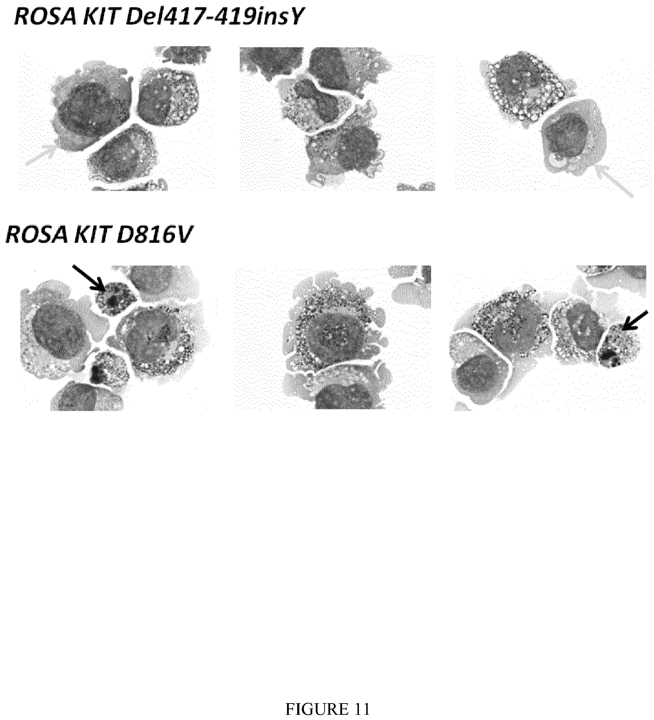

Ces deux lignées sont facilement congelables par des techniques de congélation classiques (voir plus haut). Les deux lignées ont un temps de doublement différent, de l'ordre de 48 heures pour la lignée ROSA KIT Delta 417-419 insY et de l'ordre de 72 heures pour la lignée ROSA KIT D816V. Leur aspect morphologique est aussi différent. En effet, alors qu'après coloration au MGG, les cellules de la lignée ROSA KIT Delta 417-419 insY apparaissent homogènes et relativement immatures avec peu de grains, les cellules de la lignée ROSA KIT D816V apparaissent plus matures et plus granuleuses (figure 11). Ceci est en accord avec les données de la littérature montrant que l'introduction de la mutation KIT D816V dans une lignée de précurseurs hématopoïétiques de souris induit l'apparition de caractéristiques de différenciation mastocytaire (MAYERHOFER et al. J Immunol. 2008 Apr 15;180(8):5466-76).

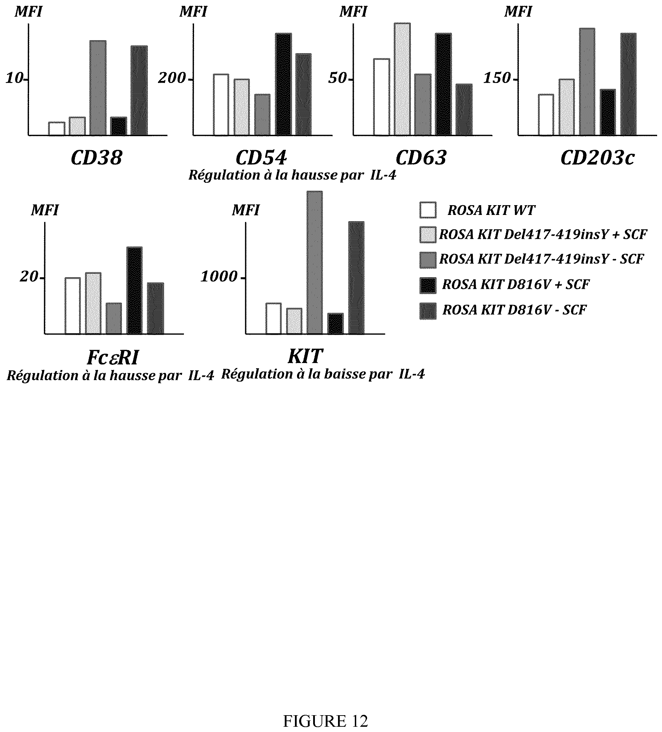

Par ailleurs, nous avons étudié l'expression, par cytométrie en flux, de certains marqueurs membranaires présents sur les cellules des lignées ROSA KIT Delta 417-419 insY et KIT D816V, mises en présence ou non de SCF, en comparant cette expression avec celle des cellules de la lignée ROSA KIT WT. Les résultats obtenus (présentés dans la figure 12) ont été les suivants :
- Les cellules des lignées ROSA KIT Delta 417-419 insY et KIT D816V expriment spontanément (en absence de SCF) plus de CD38, de CD203c et de KIT que les cellules de la lignée ROSA KIT WT, cette augmentation de l'expression étant totalement abolie en présence de SCF,
- Les cellules de la lignée KIT D816V expriment plus de CD54 que les cellules des deux autres lignées, que ce soit en présence ou en absence de SCF,
- Les cellules de la lignée KIT D816V expriment plus de FcεRI que les cellules des deux autres lignées en absence de SCF, ce phénomène étant aboli par l'ajout de SCF,
- Les cellules des lignées ROSA KIT Delta 417-419 insY et KIT D816V expriment, en présence de SCF, plus de CD63 que les cellules de la lignée ROSA KIT WT, cette augmentation de l'expression étant totalement abolie en absence de SCF,
- L'expression de CD53 et du FcεERI est augmentée dans les 3 lignées par traitement par l'IL-4 (20 ng/mL),
- L'expression de KIT est diminuée dans les trois lignées par traitement par l'IL-4 (20 ng/mL).

De plus, les inventeurs ont étudié la capacité de clonage en milieu semi-solide (méthylcellulose) des deux lignées ROSA KIT Delta 417-419 insY et KIT D816V, en absence de SCF, en la comparant à celle des cellules de la lignée ROSA KIT WT (en présence de SCF à 80 ng/mL). Pour ce faire, les cellules ont été ensemencées à raison de 5000 par boite de Pétri contenant 1 mL de milieu de culture additionné de 1% de méthylcellulose (concentration finale) et mises à incuber à 37°C en atmosphère humide contenant 5% de CO₂ dans l'air. Le décompte des colonies a été effectué après 28 jours d'incubation pour les ROSA KIT WT et 15 jours d'incubation pour les ROSA KIT Delta 417-419 insY et KIT D816V. Un exemple de culture en milieu semi-solide est montré dans la figure 12 et les résultats obtenus sont présentés dans le Tableau I.

**Tableau I : Etude de la clonogénicité des cellules des lignées ROSA KIT WT, KIT Delta 417-419 insY et KIT D816V en milieu semi-solide (méthylcellulose à 1 %).**

| **Lignée** | **Nombre de colonies boite 1** | **Nombre de colonies boite 2** | **Nombre de colonies boite 3** | **Moyenne** | **Pourcentage de cellules clonogéniques** |
|---|---|---|---|---|---|
| **ROSA KIT WT** | 238 | 408 | 372 | 333 | 6,8% |
| **ROSA KIT Delta 417-419 insY** | 173 | 438 | 445 | 352 | 7,04% |
| **ROSA KIT D816V** | 203 | 117 | 292 | 204 | 4,08% |

Les résultats de ces expériences montrent que les cellules des lignées ROSA KIT WT et KIT Delta 417-419 insY ont une clonogénicité comparable de l'ordre de 7%, tandis que les cellules de la lignée ROSA KIT D816V sont moins clonogéniques (pourcentage de cellules clonogéniques aux alentours de 4%), ce qui est en accord avec le temps de doublement un peu plus long et l'aspect plus mature des cellules de la lignée ROSA KIT D816V.

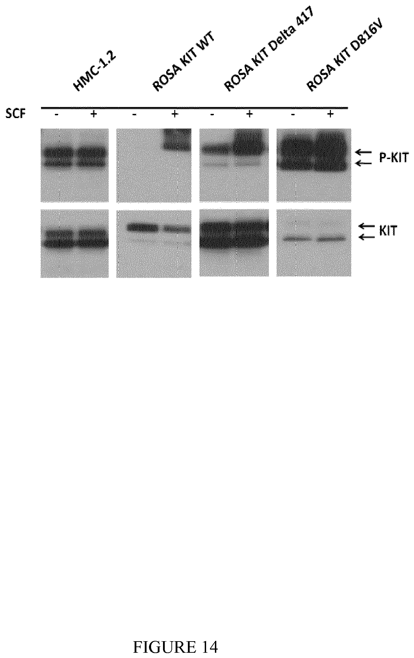

Par ailleurs, les inventeurs ont analysé la structure du récepteur KIT dans les 3 lignées ROSA KIT WT, ROSA KIT Delta 417-419 insY et ROSA KIT D816V. Pour ce faire, l'ARN total a été extrait à partir des cellules de chaque lignée en utilisant un kit RNeasy Mini (Qiagen SA, Courtaboeuf, France). L'ARN a été reverse transcrit en ADNc en utilisant un système de synthèse StrataScript premier brin (Stratagene, Massy, France) et des amorces hexamères aléatoires dans un volume total de 25 mL, selon les instructions du fabricant. Les séquences codantes de KIT ont ensuite été amplifiées par PCR à partir de 2,5 mL d'ADNc, en utilisant l'ADN polymérase HotStarTaq (Qiagen SA) et les amorces déjà publiées (Bodemer C et al, J Invest Dermatol. 2010 Mar;130(3):804-815), en appliquant 40 cycles à 94°C pendant 30 secondes, 57°C pendant 30 secondes, et 72°C pendant 45 secondes. Les produits de PCR ont été purifiés en utilisant un Kit GeneClean III (Qbiogene, Illkirch-Graffenstaden, France), et l'ensemble des régions codantes de KIT a été séquence directement en utilisant un kit BigDye Terminator v1.1 (Applied Biosystems, Courtaboeuf, France), les amorces de séquençage publiées (voir plus haut amorces de PCR), et un séquenceur ABI Prism 3100 (Applied Biosystems).
Les résultats obtenus confirment la présence du KIT sauvage dans les 3 lignées ROSA KIT WT, ROSA KIT Delta 417-419 insY et ROSA KIT D816V. En outre, pour la lignée ROSA D816V, le séquençage de KIT montre que le codon 816 de KIT WT (GAC) a été remplacé par GTC. Pour la lignée ROSA KIT Delta 417-419 insY, le séquençage de KIT montre que les codons 417/418/419 de KIT WT (ACTTACGAC) ont été remplacés par le codon TAC. Ces résultats montrent que la lignée ROSA KIT WT présente bien uniquement un KIT sauvage, expliquant sa dépendance vis-à-vis du SCF pour sa croissance, tandis que l'on retrouve la structure de KIT attendue dans les lignées ROSA KIT Delta 417-419 insY et ROSA KIT D816V, ce qui pourrait expliquer leur indépendance vis-à-vis du SCF pour leur prolifération.
Nous avons ensuite vérifié par la méthode de Western Blot la présence de KIT et son état de phosphorylation dans les trois lignées ROSA KIT WT, ROSA KIT Delta 417-419 insY et ROSA KIT D816V, traitées ou non par du SCF humain recombinant (80 ng/mL) et ce, en comparaison avec la lignée HMC-1.2. Les résultats de ces expériences, présentés dans la figure 14, montrent que la protéine KIT est spontanément présente dans les 4 lignées. De plus, il existe une phosphorylation constitutive de KIT dans la lignée HMC-1.2, mais aussi dans les lignées ROSA KIT Delta 417-419 insY et ROSA KIT D816V, et ce en l'absence de SCF. En revanche, KIT n'est phosphorylé sur tyrosine qu'en présence de SCF. Ces résultats confirment que l'indépendance vis-à-vis du SCF des lignées ROSA KIT Δ 417-419 insY et ROSA KIT D816V est bien due à une phosphorylation constitutive du récepteur KIT muté dans ces deux lignées.

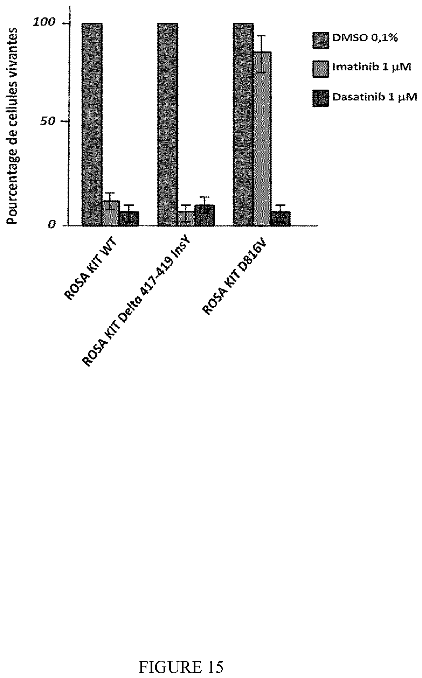

Enfin, les inventeurs ont évalué l'effet de deux molécules inhibitrices de l'activité tyrosine kinase, l'imatinib et le dasatinib (toutes les deux fournies par Sequoia Research) sur la prolifération des 3 lignées ROSA KIT WT, ROSA KIT D816V et ROSA KIT Delta 417-419 InsY. Pour ce faire, les cellules ont été ensemencées à une concentration de départ de 3.10⁵ cellules par mL dans des plaques 96 puits (100 µL par puits) et incubées à 37°C à l'étuve (5% de CO₂ dans l'air) pendant 48 heures en présence d'un µM/L d'imatinib ou de dasatinib (dans du DMSO apporté à 0,1% en concentration finale) ou en présence de DMSO seul (0,1% concentration finale) dans leur milieu de culture habituel (contenant du SCF humain à 80 ng/mL pour la lignée ROSA KIT WT mais sans SCF pour les lignées ROSA KIT D816V et ROSA KIT Delta 417-419 InsY). A la fin de cette incubation, on ajoute dans chaque puits 10 µL de WST-1 (Roche Applied Science) et les cellules sont incubées pendant 3 heures supplémentaires à l'étuve à 37°C. Le nombre de cellules vivantes est alors mesuré pour chaque condition par lecture de l'absorbance à 450 nm en utilisant un lecteur de plaques MultiSkan-MS (Thermo-LabSystems).
Les résultats obtenus pour chaque lignée (présentés dans la figure 15) sont exprimés en % de cellules vivantes (la condition DMSO seul à 0,1% final étant considérée comme contenant 100% de cellules vivantes).

Les résultats obtenus sont parfaitement conformes à ceux de la littérature (SHAH et al. Blood. 2006 Jul 1;108(1):286-91). En effet, ils montrent qu'aussi bien les cellules de la lignée ROSA KIT WT que celles de la lignée ROSA KIT Delta 417-419 InsY sont sensibles à l'effet inhibiteur de l'Imatinib (inhibiteur de KIT WT ou de KIT muté au niveau du domaine extra-cellulaire de KIT) et à l'effet inhibiteur du Dasatinib. En revanche, et comme attendu, l'Imatinib est incapable d'inhiber la prolifération des cellules de la lignée ROSA KIT D816V. Ces résultats montrent ainsi que ces trois lignées cellulaires sont parfaitement adaptées au criblage différentiel d'inhibiteurs des différentes formes de KIT sauvage ou de KIT muté.

### REFERENCES

1. Razin E, Ihle JN, Seldin D, Mencia-Huerta JM, Katz HR, LeBlanc PA, Hein A, Caulfield JP, Austen KF, Stevens RL. Interleukin 3: A differentiation and growth factor for the mouse mast cell that contains chondroitin sulfate E proteoglycan. J Immunol. 1984 Mar;132(3) :1479-86.
2. Levi-Schaffer F, Austen KF, Gravallese PM, Stevens RL. Coculture of interleukin 3-dependent mouse mast cells with fibroblasts results in a phenotypic change of the mast cells. Proc Natl Acad Sci USA. 1986 Sep;83(17):6485-8.
3. Tadokoro K, Stadler BM, De Weck AL. Factor-dependent in vitro growth of human normal bone marrow-derived basophil-like cells. J Exp Med. 1983 Sep 1 ;158(3):857-71.
4. Moqbel R, Wakelin D, MacDonald AJ, King SJ, Grencis RK, Kay AB. Release of leukotrienes during rapid expulsion of Trichinella spiralis from immune rats. Immunology, 1987 Mar;60(3):425-30.
5. Dayton ET, Pharr P, Ogawa M, Serafin WE, Austen KF, Levi-Schaffer F, Stevens RL.3T3 fibroblasts induce cloned interleukin 3-dependent mouse mast cells to resemble connective tissue mast cells in granular constituency.Proc Natl Acad Sci USA. 1988 Jan;85(2):569-72
6. Butterfield JH, Weiler D, Dewald G, Gleich GJ. Establishment of an immature mast cell line from a patient with mast cell leukemia. Leuk Res. 1988;12(4):345-55.
7. Befus AD. Mast cells are that polymorphic! Reg Immunol. 1989 May-Jun;2(3):176-87
8. Burd PR, Rogers HW, Gordon JR, Martin CA, Jayaraman S, Wilson SD, Dvorak AM, Galli SJ, Dorf ME Interleukin 3-dependent and -independent mast cells stimulated with IgE and antigen express multiple cytokines. J Exp Med. 1989 Jul 1;170(1):245-57.
9. Furitsu T, Saito H, Dvorak AM, Schwartz LB, Irani AM, Burdick JF, Ishizaka K, Ishizaka T. Development of human mast cells in vitro. Proc Natl Acad Sci USA. 1989 Dec;86(24):10039-43.
10. Galli SJ.New insights into "the riddle of the mast cells": microenvironmental régulation of mast cell development and phenotypic heterogeneity.Lab Invest. 1990 Jan;62(1):5-33.
11. Schaeffer WI. Terminology associated with cell, tissue, and organ culture, molecular biology, and molecular genetics. Tissue Culture Association Terminology Committee. In Vitro Cell Dev Biol. 1990 Jan;26(1):97-101.
12. Furitsu T, Tsujimura T, Tono T, Ikeda H, Kitayama H, Koshimizu U, Sugahara H, Butterfield JH, Ashman LK, Kanayama Y, et al. Identification of mutations in the coding sequence of the proto-oncogene c-kit in a human mast cell leukemia cell line causing ligand-independent activation of c-kit product. J Clin Invest. 1993 Oct;92(4):1736-44.
13. Valent P. The riddle of the mast cell: kit(CD117)-ligand as the missing link? Immunol Today. 1994 Mar;15(3):111-4.
14. Abraham SN, Malaviya R. Mast cells in infection and immunity. Infect Immun. 1997 Sep;65(9):3501-8.
15. Kirshenbaum AS, Goff JP, Semere T, Foster B, Scott LM, Metcalfe DD. Demonstration that human mast cells arise from a progenitor cell population that is CD34(+), c-kit(+), and expresses aminopeptidase N (CD13). Blood. 1999 Oct 1;94(7):2333-42.
16. Varadaradjalou S, Féger F, Thieblemont N, Hamouda NB, Pleau JM, Dy M, Arock M. Toll-like receptor 2 (TLR2) and TLR4 differentially activate human mast cells. Eur J Immunol. 2003 Apr;33(4):899-906.
17. Kirshenbaum AS, Akin C, Wu Y, Rottem M, Goff JP, Beaven MA, Rao VK, Metcalfe DD. Characterization of novel stem cell factor responsive human mast cell lines LAD 1 and 2 established from a patient with mast cell sarcoma/leukemia; activation following aggregation of FcepsilonRI or FcgammaRI. Leuk Res 2003; 27(8):677-82.
18. Yoshikubo T, Inoue T, Noguchi M, Okabe H. Differentiation and maintenance of mast cells from CD34(+) human cord blood cells. Exp Hematol. 2006; 34(3):320-9.
19. Valent P, Akin C, Escribano L, Födinger M, Hartmann K, Brockow K, Castells M, Sperr WR, Kluin-Nelemans HC, Hamdy NA, Lortholary O, Robyn J, van Doormaal J, Sotlar K, Hauswirth AW, Arock M, Hermine O, Hellmann A, Triggiani M, Niedoszytko M, Schwartz LB, Orfao A, Horny HP, Metcalfe DD. Standards and standardization in mastocytosis: consensus statements on diagnostics, treatment recommendations and response criteria. Eur J Clin Invest 2007; 37(6):435-53.
20. Malbec O, Roget K, Schiffer C, lannascoli B, Dumas AR, Arock M, Daëron M. Peritoneal cell-derived mast cells: an in vitro model of mature serosal-type mouse mast cells. J Immunol 2007; 178(10):6465-75.
21. Mayerhofer M, Gleixner KV, Hoelbl A, Florian S, Hoermann G, Aichberger KJ, Bilban M, Esterbauer H, Krauth MT, Sperr WR, Longley JB, Kralovics R, Moriggl R, Zappulla J, Liblau RS, Schwarzinger I, Sexl V, Sillaber C, Valent P. Unique effects of KIT D816V in BaF3 cells: induction of cluster formation, histamine synthesis, and early mast cell differentiation antigens. J Immunol. 2008 Apr 15;180(8):5466-76.
22. Bodemer C, Hermine O, Palmérini F, Yang Y, Grandpeix-Guyodo C, Leventhal PS, Hadj-Rabia S, Nasca L, Georgin-Lavialle S, Cohen-Akenine A, Launay JM, Barete S, Feger F, Arock M, Catteau B, Sans B, Stalder JF, Skowron F, Thomas L, Lorette G, Plantin P, Bordigoni P, Lortholary O, de Prost Y, Moussy A, Sobol H, Dubreuil P. Pediatric mastocytosis is a clonal disease associated with D816V and other activating c-KIT mutations. J Invest Dermatol 2010; 30(3):804-15.
23. Laidlaw TM, Steinke JW, Tĩñana AM, Feng C, Xing W, Lam BK, Paruchuri S, Boyce JA, Borish L. Characterization of a novel human mast cell line that responds to stem cell factor and expresses functional FceRI. J Allergy Clin Immunol. 2011; 127(3):815-22.

## Revendications

1. Lignée cellulaire mastocytaire humaine présentant les caractéristiques morphologiques, ultrastructurales et phénotypiques suivantes : i) présence de granulations intra-cytoplasmiques métachromatiques, ii) expression des récepteurs FcεR1 et KIT sauvage (CD117), et des marqueurs CD33, CD203c et CD300a, ainsi que les caractéristiques fonctionnelles suivantes : i) dépendance stricte vis-à-vis du SCF pour sa survie et sa croissance et ii) temps de doublement d'au plus 72 heures.

2. Lignée cellulaire mastocytaire humaine selon la revendication 1, présentant en outre les caractéristiques fonctionnelles suivantes : iii) expression du FcεR1 augmentée par traitement avec l'interleukine 4 (IL-4) et/ou avec des IgE humaines, iv) expression du CD117 diminuée par traitement avec l'interleukine 4 (IL-4), v) augmentation immédiate de l'expression membranaire de CD203c et/ou libération immédiate d'histamine, de bêta-hexosaminidase et de tryptase, et de manière retardée de TNF-α, par activation :
i) en présence d'IgE spécifique et de l'antigène capable de se lier à ladite IgE spécifique,
ii) en présence d'IgE et d'anti-IgE, ou
iii) en présence d'anticorps anti-récepteur FcεR1.

3. Lignée cellulaire mastocytaire humaine selon la revendication 1 ou 2, identifiée comme ROSA KIT WT telle qu'enregistrée sous le numéro de dépôt CNCM I-4551 auprès de la CNCM le 2 novembre 2011.

4. Lignée cellulaire dérivée de la lignée mastocytaire humaine identifiée comme ROSA KIT WT selon la revendication 3, ladite lignée dérivée étant identifiée comme ROSA KIT D816V et étant telle qu'enregistrée sous le numéro de dépôt CNCM I-4552 auprès de la CNCM le 2 novembre 2011.

5. Lignée cellulaire dérivée de la lignée mastocytaire humaine identifiée comme ROSA KIT WT selon la revendication 3, ladite lignée dérivée étant identifiée comme ROSA KIT Delta 417-419 insY et étant telle qu'enregistrée sous le numéro de dépôt CNCM I-4553 auprès de la CNCM le 2 novembre 2011.

6. Procédé de préparation d'une population de mastocytes humains capables de proliférer pendant une période supérieure à 6 mois dont au moins 99% des cellules présentent les caractéristiques des cellules d'une lignée cellulaire mastocytaire humaine selon la revendication 1 ou 2, comprenant la culture de précurseurs hématopoïétiques provenant du sang de cordon ombilical d'un sujet humain sain dans un milieu comprenant au moins 50 ng/ml de Stem Cell Factor (SCF) humain et l'obtention d'une population de mastocytes humains capables de proliférer pendant une période supérieure à 6 mois dont au moins 99% des cellules présentent les caractéristiques des cellules d'une lignée cellulaire mastocytaire humaine selon la revendication 1 ou 2.

7. Procédé de préparation d'une lignée cellulaire mastocytaire présentant une mutation du récepteur KIT, de préférence une mutation activatrice associée à une pathologie choisie parmi une mastocytose, une leucémie aiguë, un lymphome et une tumeur solide, comprenant la transformation de la lignée cellulaire mastocytaire humaine selon l'une des revendications 1 à 3, par introduction, dans les cellules de ladite lignée, d'un acide nucléique codant pour un récepteur KIT muté, et l'obtention d'une lignée cellulaire mastocytaire présentant ladite mutation de KIT.

8. Lignée cellulaire mastocytaire susceptible d'être obtenue à l'issue du procédé selon la revendication 7, **caractérisée en ce qu'**elle présente une mutation du récepteur KIT responsable i) de l'acquisition d'une indépendance vis-à-vis du SCF pour sa survie et sa croissance ; ii) de l'acquisition d'une tumorigénicité *in vivo* chez le mammifère, iii) d'une augmentation de la capacité à libérer un médiateur de l'inflammation ou de l'allergie en présence d'IgE spécifique et de l'antigène capable de se lier à ladite IgE spécifique, en présence d'IgE et d'anti-IgE, ou en présence d'anticorps anti-récepteur FcεR1, ou par activation d'un récepteur de type TLR, d'un récepteur de fraction du complément, ou d'un récepteur de cytokine ou de chimiokine ; iv) d'un changement, typiquement d'une activation, de la signalisation intra-mastocytaire ; et/ou v) d'une interaction dudit récepteur KIT muté ou de l'une au moins de ses voies de signalisation avec au moins un autre récepteur mastocytaire ou l'une au moins de ses voies de signalisation.

9. Kit permettant le criblage d'un agent d'intérêt comprenant i) au moins un produit choisi parmi la lignée cellulaire mastocytaire selon l'une des revendications 1-5 ou 8 ; ii) un produit supplémentaire choisi parmi un ou plusieurs milieux de culture, un ou plusieurs milieux d'entretien, un ou plusieurs facteurs de croissance permettant ou favorisant la culture des cellules mastocytaires ; et toute combinaison desdits produits.

10. Modèle animal non humain comprenant au moins une cellule provenant de la lignée cellulaire mastocytaire selon l'une des revendications 1-5 ou 8.

11. Utilisation de la lignée cellulaire mastocytaire selon l'une des revendications 1-5 ou 8, pour cribler un agent d'intérêt utile à la prévention, au diagnostic, au traitement et/ou au suivi d'une pathologie, de préférence choisie parmi une allergie, une maladie inflammatoire, une maladie auto-immune, une infection, l'asthme non allergique, un urticaire, et une tumeur, typiquement une mastocytose, une leucémie aigüe, un lymphome et une tumeur solide.

12. Méthode d'évaluation de la capacité d'au moins une molécule candidate à moduler la tumorigénicité, la clonogénicité, la survie, l'apoptose, la prolifération, la différentiation, l'activation, la fonction, le phénotype, l'aspect morphologique et/ou ultrastructural de mastocytes humains comprenant :
a) la mise en contact de la lignée cellulaire mastocytaire selon l'une des revendications 1-5 ou 8, avec au moins une molécule candidate ; et
b) la détermination de la tumorigénicité, la clonogénicité, la survie, l'apoptose, la prolifération, la différentiation, l'activation, la fonction, le phénotype, l'aspect morphologique et/ou ultrastructural des mastocytes appartenant à ladite lignée exposés à ladite au moins une molécule candidate, ladite détermination permettant d'évaluer la capacité correspondante de ladite au moins une molécule candidate à moduler la tumorigénicité, la clonogénicité, la survie, l'apoptose, la prolifération, la différentiation, l'activation, la fonction, le phénotype, l'aspect morphologique et/ou ultrastructural de mastocytes humains.

13. Méthode de détermination de la capacité d'au moins une molécule candidate à interférer avec la liaison d'au moins un ligand ou d'au moins un substrat à au moins un récepteur de mastocyte humain ou à au moins un autre substrat mastocytaire ; à moduler la transduction d'au moins un signal dans un mastocyte humain ; et/ou à moduler la synthèse et/ou la libération d'au moins un médiateur par un mastocyte humain, comprenant :
a) la mise en contact de la lignée cellulaire mastocytaire selon l'une des revendications 1-5 ou 8, avec au moins une molécule candidate ; et
b) la mise en évidence ou la mesure i) d'une liaison d'au moins un ligand ou d'au moins un substrat à au moins un récepteur de mastocyte humain ou à au moins un autre substrat mastocytaire, ii) de la synthèse ou de la libération d'au moins un médiateur par un mastocyte humain, et/ou iii) de la transduction d'au moins un signal dans les mastocytes de ladite lignée, de manière à déterminer respectivement i) la capacité de ladite au moins une molécule candidate à interférer avec la liaison d'au moins un ligand ou d'au moins un substrat à au moins un récepteur présent à la surface d'un mastocyte humain ou à au moins un autre substrat mastocytaire, ii) à moduler la synthèse ou la libération d'au moins un médiateur par un mastocyte humain et/ou iii) à moduler la transduction d'au moins un signal dans un mastocyte humain.

14. Méthode de détermination de la capacité d'au moins une molécule candidate à augmenter ou diminuer la survie et/ou la prolifération, et/ou à inhiber ou induire l'apoptose des mastocytes humains comprenant :
a) la mise en contact de la lignée cellulaire mastocytaire selon l'une des revendications 1-5 ou 8, avec au moins une molécule candidate ; et
b) la détermination du niveau de la survie et/ou de la prolifération et/ou de l'apoptose des mastocytes de ladite lignée, une augmentation ou une diminution de la survie et/ou de la prolifération et/ou l'inhibition ou l'induction de l'apoptose desdites cellules déterminant la capacité de la au moins une molécule candidate respectivement à augmenter ou diminuer la survie et/ou la prolifération et/ou à inhiber ou induire l'apoptose des mastocytes humains.

15. Méthode de détermination de la capacité d'au moins une molécule candidate à moduler l'infectiosité d'au moins un agent infectieux vis-à-vis des mastocytes humains, ladite méthode comprenant :
a) la mise en contact de la lignée cellulaire mastocytaire selon l'une des revendications 1-5 ou 8, avec au moins une molécule candidate et au moins un agent infectieux ; et
b) la détermination de la capacité dudit au moins un agent infectieux à infecter le mastocyte, ladite détermination permettant de déterminer la capacité de la au moins une molécule candidate à moduler l'infectiosité dudit au moins un agent infectieux vis-à-vis des mastocytes humains.

## Patentansprüche

1. Humane Mastzelllinie, die die folgenden morphologischen, ultrastrukturellen und phänotypischen Merkmale: i) Vorhandensein von metachromatischen intra-cytoplasmatischen Granulationen, ii) Expression des FcεR1-Rezeptors und des Wildtyp-KIT-Rezeptors (CD 117) und der Marker CD33, CD203c und D300a, sowie die folgenden funktionellen Merkmale aufweist: i) strikte Abhängigkeit vom SCF bezüglich Überlebensrate und Wachstum und ii) Verdopplungszeit von mehr als 72 Stunden.

2. Humane Mastzelllinie gemäß Anspruch 1, die außerdem die folgenden funktionellen Merkmale aufweist: iii) erhöhte FcεR1-Expression durch Behandlung mit Interleukin 4 (IL-4) und/oder mit humanen IgEs, iv) verringerte CD117-Expression durch Behandlung mit Interleukin 4 (IL-4), v) sofortige Erhöhung der Membranexpression von CD203c und/oder sofortige Freisetzung von Histamin, beta-Hexosaminidase und Tryptase und verzögerte Freisetzung von TNF-α durch Aktivierung:
i) in Gegenwart von spezifischem IgE und des Antigens, das in der Lage ist, an besagtes spezifisches IgE zu binden,
ii) in Gegenwart von IgE und anti-IgE, oder
iii) in Gegenwart von Anti-FcεR1-Rezeptor-Antikörper.

3. Humane Mastzelllinie gemäß Anspruch 1 oder 2, die als ROSA KIT WT identifiziert und unter der Hinterlegungsnummer CNCM 1-4551 bei der CNCM am 2. November 2011 registriert worden ist.

4. Zelllinie, die von der humanen Mastzelllinie abstammt, die als ROSA KIT WT gemäß Anspruch 3 identifiziert ist, wobei besagte Linie als ROSA KIT D816V identifiziert und unter der Hinterlegungsnummer CNCM 1-4552 bei der CNCM am 2. November 2011 registriert worden ist.

5. Zelllinie, die von der humanen Mastzelllinie abstammt, die als ROSA KIT WT gemäß Anspruch 3 identifiziert ist, wobei besagte Linie als ROSA KIT Delta 417-419 insY identifiziert und unter der Hinterlegungsnummer CNCM 1-4553 bei der CNCM am 2. November 2011 registriert worden ist.

6. Verfahren zur Herstellung einer Population humaner Mastzellen, die in der Lage sind, über einen Zeitraum von mehr als 6 Monaten zu proliferieren, darunter wenigstens 99% der Zellen die Merkmale von Zellen einer humanen Mastzelllinie gemäß Anspruch 1 oder 2 aufweisen, umfassend die Kultivierung hämatopoietischer Vorläufer, die vom Nabelschnurblut eines gesunden Menschen stammen, in einem wenigstens 50 ng/ml humanen Stammzellfaktor (SCF) umfassenden Medium und den Erhalt einer Population humaner Mastzellen, die in der Lage sind, über einen Zeitraum von mehr als 6 Monaten zu proliferieren, darunter wenigstens 99% der Zellen die Merkmale von Zellen einer humanen Mastzelllinie gemäß Anspruch 1 oder 2 aufweisen.

7. Verfahren zur Herstellung einer humanen Mastzelllinie, die eine Mutation des KIT-Rezeptors, vorzugsweise eine aktivierende Mutation, aufweist, die mit einer Krankheit assoziiert ist, ausgewählt aus einer Mastozytose, einer akuten Leukämie, einem Lymphom und einem soliden Tumor, umfassend die Transformation der humanen Mastzelllinie gemäß einem der Ansprüche 1 bis 3 durch Einführung, in die Zellen besagter Zelllinie, einer Nukleinsäure, die für einen mutierten KIT-Rezeptor codiert, und den Erhalt einer Mastzelllinie, die besagte KIT-Mutation aufweist.

8. Mastzelllinie, die mit dem Verfahren gemäß Anspruch 7 erhalten werden kann, **dadurch gekennzeichnet, dass** sie eine Mutation des KIT-Rezeptors aufweist, die verantwortlich ist i) für das Erwerben einer Unabhängigkeit vom SCF bezüglich Überlebensrate und Wachstum; ii) für das Erwerben einer Tumorgenität *in vivo* bei Säugetieren, iii) für eine Erhöhung der Fähigkeit, einen Entzündungs- oder Allergiemediator freizusetzen in Gegenwart von spezifischem IgE und des Antigens, das in der Lage ist, an besagtes spezifisches IgE zu binden, in Gegenwart von I1gE und anti-IgE oder in Gegenwart von Anti-FcεR1-Rezeptor-Antikörper, oder durch Aktivierung eines Rezeptors vom TLR-Typ, eines Komplementfraktion-Rezeptors, oder eines Cytokin- oder Chimokin-Rezeptors, iv) für eine Änderung, typischerweise eine Aktivierung, der internen Mastzellen-Signalisierung; und/oder v) für eine Interaktion besagten mutierten KIT-Rezeptors oder wenigstens eines seiner Signalisierungswege mit wenigstens einem anderen Mastzellrezeptor oder wenigstens einem seiner Signalisierungswege.

9. Kit, das die Durchmusterung eines Agens von Interesse erlaubt, umfassend i) wenigstens ein Produkt, ausgewählt aus der Mastzelllinie gemäß einem der Ansprüche 1 bis 5 oder 8; ii) ein zusätzliches Produkt, ausgewählt aus einem oder mehreren Kulturmedien, einem oder mehreren Erhaltungsmedien, einem oder mehreren Wachstumsfaktoren, die die Kultivierung der Mastzellen erlauben oder erleichtern; und beliebige Kombinationen besagter Produkte.

10. Nicht-humanes Tiermodell, umfassend wenigstens eine Zelle, die von der Mastzelllinie gemäß einem der Ansprüche 1 bis 5 oder 8 abstammt.

11. Verwendung der Mastzelllinie gemäß einem der Ansprüche 1 bis 5 oder 8 zur Durchmusterung eines Agens von Interesse, das zur Prävention, zur Diagnose, zur Behandlung und/oder zur Nachbeobachtung einer Krankheit, vorzugsweise ausgewählt aus einer Allergie, einer Entzündungskrankheit, einer Autoimmunkrankheit, einer Infektion, nicht-allergischem Asthma, einer Urticaria und einem Tumor, typischerweise einer Mastozytose, einer akuten Leukämie, einem Lymphom und einem soliden Tumor, zweckdienlich ist.

12. Verfahren zur Evaluation der Fähigkeit wenigstens eines Kandidatenmoleküls, die Tumorgenität, die Klonogenität, die Überlebensrate, die Apoptose, die Proliferation, die Differenzierung, die Aktivierung, die Funktion, den Phänotyp, die Morphologie und/oder Ultrastruktur von humanen Mastzellen zu modulieren, umfassend:
a) Inkontaktbringen der Mastzelllinie gemäß einem der Ansprüche 1 bis 5 oder 8 mit wenigstens einem Kandidatenmolekül; und
b) Bestimmung der Tumorgenität, der Klonogenität, der Überlebensrate, der Apoptose, der Proliferation, der Differenzierung, der Aktivierung, der Funktion, des Phänotyps, der Morphologie und/oder Ultrastruktur der Mastzellen, die zu besagter Linie gehören und besagtem wenigstens einem Kandidatenmolekül exponiert werden, wobei besagte Bestimmung die Evaluation der entsprechenden Fähigkeit besagten wenigstens einen Kandidatenmoleküls erlaubt, die Tumorgenität, die Klonogenität, die Überlebensrate, die Apoptose, die Proliferation, die Differenzierung, die Aktivierung, die Funktion, den Phänotyp, die Morphologie und/oder Ultrastruktur von humanen Mastzellen zu modulieren.

13. Verfahren zur Bestimmung der Fähigkeit wenigstens eines Kandidatenmoleküls, mit der Bindung wenigstens eines Liganden oder wenigstens eines Substrats an wenigstens einen humanen Mastzellrezeptor oder an wenigstens ein anderes Mastzellsubstrat zu interferieren; die Transduktion wenigstens eines Signals in einer humanen Mastzelle zu modulieren; und/oder die Synthese und/oder die Freisetzung wenigstens eines Mediators von einer humanen Mastzelle zu modulieren; umfassend
a) Inkontaktbringen der Mastzelllinie gemäß einem der Ansprüche 1 bis 5 oder 8 mit wenigstens einem Kandidatenmolekül; und
b) den Nachweis oder die Messung i) einer Bindung wenigstes eines Liganden oder wenigstens eines Substrats an wenigstens einen humanen Mastzellrezeptor oder an wenigstens ein anderes Mastzellsubstrat, ii) der Synthese oder der Freisetzung wenigstens eines Mediators von einer humanen Mastzelle, und/oder iii) der Transduktion wenigstens eines Signals in den Mastzellen besagter Linie, um jeweils die Fähigkeit besagten wenigstens einen Kandidatenmoleküls zu bestimmen i) mit der Bindung wenigstens eines Liganden oder wenigstens eines Substrats an wenigstens einen Rezeptor, der auf der Oberfläche besagter humanen Mastzelle vorhanden ist, oder an wenigstens ein anderes Mastzellsubstrat zu interferieren, ii) die Synthese oder die Freisetzung wenigstens eines Mediators von einer humanen Mastzelle zu modulieren und/oder iii) die Transduktion wenigstes eines Signals in einer humanen Mastzelle zu modulieren.

14. Verfahren zur Bestimmung der Fähigkeit wenigstens eines Kandidatenmoleküls, von humanen Mastzellen die Überlebensrate und/oder die Proliferation zu erhöhen oder zu vermindern und/oder die Apoptose zu hemmen oder zu induzieren, umfassend
a) Inkontaktbringen der Mastzelllinie gemäß einem der Ansprüche 1 bis 5 oder 8 mit wenigstens einem Kandidatenmolekül; und
b) Bestimmung des Niveaus der Überlebensrate und/oder der Proliferation und/oder der Apoptose der Mastzellen besagter Linie, wobei eine Erhöhung oder eine Verminderung der Überlebensrate und/oder der Proliferation und/oder die Hemmung oder die Induktion der Apoptose besagter Zellen die Fähigkeit des wenigstens einen Kandidatenmoleküls bestimmt, jeweils die Überlebensrate und/oder die Proliferation zu erhöhen oder zu vermindern und/oder die Apoptose der humanen Mastzellen zu hemmen oder zu induzieren.

15. Verfahren zur Bestimmung der Fähigkeit wenigstens eines Kandidatenmoleküls, die Infektiosität wenigstens eines infektiösen Agens für humane Mastzellen zu modulieren, wobei besagtes Verfahren umfasst:
a) Inkontaktbringen der Mastzelllinie gemäß einem der Ansprüche 1 bis 5 oder 8 mit wenigstens einem Kandidatenmolekül und wenigstens einem infektiösem Agens; und
b) Bestimmung der Fähigkeit besagten wenigstens einen infektiösen Agens, die Mastzelle zu infizieren, wobei besagte Bestimmung erlaubt, die Fähigkeit des wenigstens einen Kandidatenmoleküls zu bestimmen, die Infektiosität besagten wenigstens einen infektiösen Agens für humane Mastzellen zu modulieren.

## Claims

1. Human mast cell line having the following morphological, ultrastructural and phenotypic characteristics: i) presence of metachromatic intra-cytoplasmic granulations, ii) expression of FcεR1 and wild-type KIT (CD117) receptors, and of CD33, CD203c and CD300a markers, and also the following functional characteristics: i) strict dependence with respect to SCF for its survival and its growth and ii) doubling time of at most 72 hours.

2. Human mast cell line according to claim 1, further having the following functional features: iii) FcεR1 expression increased by treatment with interleukin 4 (IL-4) and/or with human IgEs, iv) CD117 expression decreased by treatment with interleukin 4 (IL-4), v) immediate increase in membrane expression of CD203c and/or immediate release of histamine, of beta-hexosaminidase and of tryptase, and delayed release of TNF-α, by activation:
i) in the presence of specific IgE and of the antigen capable of binding to said specific IgE,
ii) in the presence of IgE and of anti-IgE, or
iii) in the presence of anti-FcεR1 receptor antibody.

3. Human mast cell line according to claim 1 or 2, identified as ROSA KIT WT as registered under deposit number CNCM 1-4551 with the CNCM [French National Collection of Microorganism Cultures] on November 2, 2011.

4. Cell line derived from the human mast cell line identified as ROSA KIT WT according to claim 3, said derived line being identified as ROSA KIT D816V and being as registered under deposit number CNCM 1-4552 with the CNCM on November 2, 2011.

5. Cell line derived from the human mast cell line identified as ROSA KIT WT according to claim 3, said derived line being identified as ROSA KIT Delta 417-419 insY and being as registered under deposit number CNCM 1-4553 with the CNCM on November 2, 2011.

6. Process for preparing a population of human mast cells capable of proliferating for a period greater than 6 months, of which at least 99% of the cells have the characteristics of the cells of a human mast cell line according to claim 1 or 2, which comprises culturing haematopoietic precursors originating from the umbilical cord blood of a healthy human subject in a medium comprising at least 50 ng/ml of human Stem Cell Factor (SCF) and obtaining a population of human mast cells capable of proliferating for a period greater than 6 months, of which at least 99% of the cells have the characteristics of the cells of a human mast cell line according to claim 1 or 2.

7. Process for preparing a mast cell line exhibiting a KIT receptor mutation, preferably an activating mutation associated with a pathological condition chosen from mastocytosis, acute leukaemia, a lymphoma and a solid tumour, comprising the transformation of the human mast cell line according to one of claims 1 to 3, by introducing, into the cells of said line, a nucleic acid encoding a mutated KIT receptor, and obtaining a mast cell line exhibiting said KIT mutation.

8. Mast cell line which can be obtained at the end of the process according to claim 7, **characterized in that** it exhibits a KIT receptor mutation responsible i) for the acquisition of independence with respect to SCF for its survival and its growth; ii) for the acquisition of tumorigenicity *in vivo* in mammals, iii) for an increase in the capacity to release a mediator of inflammation or of an allergy in the presence of specific IgE and of the antigen capable of binding to said specific IgE, in the presence of IgE and of anti-IgE, or in the presence of anti-FcεR1 receptor antibody, or by activation of a receptor of TLR type, of a complement fraction receptor, or of a cytokine or chemokine receptor; iv) for a change, typically an activation, of the intra-mast cell signaling; and/or v) for an interaction of said mutated KIT receptor or of at least one of it signaling pathways with at least one other mast cell receptor or at least one of its signaling pathways.

9. Kit for screening for an agent of interest comprising i) at least one product chosen from the mast cell line according to one of claims 1-5 or 8; ii) a supplementary product chosen from one or more culture media, one or more maintenance media, one or more growth factors enabling or promoting the culturing of the mast cells; and any combination of said products.

10. Non-human animal model comprising at least one cell originating from the mast cell line according to one of claims 1-5 or 8.

11. Use of the mast cell line according to one of claims 1-5 or 8, for screening for an agent of interest which is of use in the prevention, diagnosis, treatment and/or monitoring of a pathological condition, preferably chosen from an allergy, an inflammatory disease, an autoimmune disease, an infection, non-allergic asthma, urticaria, and a tumour, typically mastocytosis, acute leukaemia, lymphoma and a solid tumour.

12. Method for evaluating the capacity of at least one candidate molecule to modulate the tumorigenicity, the clonogenicity, the survival, the apoptosis, the proliferation, the differentiation, the activation, the function, the phenotype, the morphological appearance and/or the ultrastructural appearance of human mast cells, comprising:
a) bringing the mast cell line according to one of claims 1-5 or 8 into contact with at least one candidate molecule; and
b) determining the tumorigenicity, the clonogenicity, the survival, the apoptosis, the proliferation, the differentiation, the activation, the function, the phenotype, the morphological appearance and/or the ultrastructural appearance of the mast cells belonging to said line exposed to said at least one candidate molecule, said determining making it possible to evaluate the corresponding capacity of said at least one candidate molecule to modulate the tumorigenicity, the clonogenicity, the survival, the apoptosis, the proliferation, the differentiation, the activation, the function, the phenotype, the morphological appearance and/or the ultrastructural appearance of human mast cells.

13. Method for determining the capacity of at least one candidate molecule to interfere with the binding of at least one ligand or of at least one substrate to at least one human mast cell receptor or to at least one other mast cell substrate; to modulate the transduction of at least one signal in a human mast cell; and/or to modulate the synthesis and/or the release of at least one mediator by a human mast cell, comprising:
a) bringing the mast cell line according to one of claims 1-5 or 8 into contact with at least one candidate molecule; and
b) demonstrating or measuring i) binding of at least one ligand or of at least one substrate to at least one human mast cell receptor or to at least one other mast cell substrate, ii) the synthesis or the release of at least one mediator by a human mast cell, and/or iii) the transduction of at least one signal in the mast cells of said line so as to determine respectively i) the capacity of said at least one candidate molecule to interfere with the binding of at least one ligand or of at least one substrate to at least one receptor present at the surface of a human mast cell or to at least one other mast cell substrate, ii) to modulate the synthesis or the release of at least one mediator by a human mast cell and/or iii) to modulate the transduction of at least one signal in a human mast cell.

14. Method for determining the capacity of at least one candidate molecule to increase or to decrease the survival and/or the proliferation, and/or to inhibit or induce the apoptosis of human mast cells, comprising:
a) bringing the mast cell line according to one of claims 1-5 or 8 into contact with at least one candidate molecule; and
b) determining the level of survival and/or of proliferation and/or of apoptosis of the mast cells of said line, an increase or a decrease in the survival and/or the proliferation and/or the inhibition or the induction of the apoptosis of said cells determining the capacity of the at least one candidate molecule respectively to increase or decrease the survival and/or the proliferation and/or to inhibit or to induce the apoptosis of human mast cells.

15. Method for determining the capacity of at least one candidate molecule to modulate the infectiousness of at least one infectious agent with respect to human mast cells, said method comprising:
a) bringing the mast cell line according to one of claims 1-5 or 8 into contact with at least one candidate molecule and at least one infectious agent; and
b) determining the capacity of said at least one infectious agent to infect the mast cell, said determining making it possible to determine the capacity of the at least one candidate molecule to modulate the infectiousness of said at least one infectious agent with respect to human mast cells.
